(19) Europäisches Patentamt
European Patent Office
Office européen des brevets

(11) EP 0 893 424 A1

(12) EUROPEAN PATENT APPLICATION
published in accordance with Art. 158(3) EPC

(43) Date of publication:
**27.01.1999 Bulletin 1999/04**

(21) Application number: **97915683.3**

(22) Date of filing: **09.04.1997**

(51) Int. Cl.$^6$: **C07C 43/225**, C07C 255/54, C09K 19/20, C09K 19/30, C09K 19/42, G02F 1/13

(86) International application number:
**PCT/JP97/01214**

(87) International publication number:
**WO 97/37960 (16.10.1997 Gazette 1997/44)**

(84) Designated Contracting States:
**DE FR GB**

(30) Priority: **11.04.1996 JP 89448/96**

(71) Applicant: **CHISSO CORPORATION**
**Osaka-shi Osaka 530 (JP)**

(72) Inventors:
- **MATSUI, Shuichi**
  **Ichihara-shi, Chiba 290 (JP)**
- **KOIZUMI, Yasuyuki**
  **Ichihara-shi, Chiba 290 (JP)**
- **MIYAZAWA, Kazutoshi**
  **Ichihara-shi, Chiba 290-01 (JP)**
- **SEKIGUCHI, Yasuko**
  **Ichihara-shi, Chiba 290 (JP)**
- **NAKAGAWA, Etsuo**
  **Ichihara-shi, Chiba 290 (JP)**

(74) Representative: **HOFFMANN - EITLE**
**Patent- und Rechtsanwälte**
**Arabellastrasse 4**
**81925 München (DE)**

# (54) ALPHA,ALPHA-DIFLUOROBENZYL ETHER DERIVATIVES, LIQUID CRYSTAL COMPOSITIONS, AND LIQUID CRYSTAL DISPLAY DEVICES

(57) An object of the invention is to provide novel liquid crystalline compounds which 1) exhibit a low threshold voltage, 2) have a low viscosity, 3) maintain or lower dielectric anisotropy of liquid crystal compositions when added thereto, and further are excellent in miscibility with other known liquid crystalline compounds, particularly excellent in the miscibility at low temperatures; to provide liquid crystal compositions comprising the compound; and to provide liquid crystal display devices comprising the composition; and the liquid crystalline compounds are $\alpha$, $\alpha$-difluorobenzyl ether derivatives expressed by the general formula (1)

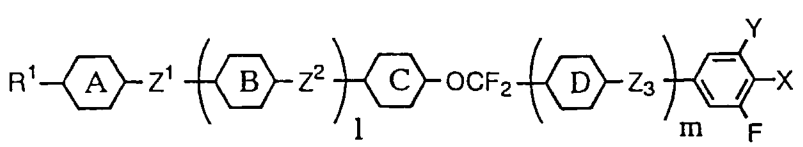

(1)

wherein $R^1$ represents a straight chain or branched alkyl group having 1 to 15 carbon atoms or an alkenyl group having 2 to 15 carbon atoms one or not-adjacent two or more $CH_2$ groups in the alkyl or alkenyl group may be replaced by oxygen atom; ring A and ring B independently represent 1,4-cyclohexylene group one or more $CH_2$ groups on the ring may be replaced by oxygen atom or sulfur atom, or 1,4-phenylene group one or more CH groups on the ring may be replaced by nitrogen atom, and one or more hydrogen atoms on the ring may be replaced by a halogen atom; ring C and ring D independently represent 1,4-phenylene group one or more hydrogen atoms on the ring may be replaced by a halogen atom; $Z^1$, $Z^2$, and $Z^3$ independently represent $-CH_2CH_2-$, -CH=CH-, $-C\equiv C-$, or single bond; X represents a halogen atom, $CF_3$, $OCF_3$, $OCHF_2$, or CN; Y represents hydrogen atom or a halogen atom; $\ell$ and m are 0 or 1; $\ell + m \leqq 1$ ; and an element which constitutes the compound may be replaced by its isotope.

EP 0 893 424 A1

## Description

### TECHNICAL FIELD

The present invention relates to α, α-difluorobenzyl ether derivatives which are novel liquid crystalline compounds developing various preferable physical properties in liquid crystal compositions principally for TN, STN, or TFT; relates to liquid crystal compositions having various preferable physical properties and comprising the novel liquid crystalline compound; and relates to liquid crystal display devices comprising the liquid crystal composition.

### BACKGROUND ART

Liquid crystal display devices utilize optical anisotropy and dielectric anisotropy of liquid crystal substances. The liquid crystal display devices are widely used in tabletop calculators, word processors, and television sets, including watches, and demand for the display devices is in a trend to increase year by year. Liquid crystal phase exists between solid phase and liquid phase, and is classified broadly into nematic phase, smectic phase, and cholesteric phase. Among them, nematic phase is most widely employed for display devices at present. On the other hand, while many display modes were devised up to date, three types of twisted nematic (TN) mode, super twisted nematic (STN) mode, and thin film transistor (TFT) mode have now become main currents. Properties required of liquid crystalline substances for these various liquid crystal display devices are different depending on their uses, but any liquid crystalline substance is required to be stable against outside environmental factors such as moisture, air, heat, and light, to exhibit liquid crystal phase at a temperature range as wide as possible with room temperature being its center, to be in low viscosity, and to have low driving voltage. However, no liquid crystal substances which satisfy such requirements at the same time by a single compound have been found.

With respect to liquid crystal substances used for liquid crystal display devices, it is an actual circumstance that several or several tens kind of liquid crystalline compounds, and further several kind of liquid non-crystalline compounds when necessary, are usually mixed to produce liquid crystal compositions and used for display devices, to obtain various physical properties such as most suitable dielectric anisotropy (Δε), optical anisotropy (Δn), viscosity, elastic constant ratio $K_{33}/K_{11}$ ($K_{33}$: bend elastic constant, $K_{11}$: splay elastic constant) which are usually required of each display device. Accordingly, liquid crystalline compounds are required to have a good miscibility with other liquid crystal compounds, and recently required to have a good miscibility even at low temperatures in particular from the requirement of being used in various environments.

Meanwhile, active matrix mode, especially thin film transistor (TFT) mode is extensively adopted in recent years as display mode, for example, for TV sets and viewfinders from the aspect of display performances such as contrast, display capacity, and response time. Also, STN mode having a large display capacity, and display devices of which mode are comparatively simple in structure and can be produced at a low production cost compared with display devices of active matrix mode, is largely adopted in displays, for example, for personal computers.

Recent development in these fields is being progressed while placing stress on downsizing of liquid crystal display devices into a portable size as shown by the development of small size TV sets and notebook size personal computers both of which are characterized by being portable. From the aspect of liquid crystal materials, development is being progressed mainly with exploration of liquid crystalline compounds and liquid crystal compositions having low driving voltage, namely, low threshold voltage, because of a problem connected to withstand voltage of integrated circuits.

It is known that threshold voltage (Vth) can be expressed by the following equation (H. J. Deuling et al., Mol. Cryst. Liq. Cryst., 27 (1975) 81):

$$Vth = \pi \, (K/\varepsilon_0 \Delta\varepsilon)^{1/2}$$

In the equation described above, K is an elastic constant and $\varepsilon_0$ is a dielectric constant in vacuum. As will be seen from this equation, two methods, that is, a method of increasing dielectric anisotropy (Δε) and a method of lowering elastic constant, can be considered to lower the threshold voltage. However, since actual control of elastic constant is very difficult, it is a current situation that liquid crystal materials having high dielectric anisotropy (Δε) are used. With the facts described above as its background, development of liquid crystalline compounds having high dielectric anisotropy (Δε) has actively been conducted.

Almost all liquid crystal compositions used in display devices for TFT mode are composed of fluorine type liquid crystal materials at present. This is because a high voltage holding ratio (V.H.R.) is required in TFT mode from the aspect of construction of devices, the materials must be a small in its dependency on temperature, and theses requirements can not be satisfied with materials other than those of fluorine type. As conventional fluorine type materials for low voltage, the following compounds are known:

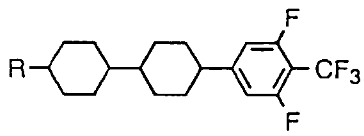

(a) DE-4027840A1

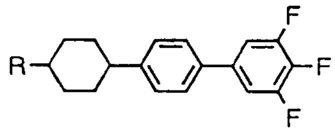

(b) USP-5,032,313

In the structural formula described above, R represents an alkyl group.

Any compounds of the formula (a) or (b) have several fluorine atoms at a terminal of the molecule, and are reported to exhibit comparatively high dielectric anisotropy. However, it is known that their clearing point (NI point) is low and their viscosity is comparatively high. Whereas the relation between the number of substituted fluorine atoms and the clearing point or viscosity is not simple, it is known among persons skilled in the art through experience that there is an inverse proportional and direct proportional relation, respectively. Accordingly, there were such many problems that when liquid crystal compositions were produced only from a series of these compounds, not only required clearing point and viscosity (response speed) can not be achieved, but also ionic impurities are tend to be taken in the compositions from the outside environment in actual uses due to their large polarity and their voltage holding ratio lowers as its result.

In order to solve the problems described above, compounds which control elastic constant ratio and cope with lowering of the voltage are quite recently reported in patent publications. As examples, the following compounds are disclosed:

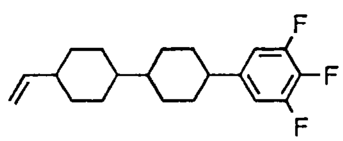

(c) Laid-open Japanese Patent Publication No. Hei 6-211711

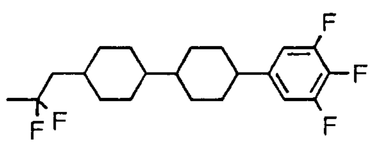

(d) Laid-open Japanese Patent Publication No. Hei 6-192142

Whereas the compounds of the formula (c) have low viscosity compared with other compounds having the same skeleton, the compounds of the formula (c) are unsuitable for solving the problems described above since the stability of unsaturated bonding groups in a lateral chain against the changes of the outside environment including heat and light is low compared with the compounds having saturated bonding groups in a lateral chain, and the value of dielectric anisotropy is high. On the other hand, whereas it is disclosed in the specification that the compounds of the formula (d) have such characteristics that they have a high clearing point compared with other compounds having the same skeleton and that they have a small elastic constant, the compounds of the formula (d) are considered, according to the study by the present inventors, to be unsuitable to use for liquid crystal compositions of which high response speed is required since the compounds have an extremely high viscosity due to two fluorine atoms substituted at a side chain.

As described above, it is a current situation that no liquid crystalline compounds which have a small elastic constant, high voltage holding ratio, and low viscosity, are capable of being driven at a low voltage, and are capable of responding at a high speed, have been found. Thus, compounds having improved characteristics sufficient to solve the problems described above have been long-awaited.

DISCLOSURE OF THE INVENTION

Subject of the present invention is to provide novel liquid crystalline compounds which 1) have low threshold voltage, 2) have low viscosity, 3) maintain or lower dielectric anisotropy when added to liquid crystal compositions, and 4)

are excellent in miscibility with other known liquid crystalline compounds, and particularly good in miscibility at low temperatures; to provide liquid crystal compositions comprising the compound; and to provide liquid crystal display devices comprising the composition.

With respect to compounds having a partial structure wherein two phenylene groups are cross-linked with bonding group $-CF_2O-$, compounds of the formulas (e) and (f) have already been disclosed in Laid-open Japanese Patent Publication No. Hei 2-289529 and Laid-open Japanese Patent Publication No. Hei 5-112778, respectively. The structural formula of the compounds have been described in the patent publications, however, physical data of the compounds and specific physical properties necessary for evaluating the utilities of the compounds as liquid crystalline ones have not been disclosed in the least. Accordingly, characteristics of the compounds have not been known at all.

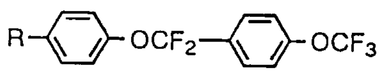

(e) Laid-open Japanese Patent Publication No. Hei 2-289529

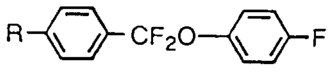

(f) Laid-open Japanese Patent Publication No. Hei 5-112778

Then, compounds which are expressed by the general formula (1), which have a partial structure wherein two 1,4-phenylene groups are cross-linked with bonding group $-OCF_2-$, and which have fluorine atom at position 3, hydrogen atom or a halogen atom at position 5, and a halogen atom, $CF_3$, $OCF_3$, $OCHF_2$, or CN group at position 4, respectively, in $\alpha$, $\alpha$-difluorobenzyl group were devised by the present invenotrs; and the physical properties of the compounds were studied to find the fact that the compounds are not only remarkably stable against the outside environment but also arehave extremely low viscosity than expected by the present inventors. It was found that the compounds of the present invention exhibit a low threshold voltage which is about the same degree as that of the compounds (a), (b), and (c) discussed in the section of BACKGROUND ART described above and is considered to be caused by their small elastic constant, despite the fact that the compounds of the present invention have a low dielectric anisotropy compared with the compounds of (a), (b), and (c). It was also found that the compounds of the present invention are excellent in miscibility with known compounds, particularly good in miscibility at low temperatures. Further, it was found that the compounds of the present invention expressed by the general formula (1) wherein an alkyl group was selected as substituent $R^1$ and one of the groups other than CN group was selected as X exhibit a high voltage holding ratio and exhibit preferable characterisitcs as liquid crystal materials for TFT mode display devices, particularly for a low voltage driving or high response speed, while the compounds of the present invention of the formula (1) wherein an alkenyl group was selected as substituent $R^1$ and CN group was selceted as X exhibit a remarkably low viscosity or a high clearing point, and thus are very useful as viscosity reducing agent for STN mode display devices, leading to the accomplishment of invention of the compounds recited in the appended claims as novel liquid crystalline compound.

That is, in order to solve the problems described above, the present invention is summarized as follows:

[1] An $\alpha$, $\alpha$-difluorobenzyl ether derivative expressed by the general formula (1)

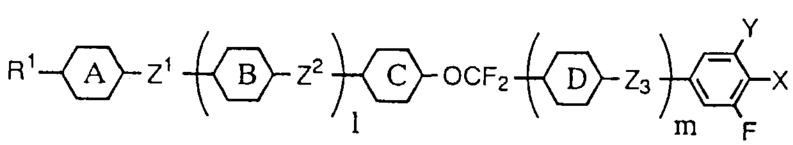

(1)

wherein $R^1$ represents a straight chain or branched alkyl group having 1 to 15 carbon atoms or an alkenyl group having 2 to 15 carbon atoms one or not-adjacent two or more $CH_2$ groups in the alkyl or alkenyl group may be replaced by oxygen atom; ring A and ring B independently represent 1,4-cyclohexylene group one or more $CH_2$ groups in the ring may be replaced by oxygen atom or sulfur atom, or 1,4-phenylene group one or more CH groups in the ring may be replaced by nitrogen atom, and one or more hydrogen atoms on the ring may be replaced by a halogen atom; ring C and ring D independently represent 1,4-phenylene group one or more hydrogen atoms on the ring may be replaced by a halogen atom; $Z^1$, $Z^2$, and $Z^3$ independently represent $-CH_2CH_2-$, $-CH=CH-$, $-C\equiv C-$, or single bond; X represents a halogen atom, $CF_3$, $OCF_3$, $OCHF_2$, or CN group; Y represents hydrogen atom or a halogen atom; $\ell$ and m are 0 or 1; $\ell + m \leqq 1$ ; and an element which constitutes the compound may be replaced by

its isotope.

[2] The compound recited in [1] above wherein ℓ and m are 0 in the general formula (1).

[3] The compound recited in [2] above wherein ring A represents 1,4-cyclohexylene group, and ring C represents 1,4-phenylene group in the general formula (1).

[4] The compound recited in [3] above wherein X represents fluorine atom, $CF_3$, or $OCF_3$ group, and Y represents hydrogen atom in the general formula (1).

[5] The compound recited in [3] above wherein X represents fluorine atom, $CF_3$, or $OCF_3$ group, and Y represents a halogen atom in the general formula (1).

[6] The compound recited in [1] above wherein ℓ is 0, and m is 1 in the general formula (1).

[7] The compound recited in [6] above wherein ring A represents 1,4-cyclohexylene group, and ring C and ring D represent 1,4-phenylene group in the general formula (1).

[8] The compound recited in [1] above wherein ℓ is 1, and m is 0 in the general formula (1).

[9] The compound recited in [8] above wherein ring A and ring B represent 1,4-cyclohexylene group, and ring C represents 1,4-phenylene group in the general formula (1).

[10] The compound recited in [1] above wherein $R^1$ represents an alkenyl group in the general formula (1).

[11] A liquid crystal composition comprising at least two components and comprising at least one compound expressed by the general formula (1).

[12] A liquid crystal composition comprising, as a first component, at least one liquid crystalline compound recited in any one of [1] to [10] above, and comprising, as a second component, at least one compound selected from the group consisting of the compounds expressed by any one of the general formulas (2), (3), and (4)

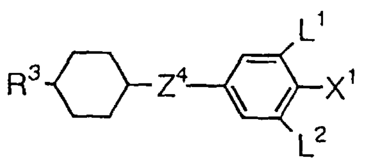

(2)

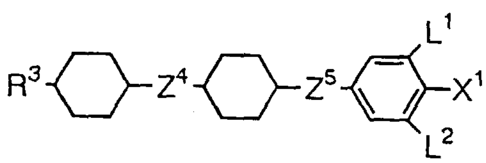

(3)

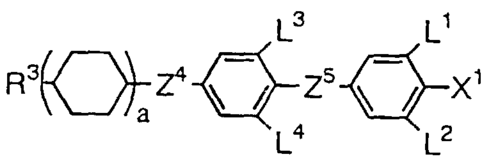

(4)

wherein $R^3$ represents an alkyl group having 1 to 10 carbon atoms, $X^1$ represents F, Cl, $OCF_3$, $OCF_2H$, $CF_3$, $CF_2H$, or $CFH_2$; $L^1$, $L^2$, $L^3$, and $L^4$ independently represent H or F; $Z^4$ and $Z^5$ independently represent $-(CH_2)_2-$, -CH=CH-, or single bond; and a is 1 or 2.

[13] A liquid crystal composition comprising, as a first component, at least one liquid crystalline compound recited in any one of [1] to [10] above, and comprising, as a second component, at least one compound selected from the group consisting of the compounds expressed by any one of the general formulas (5), (6), (7), (8), and (9)

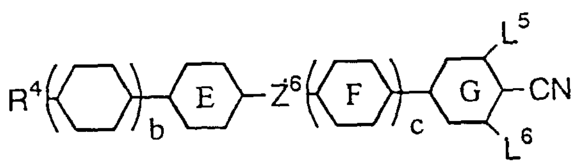

(5)

wherein $R^4$ represents F, an alkyl group having 1 to 10 carbon atoms, or an alkenyl group having 2 to 10 carbon

atoms any methylene group ($-CH_2-$) in the alkyl or alkenyl group may be replaced by oxygen atom (-O-), but in no case two or more methylene groups are continuously replaced by oxygen atom; ring E represents trans-1,4-cyclohexylene group, 1,4-phenylene group, pyrimidine-2,5-diyl group, or 1,3-dioxane-2,5-diyl group; ring F represents trans-1,4-cyclohexylene group, 1,4-phenylene group, or pyrimidine-2,5-diyl group; ring G represents trans-1,4-cyclohexylene group or 1,4-phenylene group; $Z^6$ represents $-(CH_2)_2-$, -COO-, or single bond; $L^5$ and $L^6$ independently represent H or F; b and c are independently 0 or 1,

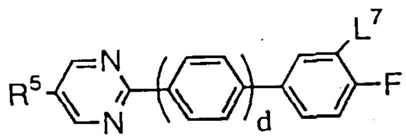

(6)

wherein $R^5$ represents an alkyl group having 1 to 10 carbon atoms; $L^7$ represents H or F; and d is 0 or 1,

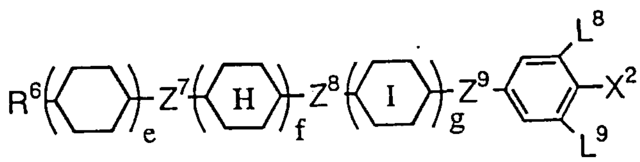

(7)

wherein $R^6$ represents an alkyl group having 1 to 10 carbon atoms; ring H and ring I independently represent trans-1,4-cyclohexylene group or 1,4-phenylene group; $Z^7$ and $Z^8$ independently represent -COO- or single bond; $Z^9$ represents -COO- or -C≡C-; $L^8$ and $L^9$ independently represent H or F; $X^2$ represents F, $OCF_3$, $OCF_2H$, $CF_3$, $CF_2H$, or $CFH_2$ provided that when $X^2$ represents $OCF_3$, $OCF_2H$, $CF_3$, $CF_2H$, or $CFH_2$, both $L^8$ and $L^9$ represent H; and e, f, and g are independently 0 or 1,

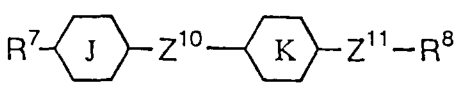

(8)

wherein $R^7$ and $R^8$ independently represent an alkyl group having 1 to 10 carbon atoms or an alkenyl group having 2 to 10 carbon atoms any methylene group ($-CH_2-$) in the groups may be replaced by oxygen atom (-O-), but in no case two or more methylene groups are continuously replaced by oxygen atom; ring J represents trans-1,4-cyclohexylene group, 1,4-phenylene group, or pyrimidine-2,5-diyl group; ring K represents trans-1,4-cyclohexylene group or 1,4-phenylene group; $Z^{10}$ represents -C≡C-, -COO-, $-(CH_2)_2-$, -CH=CH-C≡C-, or single bond; and $Z^{11}$ represents -COO- or single bond

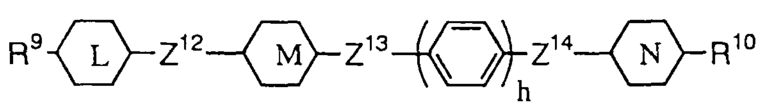

(9)

wherein $R^9$ and $R^{10}$ independently represent an alkyl group having 1 to 10 carbon atoms or an alkenyl group having 2 to 10 carbon atoms any methylene group ($-CH_2-$) in the groups may be replaced by oxygen atom (-O-), but in no case two or more methylene groups are continuously replaced by oxygen atom; ring L represents trans-1,4-cyclohexylene group, 1,4-phenylene group, or pyrimidine-2,5-diyl group; ring M represents trans-1,4-cyclohexylene group, 1,4-phenylene group one or more hydrogen atoms on the ring may be replaced by F, or pyrimidine-2,5-diyl group; ring N represents trans-1,4-cyclohexylene group or 1,4-phenylene group; $Z^{12}$ and $Z^{14}$ independently represent -COO-, $-(CH_2)_2-$, or single bond; $Z^{13}$ represents -CH=CH-, -C≡C-, -COO-, or single bond; and h is 0 or 1.

[14] A liquid crystal composition comprising, as a first component, at least one liquid crystalline compound recited

in in any one of [1] to [10] above, comprising, as a part of a second component, at least one compound selected from the group consisting of the compounds expressed by any one of the general formulas (2), (3), and (4), and comprising, as another part of the second component, at least one compound selected from the group consisting of the compounds expressed by any one of the general formulas (5), (6), (7), (8), and (9).

[15] A liquid crystal display device comprising the liquid crystal composition defined in any one of [11] to [14] above.

The compounds of the present invention expressed by the general formula (1) are remarkably stable against the outside environment, are considerably low in viscosity, and exhibit a low threshold voltage which is considered to be caused by their elastic constant. The compounds of the present invention are excellent in miscibility with other liquid crystal compounds, particularly in miscibility at low temperatures. The compounds of the present invention expressed by the general formula (1), wherein an alkyl group was selected as substituent $R^1$ and one of the groups other than CN group was selected as X, exhibit a high voltage holding ratio and exhibit preferable characterisitcs as liquid crystal materials for TFT mode display devices, particularly for a low voltage driving or high response speed, while the compounds of the present invention of formula (1), wherein an alkenyl group was selected as substituent $R^1$ and CN group was selceted as X, exhibit remarkably low viscosity or a high clearing point, and thus are very useful as viscosity reducing agent for STN mode display devices. Further, the compounds of the present invention have low dielectric anisotropy even when compared with the known compounds discussed in the section of the BACKGROUND ART. Since the compounds of the present invention have a small melucular polarity, they are extremely small in the intake of ionic impurities which becomes a problem particularly in TFT mode, and the compounds exhibit a stabilized high voltage holding ratio having small dependency on temperture.

By using the compounds of the present invention, novel liquid crystal compositions and liquid crystal display devices which are stable against the changes in the outside environment, and capable of being driven at low voltage and capable of response at a high speed, can be provided.

As will be understood from the Comparative Example in Examples, excellent characterisitcs, particularly low viscosity and low threshold voltage which is considered to attributable to small elastic constant of the compounds of the present invention are caused by oxydifluoromethylene group ($-OCF_2-$) which is a central bonding group inserted between two 1,4-phenylene groups.

Compounds having no central bonding group and the compounds having a saturated alkylene or oxymethylene group as a central bonding group have alredy been disclosed in DE 4,027,840 A1 and US 5,032,313 as explained in the section of BACKGROUND ART. However, those compounds have, as liquid crystal material, such defects that 1) they tend to take in impurities from the outside environment based on their large molecular polarity and 2) they exhibit comparatively high viscosity. With respect to the compounds which are disclosed in Laid-open Japanese Patent Publication No. Hei 6-211711 or Laid-open Japanese Patent Publication No. Hei 6-192142, and elatic constant of which is controlled, such a defect as described in 2) above and such a defect that 3) the stability (that of voltage holding ratio at high temperatures) against the changes in the outside environment is low remain unsolved.

The compounds of the present invention can be used for producing liquid crystal compositions satisfying various purposes, by properly selecting $R^1$, rings A, B, C, and D, $Z^1$, $Z^2$, $Z^3$, $\ell$, and m in the formula (1).

That is, when the compounds are used for liquid crystal compositions which must have the temperature range of liquid crystal phase particularly at high temperature side, it is sufficient to use a four-ring system compound wherein $\ell$ or m is 1, and when not so, it is sufficient to use a three-ring system compound.

When a particularly high voltage holding ratio is required for liquid crystal compositions for active matrix or the likes, it is sufficient to select an alkyl group for side chain $R^1$ and select one of the groups other than CN group for substituent X. When the compositions are used as viscosity reducing agent for STN, for example, it is sufficient to select an alkenyl group as lateral chain $R^1$.

In order to obtain compounds having comparatively high dielectric anisotropy, it is sufficient to select a halogen atom for substituent Y and selcect CN, $CF_3$, $OCF_3$, or $OCHF_2$ for substituent X. When still higher dielectric anisotropy is required, the purpose can be accomplished by substituting one or two fluorine atoms at the ortho position of oxo side phenyl ring (ring C) of oxydifluoromethylene group which is a central bonding group so that the dipoles faces the same direction. Further, the compounds wherein fluorine atom substituted at lateral position of ring A, ring B, or ring C are characterized by having excellent miscibility, particularly excellent miscibility at low temperatures compared with the compounds having no substituted fluorine atom.

Optical anisotropy can also be optionally adjusted by properly selecting $R^1$, ring A, ring C, $Z^1$, $Z^2$, X, and m in the formula (1). That is, when a large optical anisotropy value is necessary, it is sufficient to select compounds which contain many 1,4-phenylene rings and have single bond as $Z^1$ and $Z^2$, or chlorine atom as substituent X. When a small anisotropy value is necessary, it is sufficient to select compounds containing many trans-1,4-cyclohexylene rings.

The term "alkyl group" as used in this specification means a straight chain or branched alkyl group having 1 to 15 carbon atoms, and the alkyl groups having 1 to 5 carbon atoms are preferable particularly from the viewpoint of low viscosity. Specifically, methyl group, ethyl group, propyl group, butyl group, pentyl group, hexyl group, heptyl group, octyl

group, nonyl group, isopropyl group, isobutyl group, isoamyl group, isohexyl group, 2-methylbutyl group, 2-methylpentyl group, and 3-methylpentyl group are preferable, and racemic compounds, S isomers, and R isomers are comprehended.

The term "alkenyl group" as used in this specification means a straight chain alkenyl group having 2 to 15 carbon atoms, and 1E-alkenyl, 2Z-alkenyl, 3E-alkenyl, and 4-alkenyl group are preferable. Specifically, 1-ethenyl, 1E-propenyl, 1E-butenyl, 1E-hexenyl, 2-propenyl, 2Z-butenyl, 2Z-pentenyl, 2Z-hexenyl, 3-butenyl, 3E-pentenyl, and 3E-hexenyl group can be mentioned.

As ring A, benzene ring, cyclohexane ring, pyrimidine ring, pyridine ring, pyrazine ring, pyridazine ring, dioxane ring, dithian ring, and those rings substituted with a halogen atom can be mentioned; and benzene ring, cyclohexane ring, and these rings substituted with halogen atom(s) are especially preferable.

BRIEF DESCRIPTION OF THE DRAWING

Fig. 1 shows a measured chart of $^{19}F$-NMR spectrum of Compound No. 1 of the present invention.

BEST MODE FOR CARRYING OUT THE INVENTION

In the first aspect of the present invention, preferable embodiments of alkylcyclohexane derivatives expressed by the general formula (1) are compounds expressed by one of the following general formulas (1-a) to (1-c):

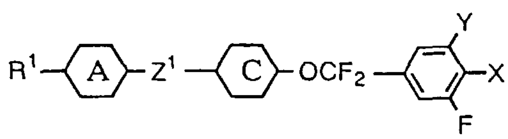

(1-a)

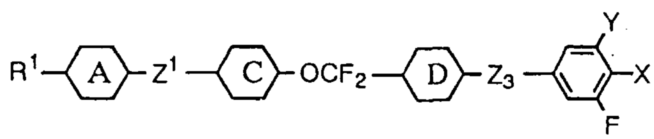

(1-b)

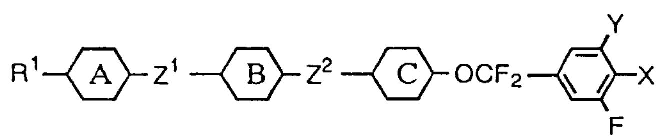

(1-c)

wherein $R^1$, ring A, ring B, ring C, ring D, $Z^1$, $Z^2$, and $Z^3$ have the same meaning as described above.

More preferable compounds are ones expressed by one of the group of general formulas (1-a-1) to (1-a-108), (1-b-1) to (1-b-144), and (1-c-1) to (1-c-108):

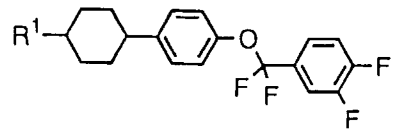

(1-a-1)

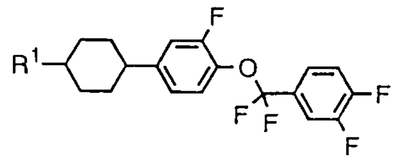

(1-a-2)

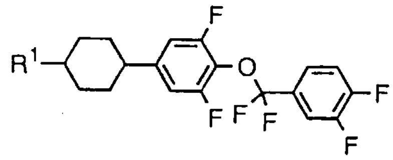

(1-a-3)

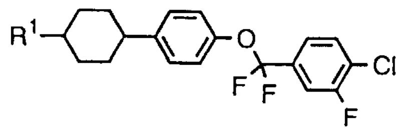

(1-a-4)

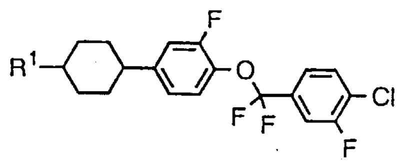

(1-a-5)

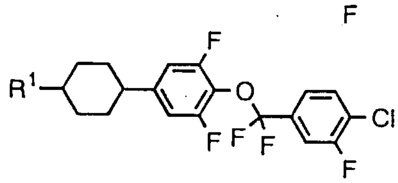

(1-a-6)

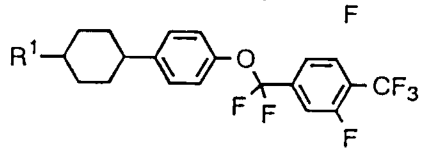

(1-a-7)

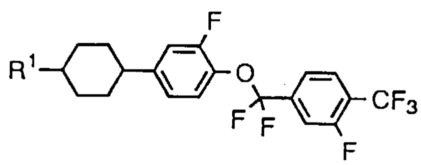

(1-a-8)

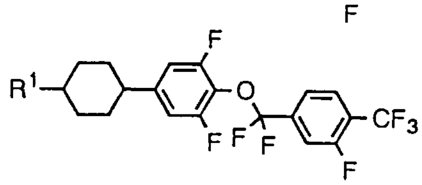

(1-a-9)

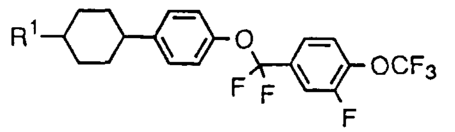 (1-a-10)

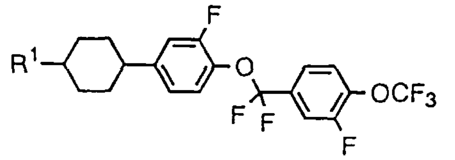 (1-a-11)

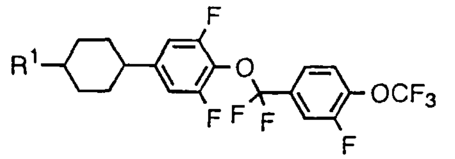 (1-a-12)

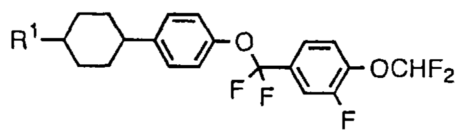 (1-a-13)

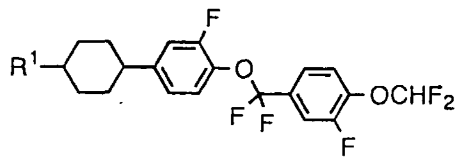 (1-a-14)

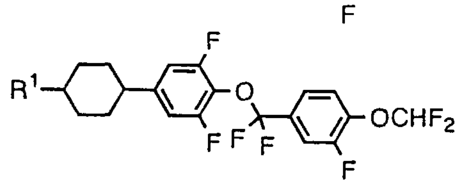 (1-a-15)

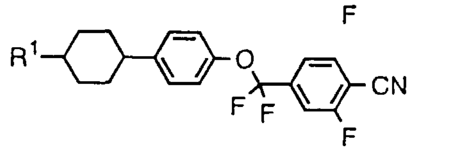 (1-a-16)

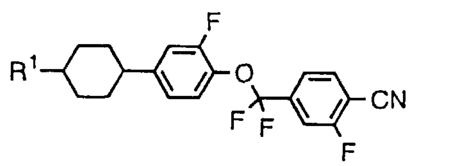 (1-a-17)

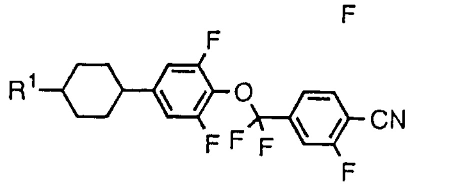 (1-a-18)

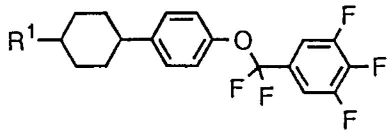

(1-a-19)

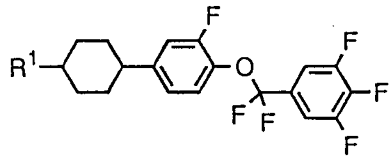

(1-a-20)

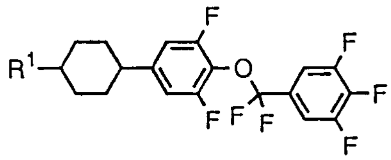

(1-a-21)

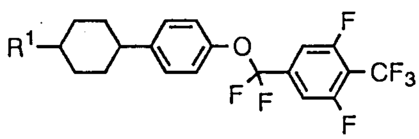

(1-a-22)

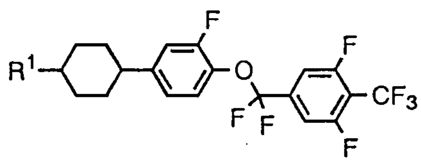

(1-a-23)

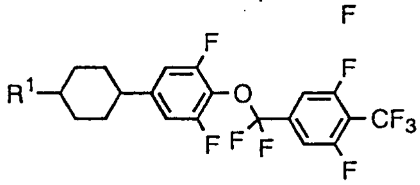

(1-a-24)

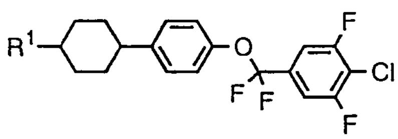

(1-a-25)

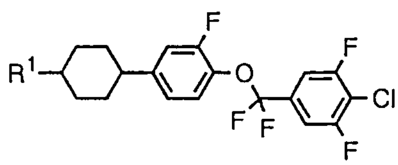

(1-a-26)

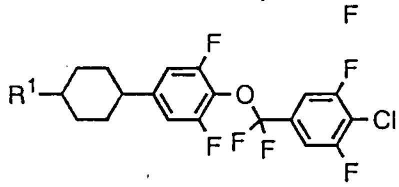

(1-a-27)

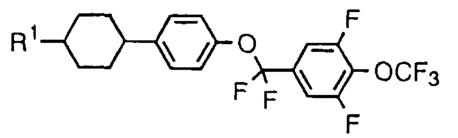 (1-a-28)

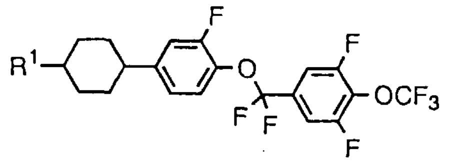 (1-a-29)

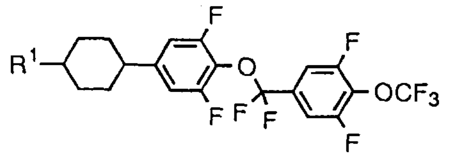 (1-a-30)

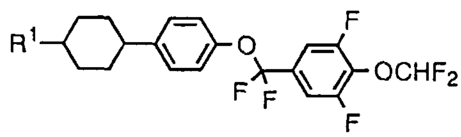 (1-a-31)

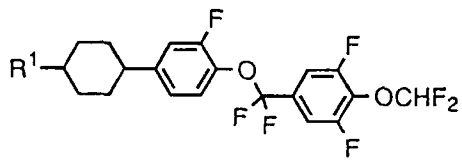 (1-a-32)

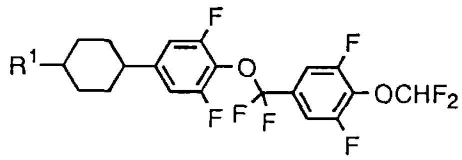 (1-a-33)

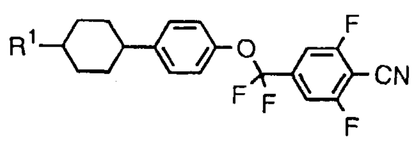 (1-a-34)

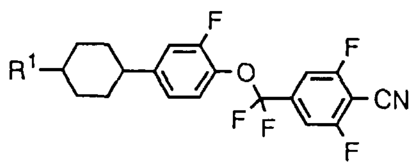 (1-a-35)

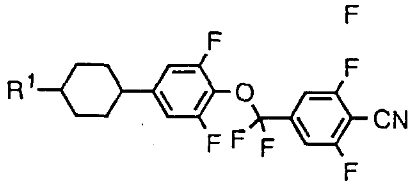 (1-a-36)

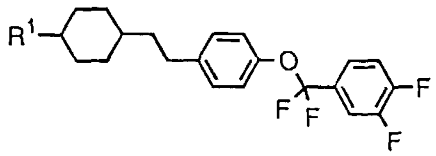

(1-a-37)

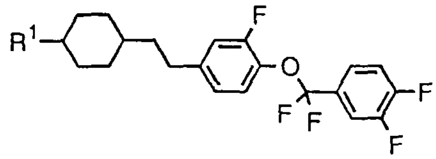

(1-a-38)

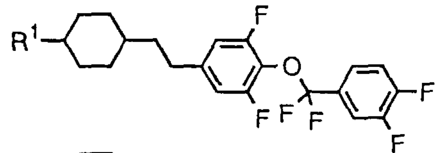

(1-a-39)

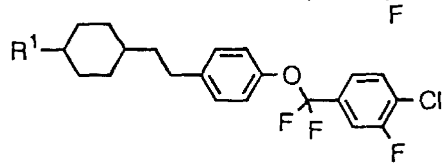

(1-a-40)

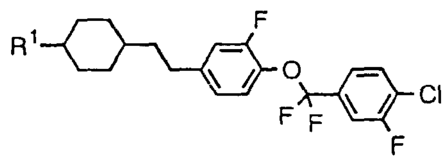

(1-a-41)

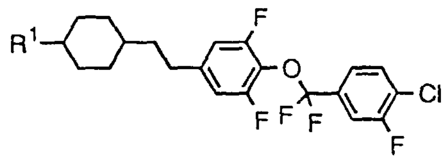

(1-a-42)

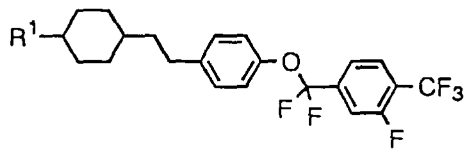

(1-a-43)

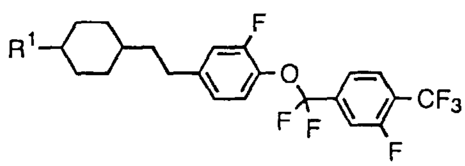

(1-a-44)

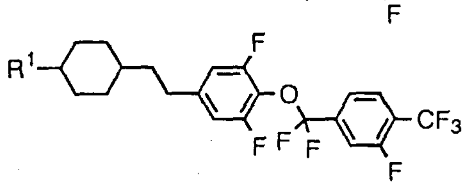

(1-a-45)

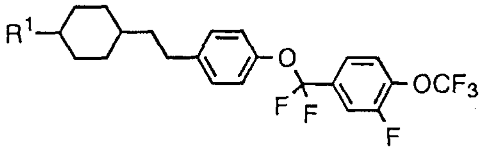

(1-a-46)

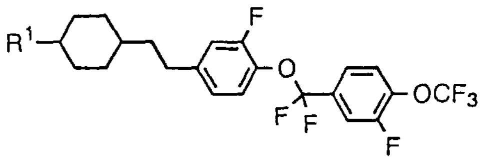

(1-a-47)

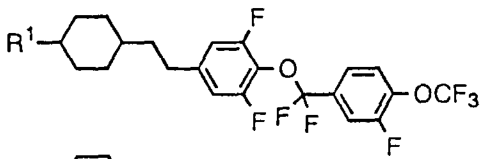

(1-a-48)

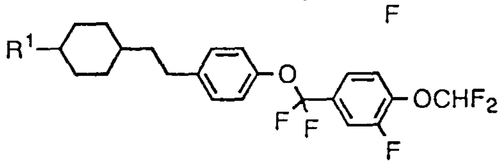

(1-a-49)

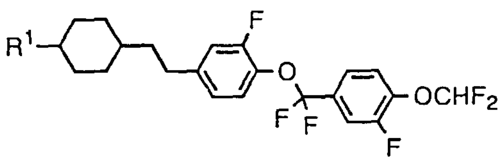

(1-a-50)

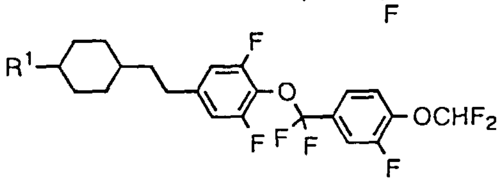

(1-a-51)

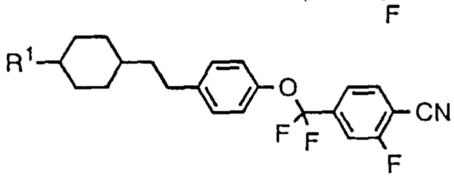

(1-a-52)

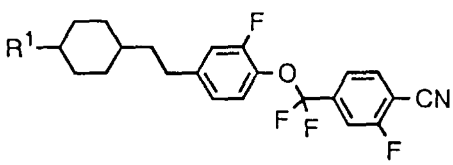

(1-a-53)

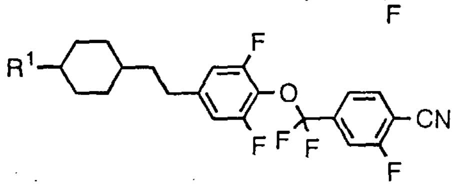

(1-a-54)

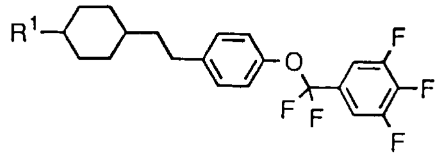

(1-a-55)

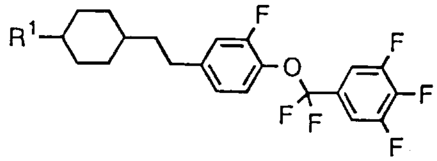

(1-a-56)

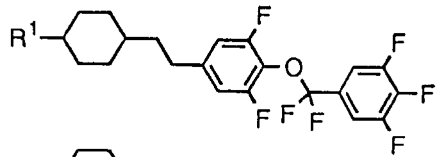

(1-a-57)

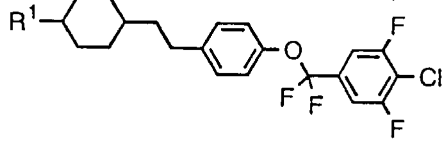

(1-a-58)

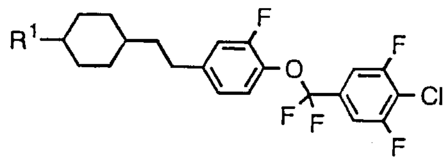

(1-a-59)

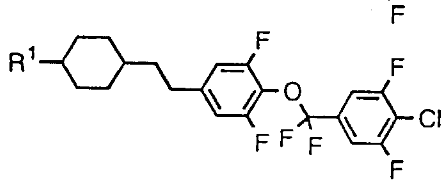

(1-a-60)

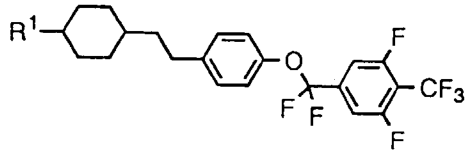

(1-a-61)

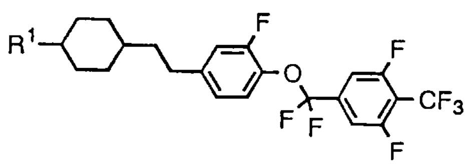

(1-a-62)

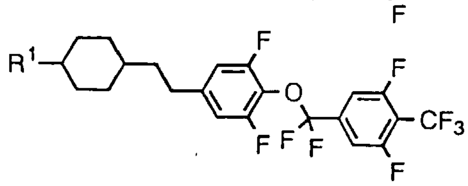

(1-a-63)

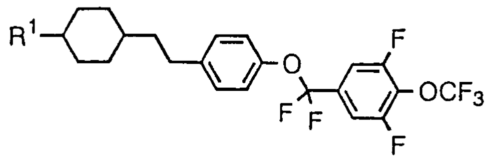

(1-a-64)

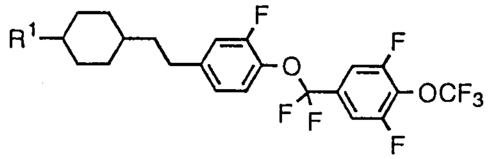

(1-a-65)

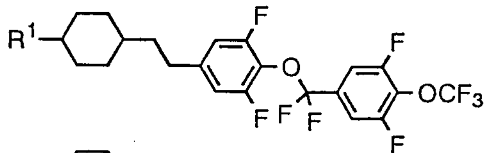

(1-a-66)

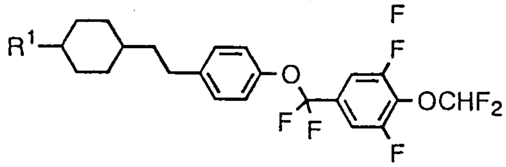

(1-a-67)

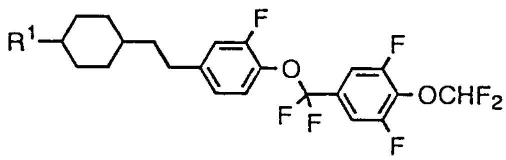

(1-a-68)

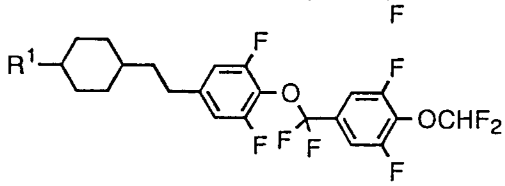

(1-a-69)

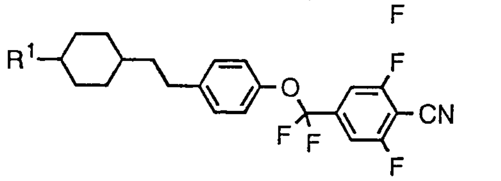

(1-a-70)

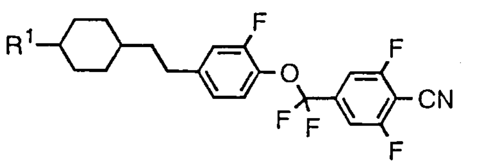

(1-a-71)

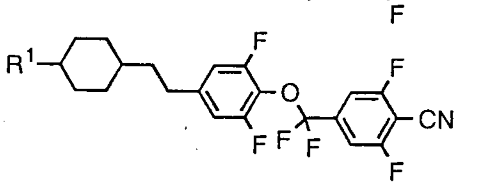

(1-a-72)

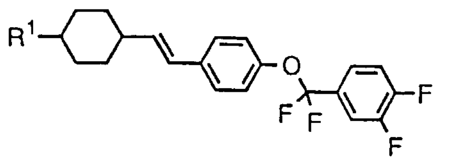
(1-a-73)

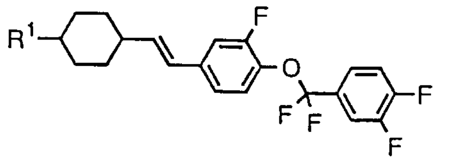
(1-a-74)

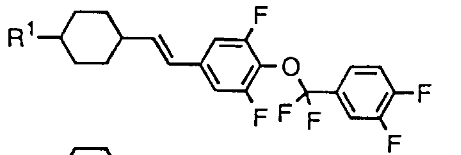
(1-a-75)

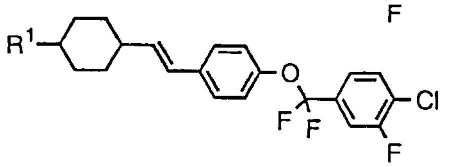
(1-a-76)

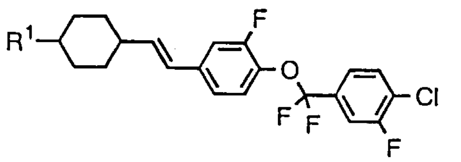
(1-a-77)

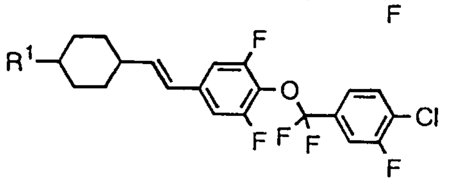
(1-a-78)

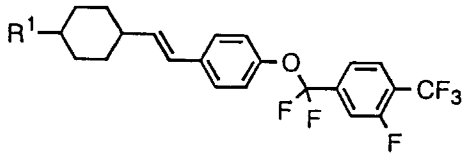
(1-a-79)

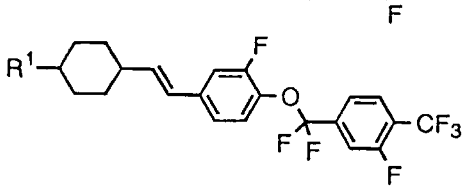
(1-a-80)

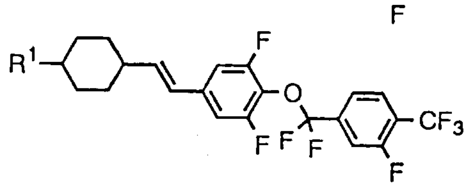
(1-a-81)

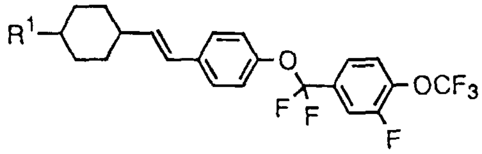
(1-a-82)

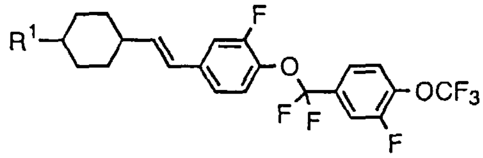
(1-a-83)

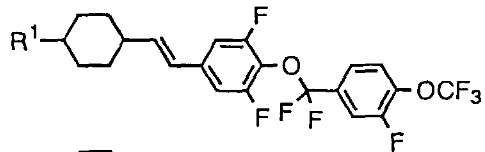
(1-a-84)

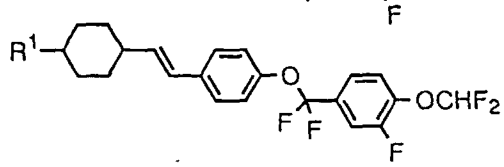
(1-a-85)

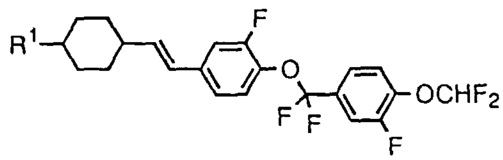
(1-a-86)

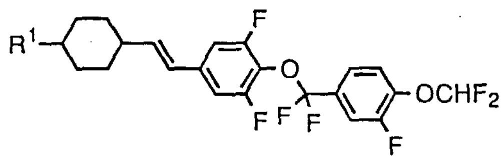
(1-a-87)

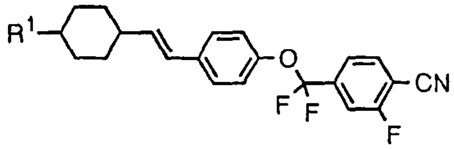
(1-a-88)

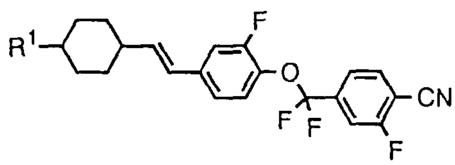
(1-a-89)

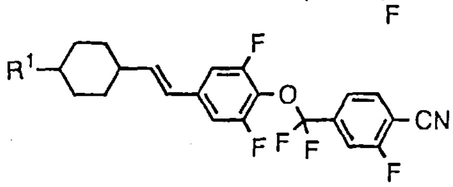
(1-a-90)

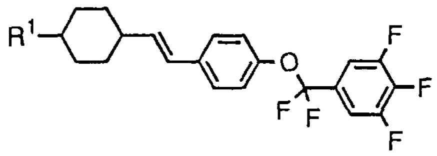
(1-a-91)

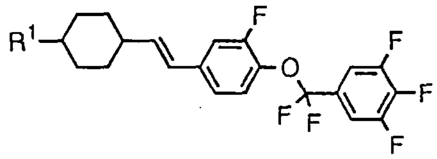
(1-a-92)

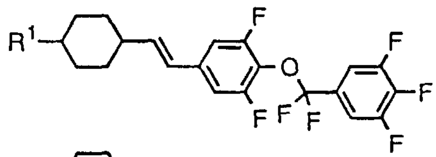
(1-a-93)

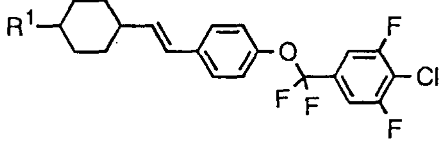
(1-a-94)

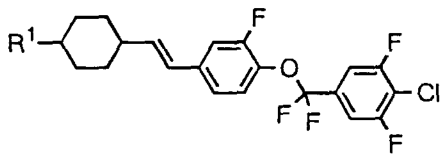
(1-a-95)

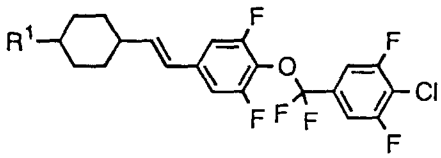
(1-a-96)

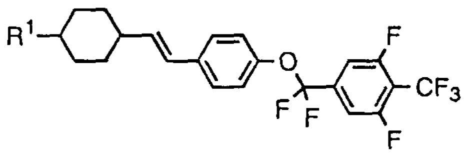
(1-a-97)

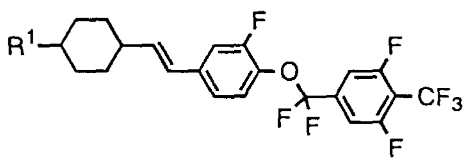
(1-a-98)

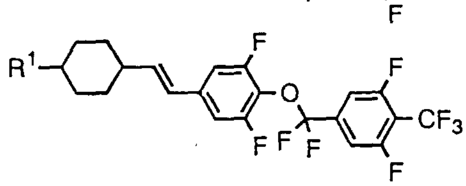
(1-a-99)

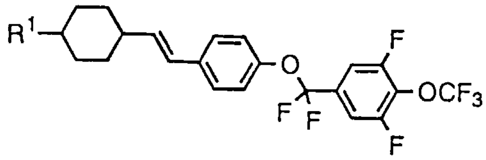
(1-a-100)

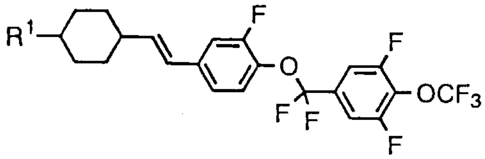
(1-a-101)

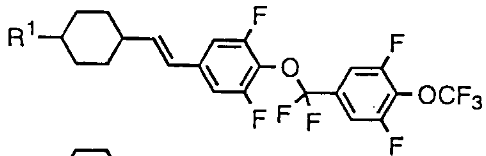
(1-a-102)

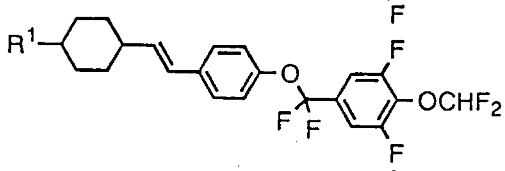
(1-a-103)

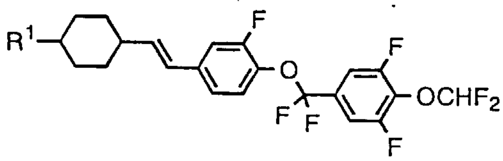
(1-a-104)

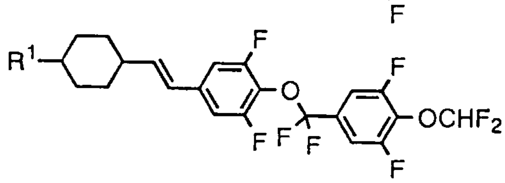
(1-a-105)

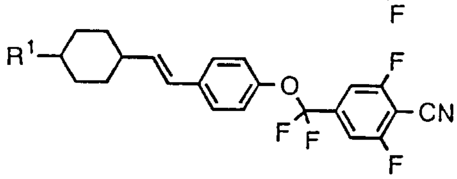
(1-a-106)

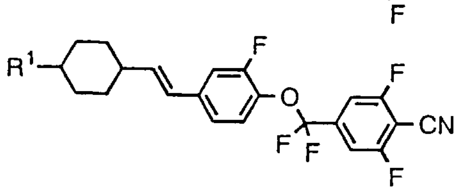
(1-a-107)

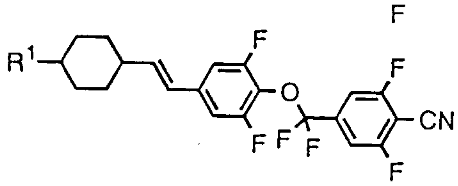
(1-a-108)

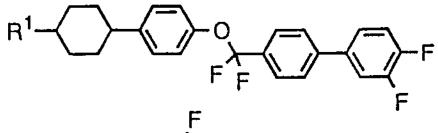
(1-b-1)

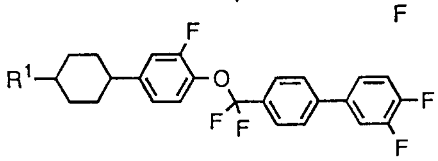
(1-b-2)

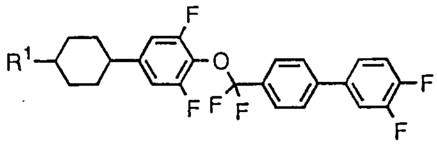
(1-b-3)

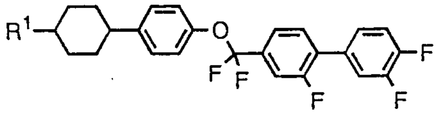
(1-b-4)

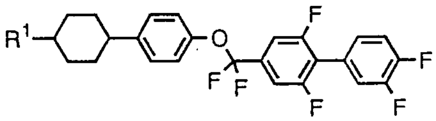
(1-b-5)

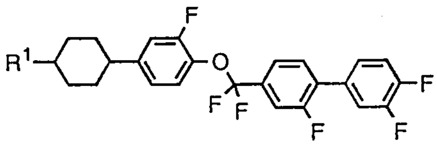
(1-b-6)

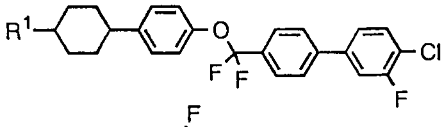
(1-b-7)

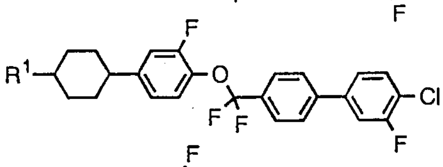
(1-b-8)

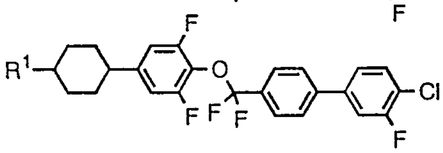
(1-b-9)

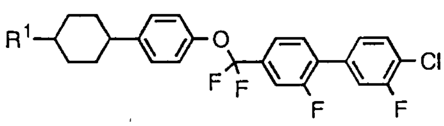
(1-b-10)

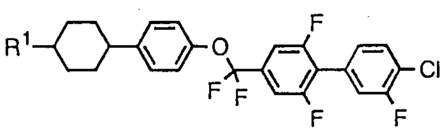
(1-b-11)

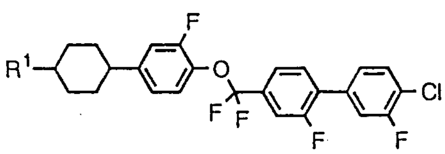
(1-b-12)

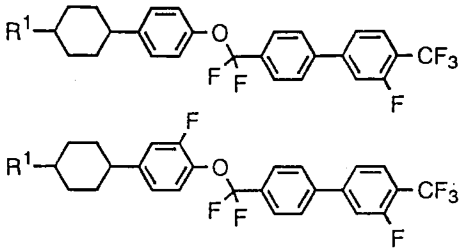

(1-b-13)

(1-b-14)

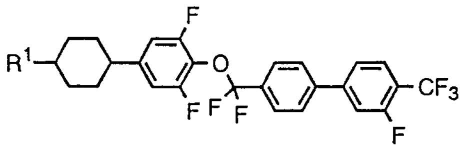

(1-b-15)

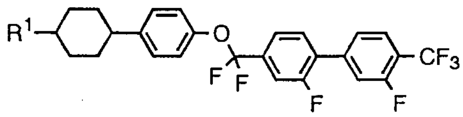

(1-b-16)

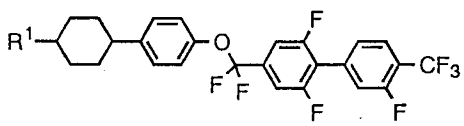

(1-b-17)

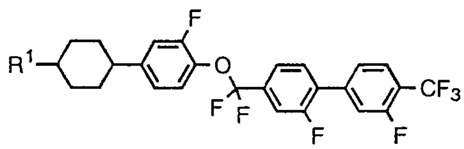

(1-b-18)

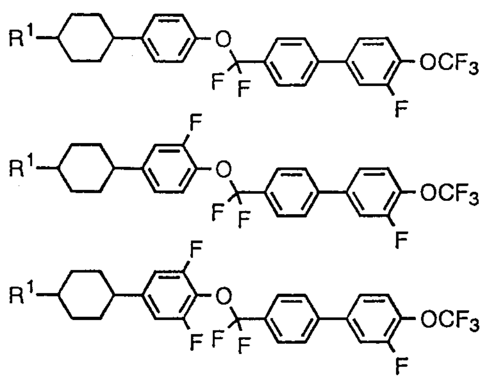

(1-b-19)

(1-b-20)

(1-b-21)

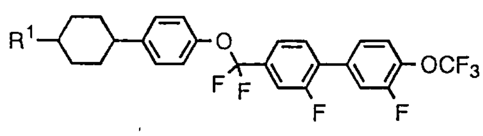

(1-b-22)

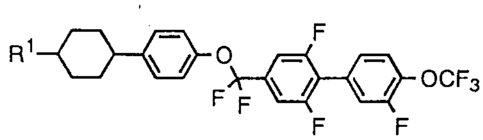

(1-b-23)

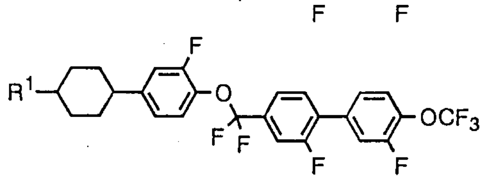

(1-b-24)

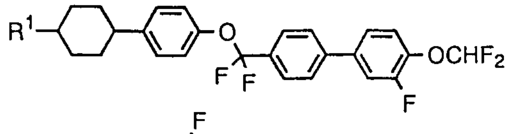
(1-b-25)

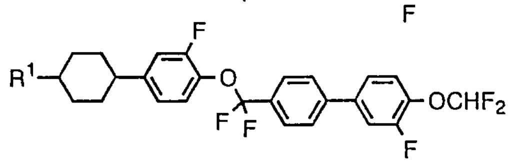
(1-b-26)

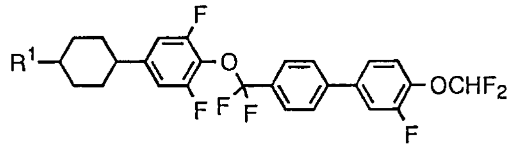
(1-b-27)

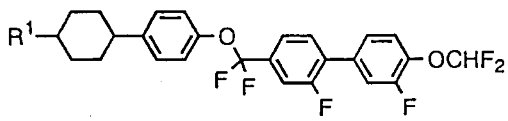
(1-b-28)

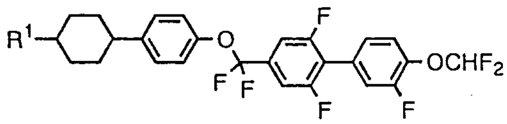
(1-b-29)

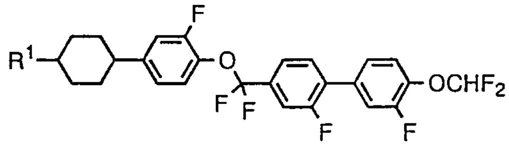
(1-b-30)

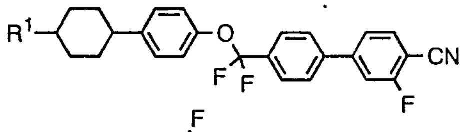
(1-b-31)

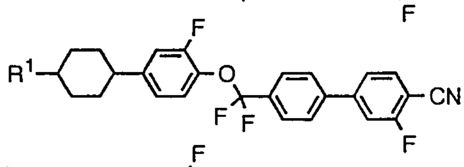
(1-b-32)

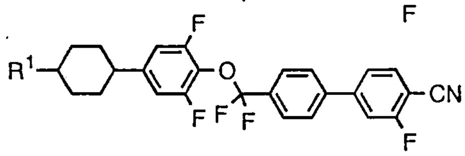
(1-b-33)

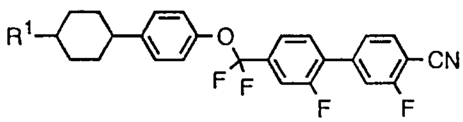
(1-b-34)

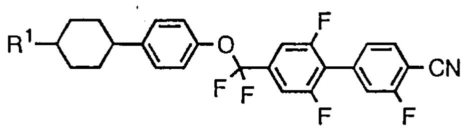
(1-b-35)

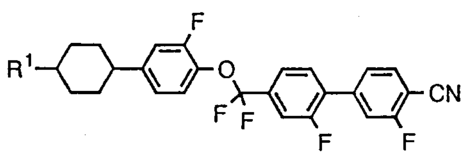
(1-b-36)

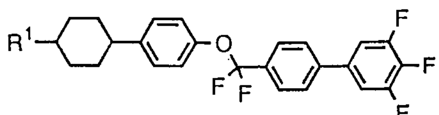
(1-b-37)

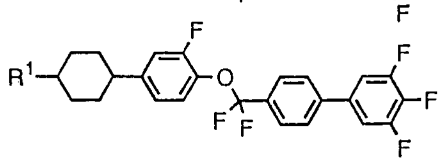
(1-b-38)

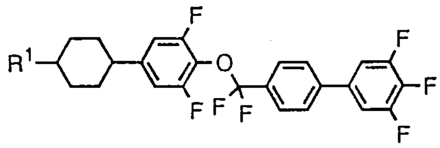
(1-b-39)

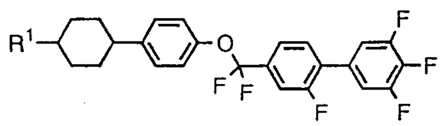
(1-b-40)

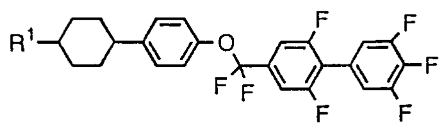
(1-b-41)

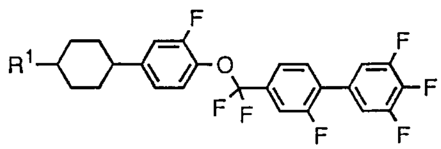
(1-b-42)

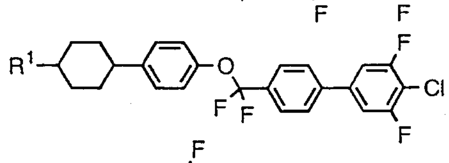
(1-b-43)

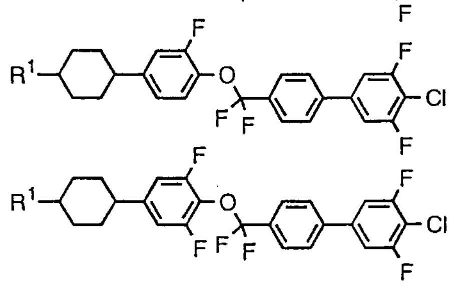
(1-b-44)

(1-b-45)

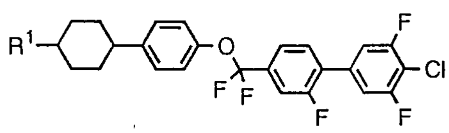
(1-b-46)

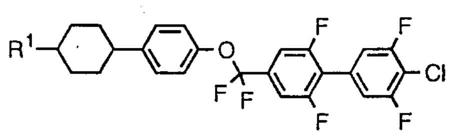
(1-b-47)

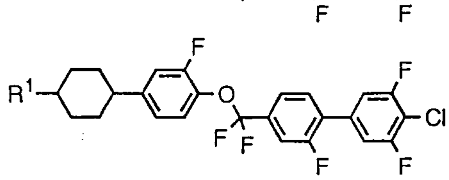
(1-b-48)

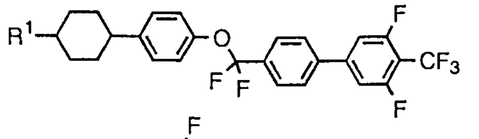

(1-b-49)

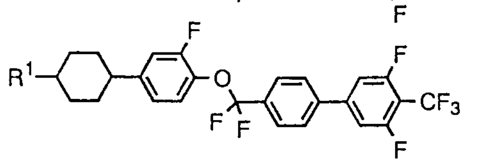

(1-b-50)

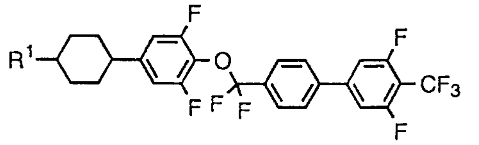

(1-b-51)

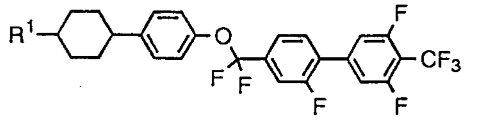

(1-b-52)

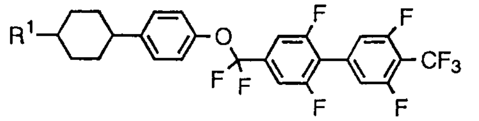

(1-b-53)

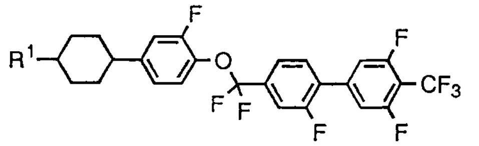

(1-b-54)

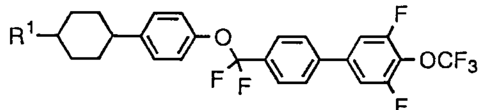

(1-b-55)

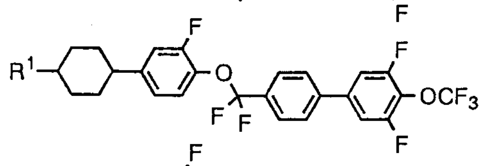

(1-b-56)

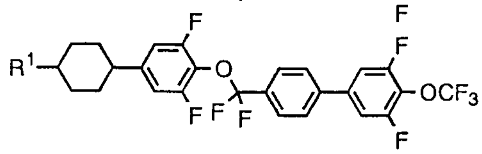

(1-b-57)

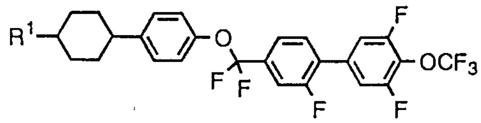

(1-b-58)

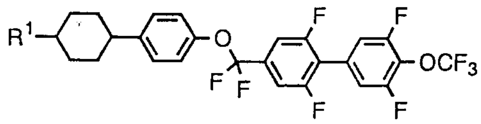

(1-b-59)

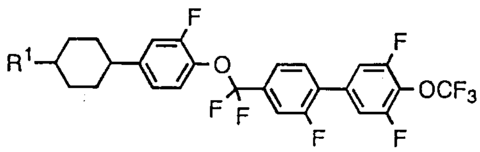

(1-b-60)

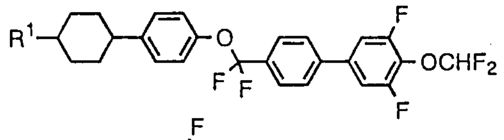 (1-b-61)

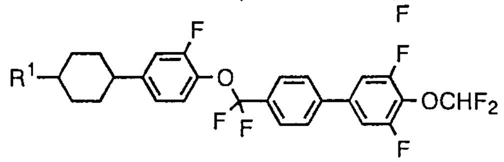 (1-b-62)

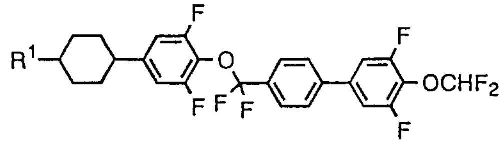 (1-b-63)

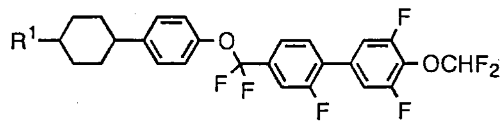 (1-b-64)

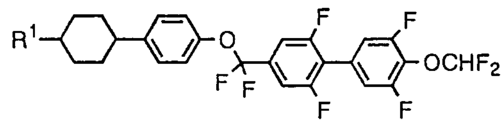 (1-b-65)

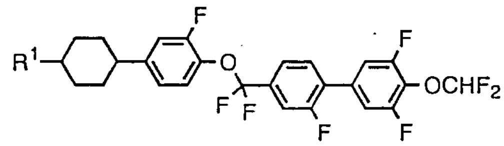 (1-b-66)

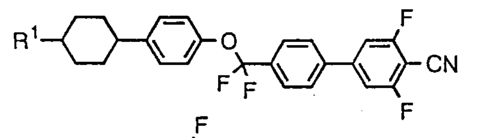 (1-b-67)

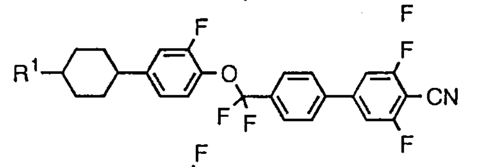 (1-b-68)

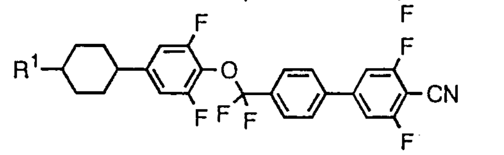 (1-b-69)

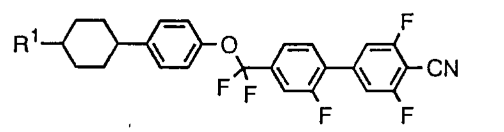 (1-b-70)

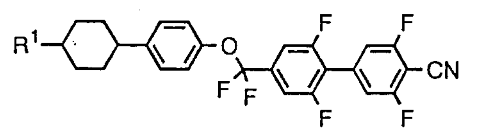 (1-b-71)

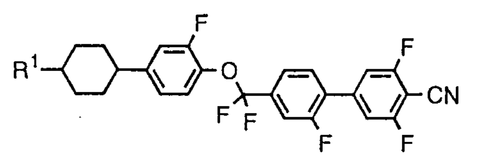 (1-b-72)

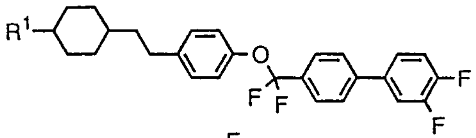
(1-b-73)

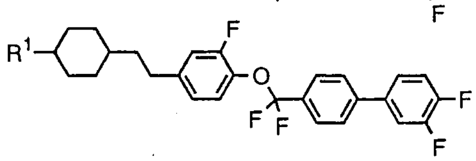
(1-b-74)

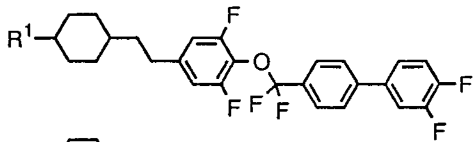
(1-b-75)

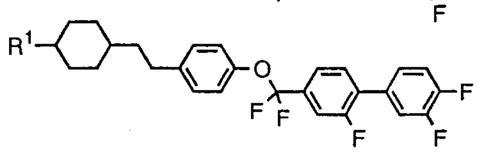
(1-b-76)

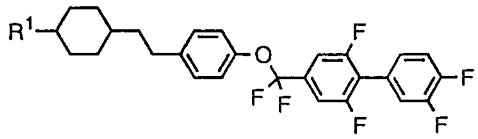
(1-b-77)

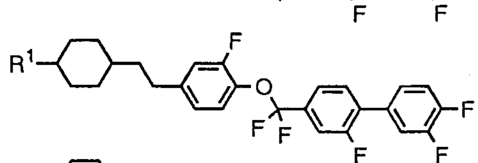
(1-b-78)

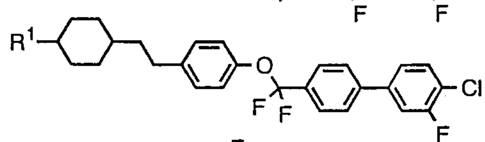
(1-b-79)

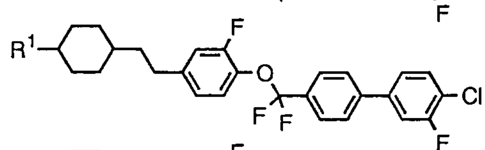
(1-b-80)

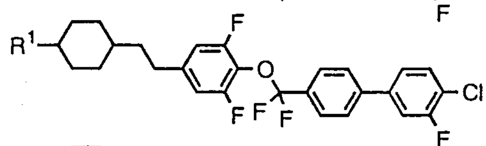
(1-b-81)

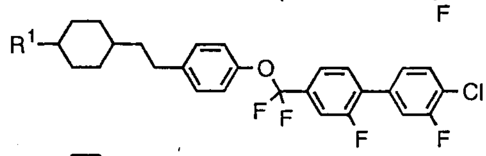
(1-b-82)

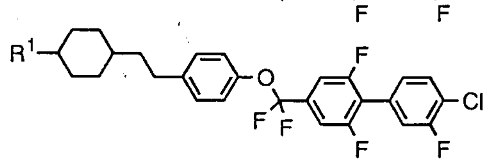
(1-b-83)

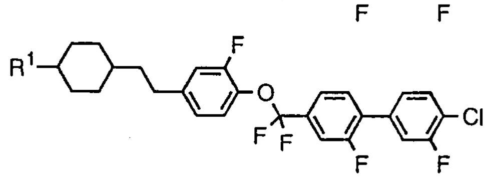
(1-b-84)

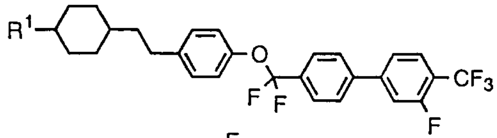
(1-b-85)

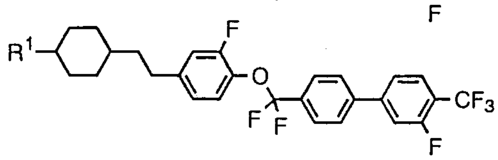
(1-b-86)

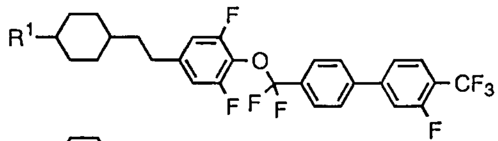
(1-b-87)

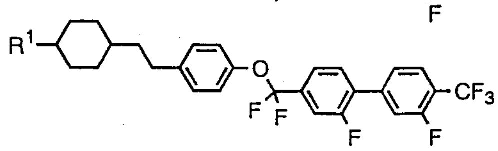
(1-b-88)

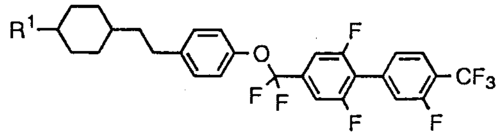
(1-b-89)

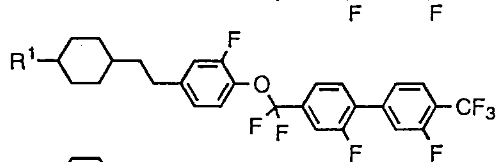
(1-b-90)

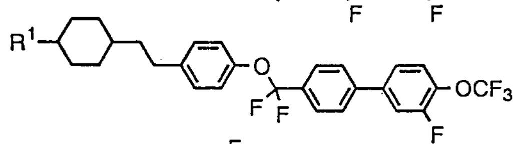
(1-b-91)

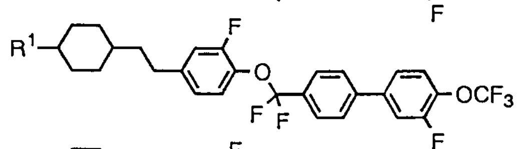
(1-b-92)

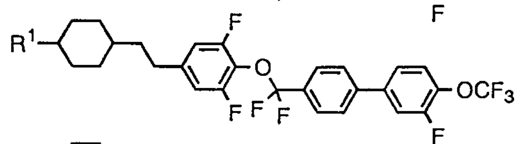
(1-b-93)

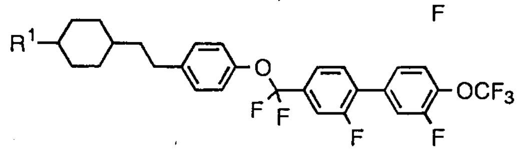
(1-b-94)

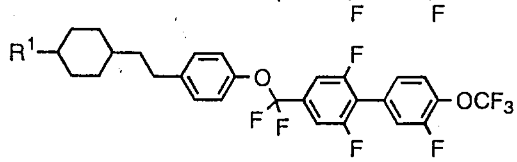
(1-b-95)

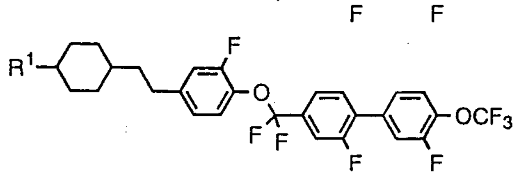
(1-b-96)

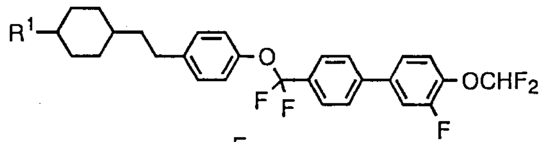
(1-b-97)

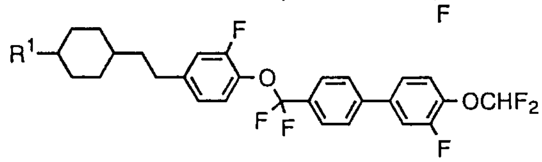
(1-b-98)

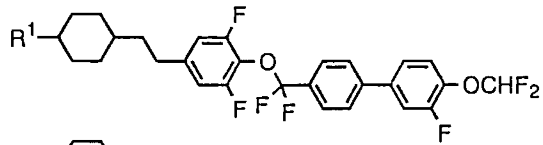
(1-b-99)

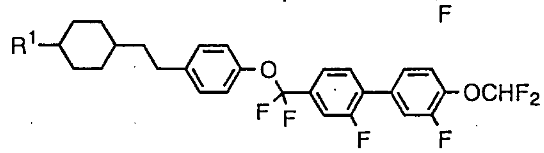
(1-b-100)

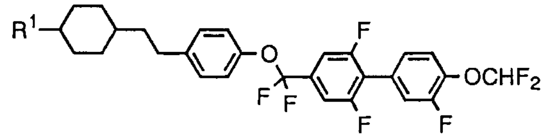
(1-b-101)

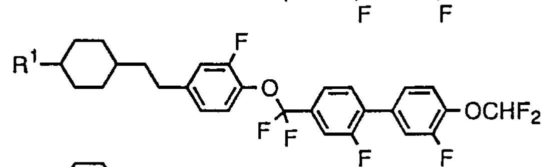
(1-b-102)

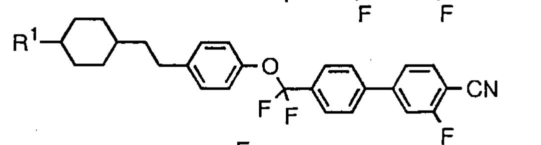
(1-b-103)

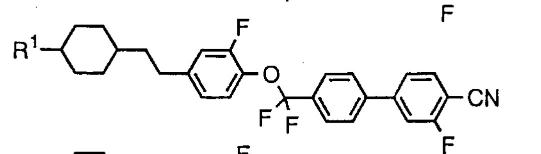
(1-b-104)

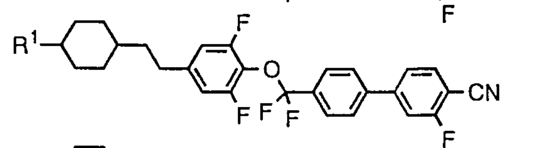
(1-b-105)

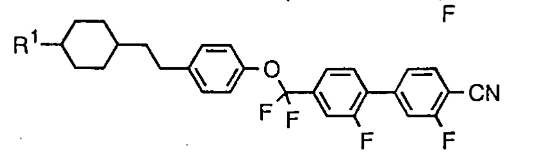
(1-b-106)

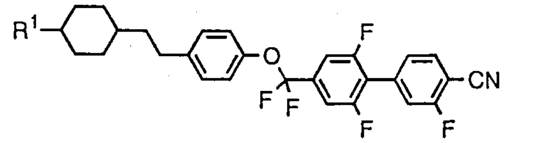
(1-b-107)

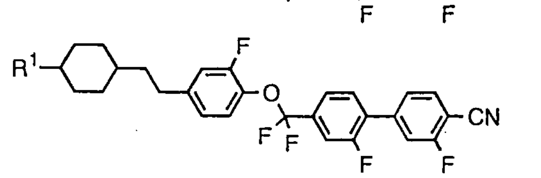
(1-b-108)

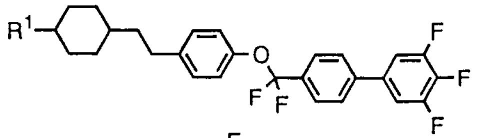
(1-b-109)

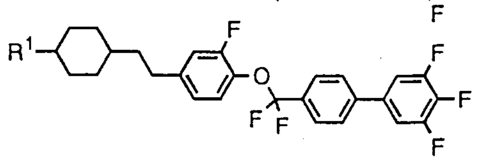
(1-b-110)

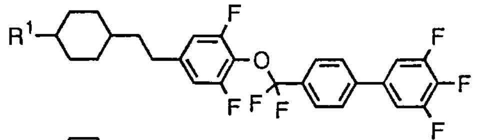
(1-b-111)

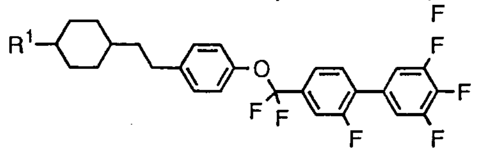
(1-b-112)

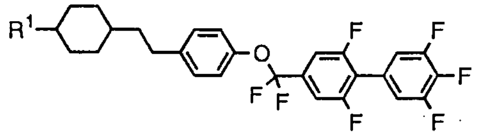
(1-b-113)

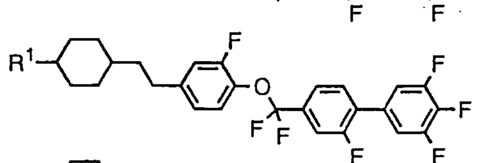
(1-b-114)

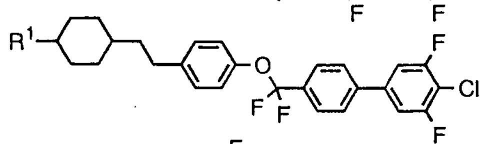
(1-b-115)

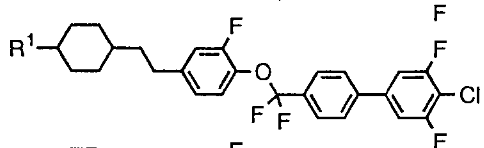
(1-b-116)

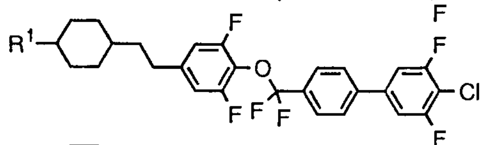
(1-b-117)

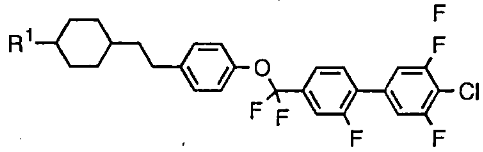
(1-b-118)

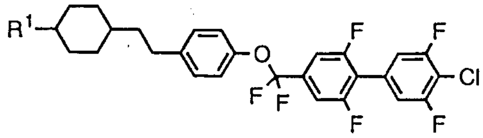
(1-b-119)

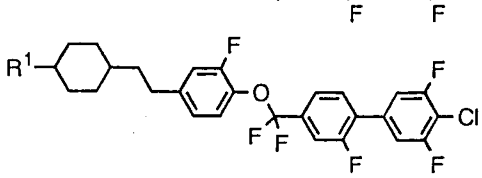
(1-b-120)

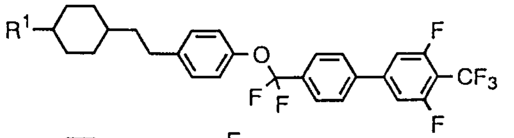

(1-b-121)

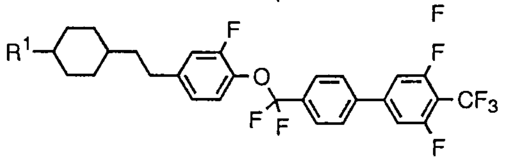

(1-b-122)

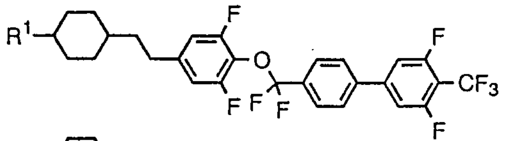

(1-b-123)

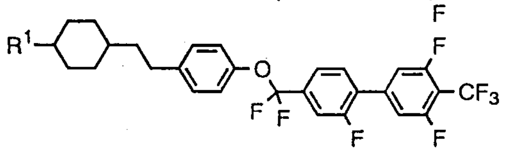

(1-b-124)

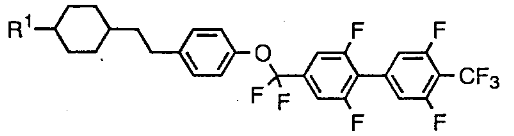

(1-b-125)

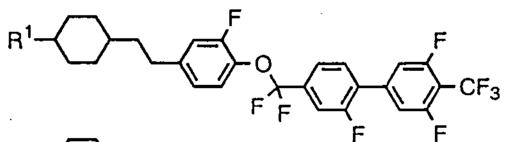

(1-b-126)

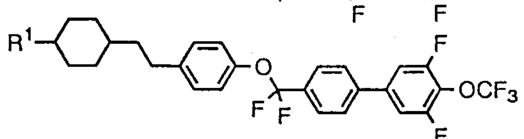

(1-b-127)

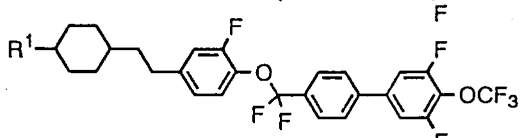

(1-b-128)

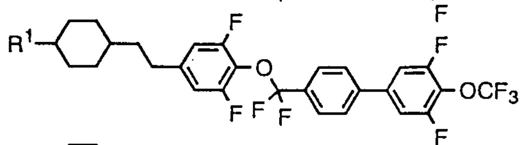

(1-b-129)

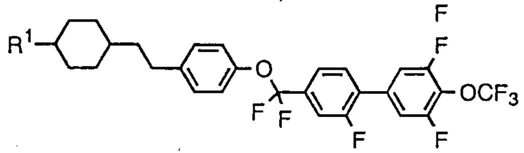

(1-b-130)

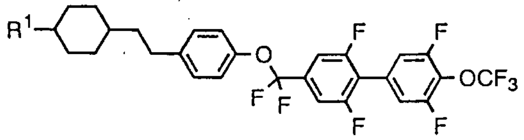

(1-b-131)

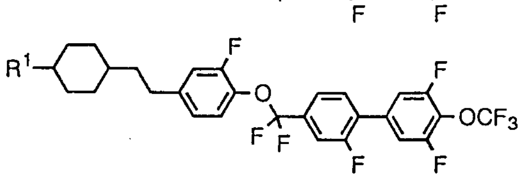

(1-b-132)

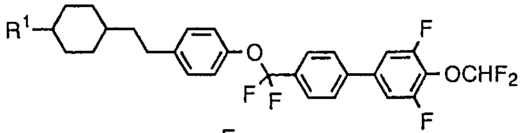
(1-b-133)

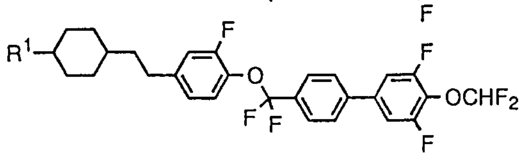
(1-b-134)

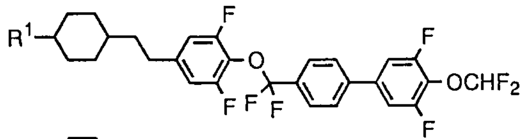
(1-b-135)

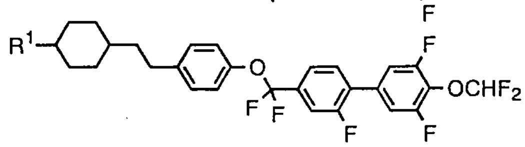
(1-b-136)

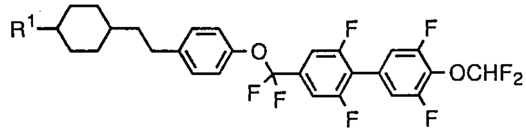
(1-b-137)

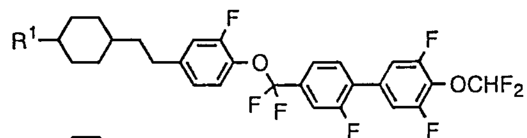
(1-b-138)

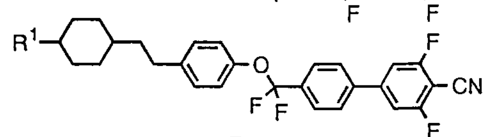
(1-b-139)

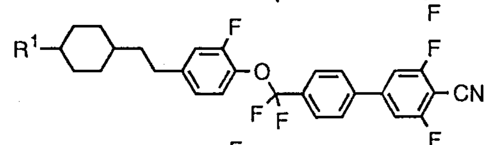
(1-b-140)

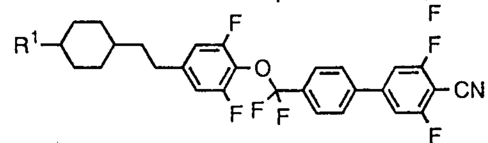
(1-b-141)

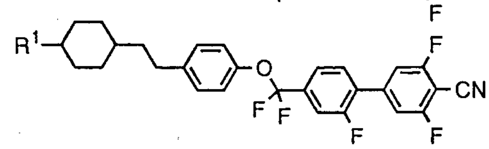
(1-b-142)

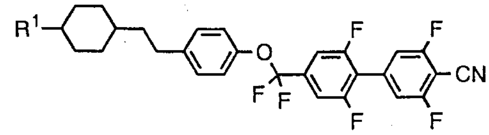
(1-b-143)

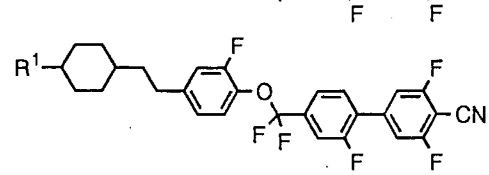
(1-b-144)

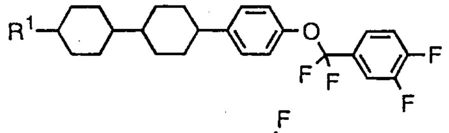
(1-c-1)

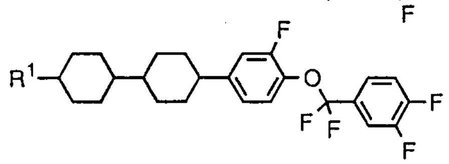
(1-c-2)

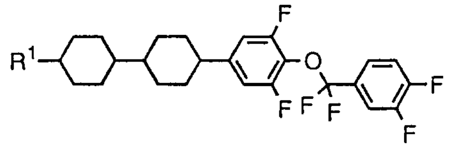
(1-c-3)

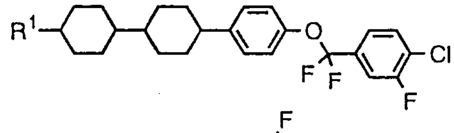
(1-c-4)

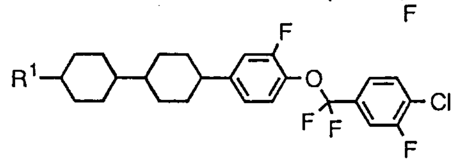
(1-c-5)

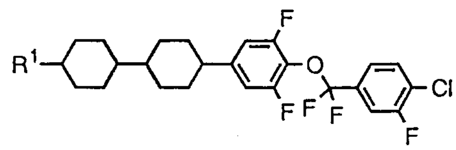
(1-c-6)

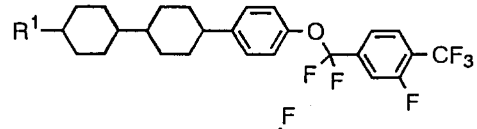
(1-c-7)

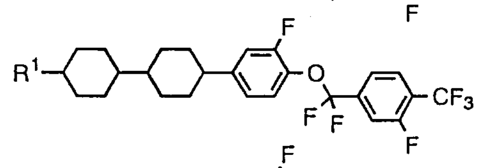
(1-c-8)

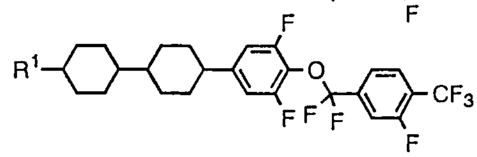
(1-c-9)

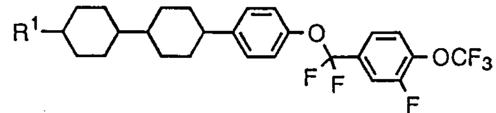
(1-c-10)

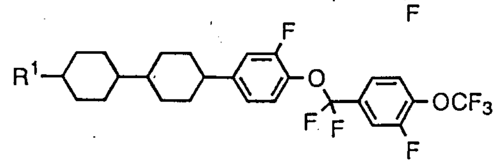
(1-c-11)

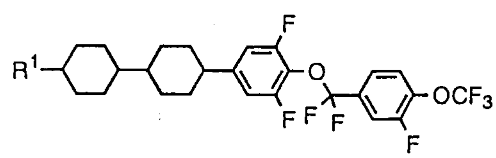
(1-c-12)

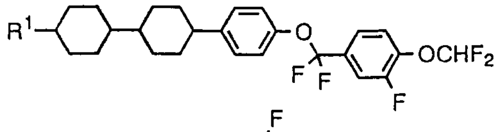
(1-c-13)

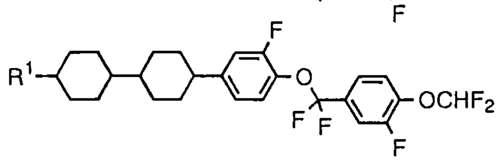
(1-c-14)

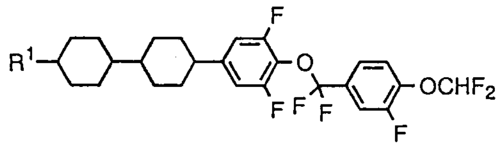
(1-c-15)

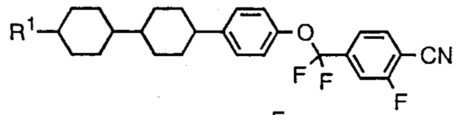
(1-c-16)

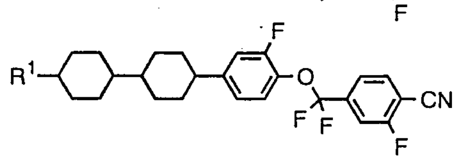
(1-c-17)

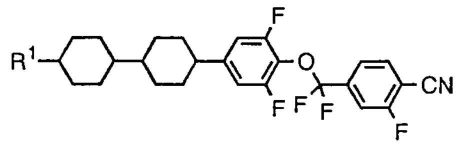
(1-c-18)

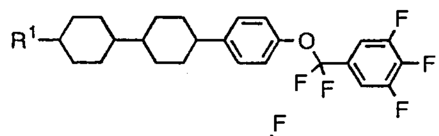
(1-c-19)

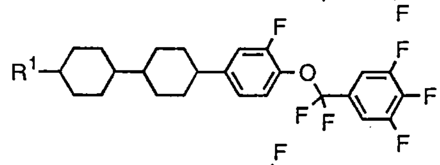
(1-c-20)

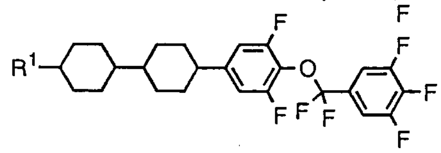
(1-c-21)

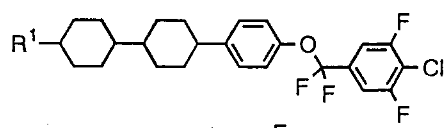
(1-c-22)

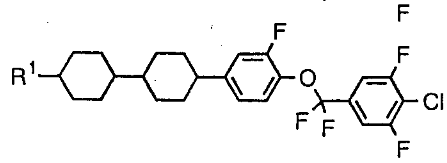
(1-c-23)

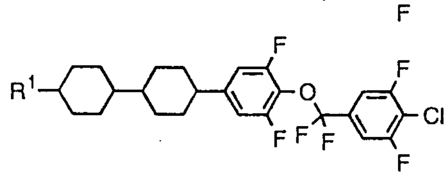
(1-c-24)

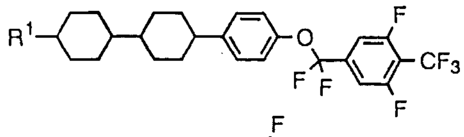

(1-c-25)

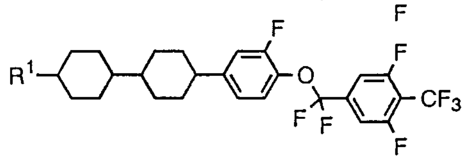

(1-c-26)

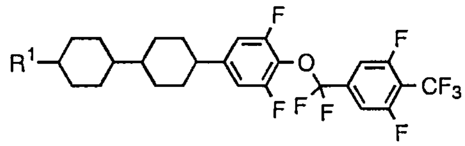

(1-c-27)

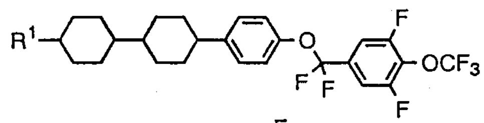

(1-c-28)

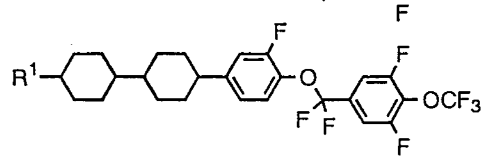

(1-c-29)

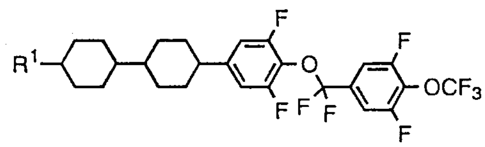

(1-c-30)

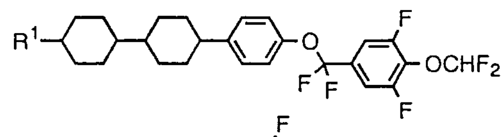

(1-c-31)

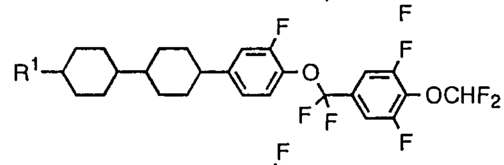

(1-c-32)

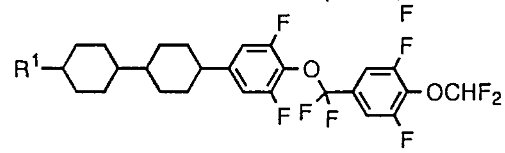

(1-c-33)

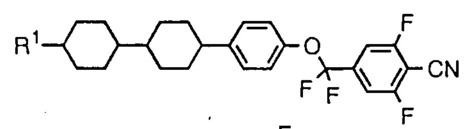

(1-c-34)

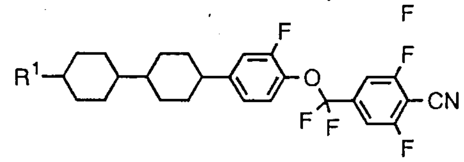

(1-c-35)

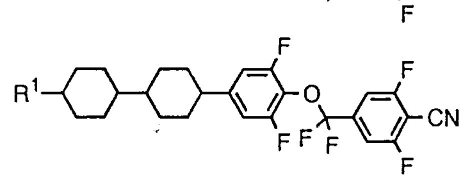

(1-c-36)

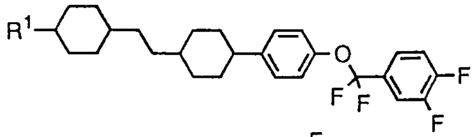
(1-c-37)

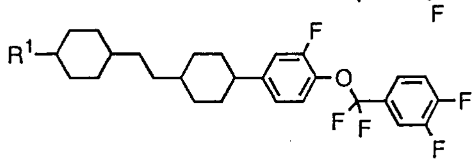
(1-c-38)

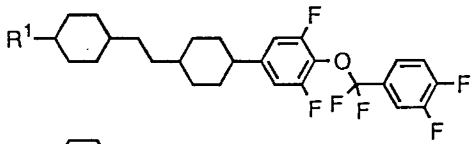
(1-c-39)

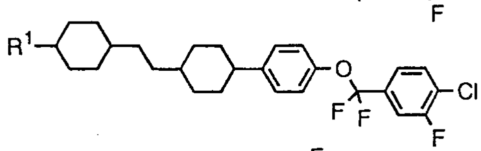
(1-c-40)

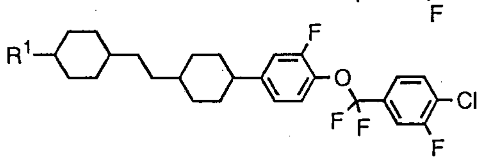
(1-c-41)

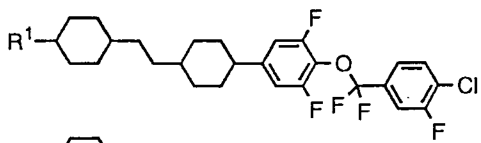
(1-c-42)

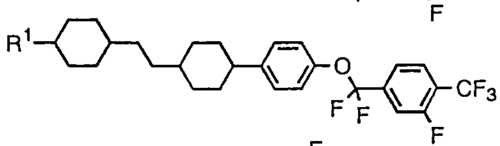
(1-c-43)

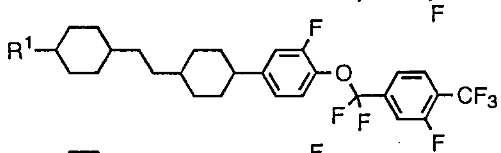
(1-c-44)

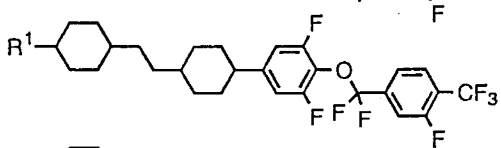
(1-c-45)

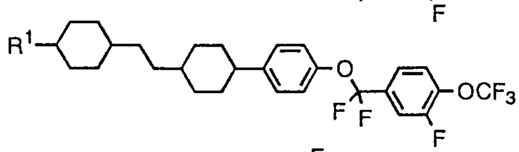
(1-c-46)

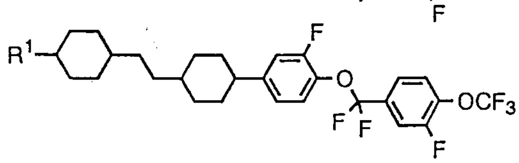
(1-c-47)

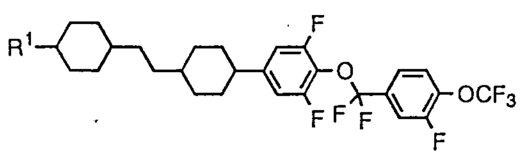
(1-c-48)

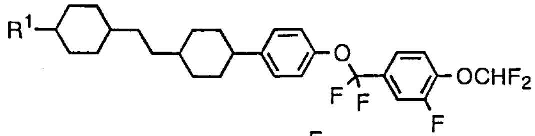
(1-c-49)

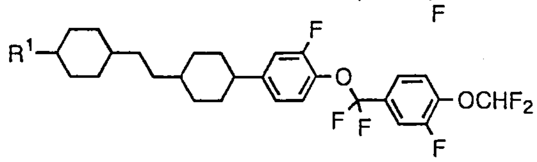
(1-c-50)

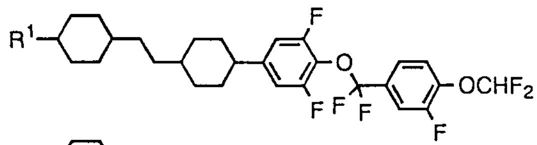
(1-c-51)

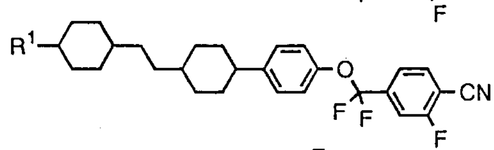
(1-c-52)

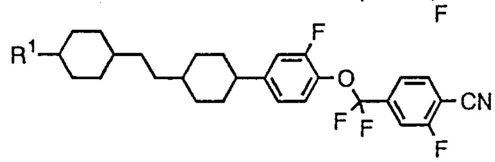
(1-c-53)

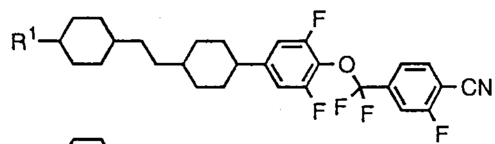
(1-c-54)

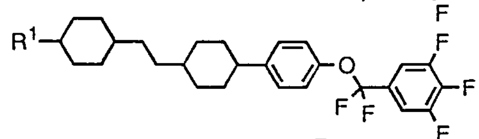
(1-c-55)

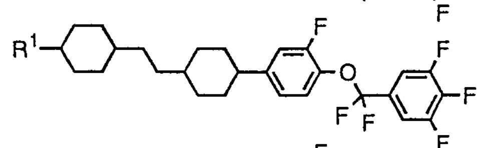
(1-c-56)

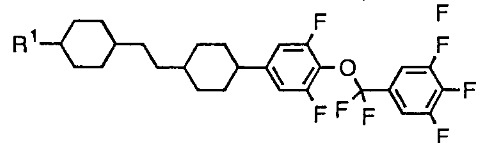
(1-c-57)

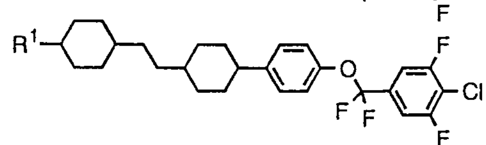
(1-c-58)

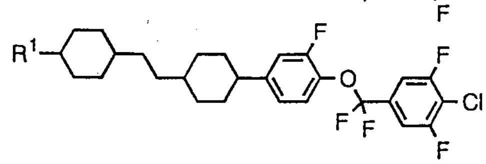
(1-c-59)

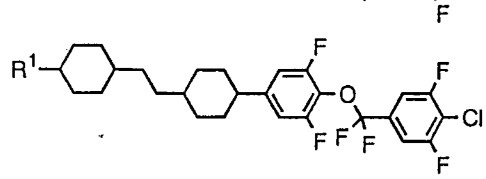
(1-c-60)

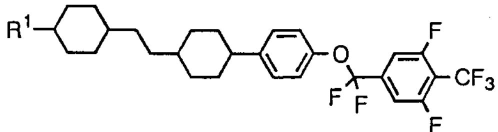 (1-c-61)

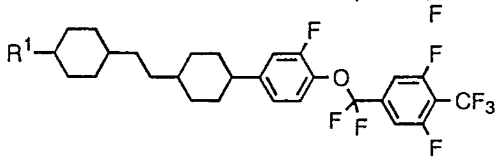 (1-c-62)

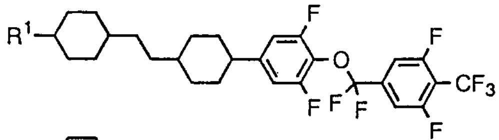 (1-c-63)

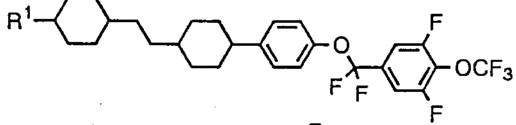 (1-c-64)

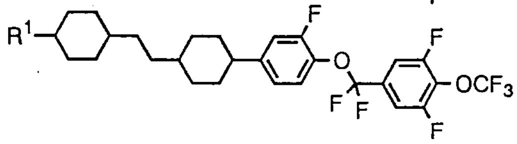 (1-c-65)

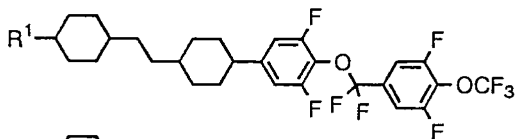 (1-c-66)

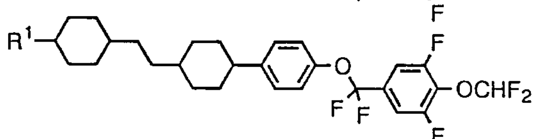 (1-c-67)

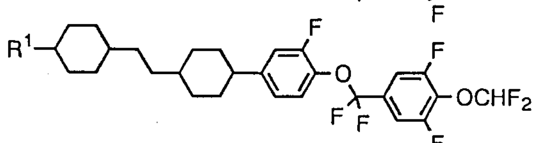 (1-c-68)

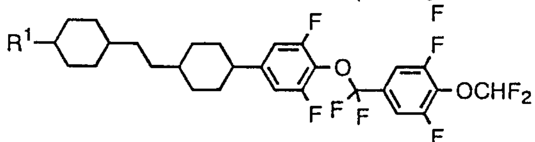 (1-c-69)

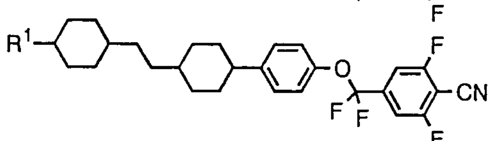 (1-c-70)

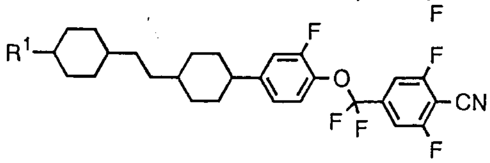 (1-c-71)

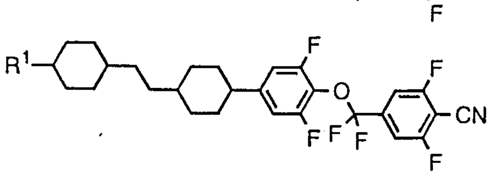 (1-c-72)

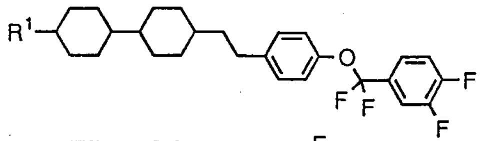 (1-c-73)

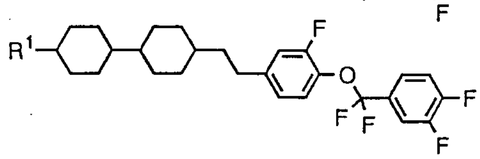 (1-c-74)

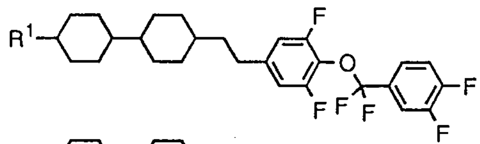 (1-c-75)

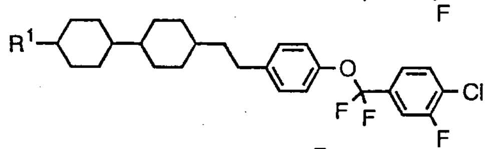 (1-c-76)

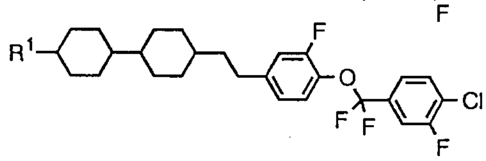 (1-c-77)

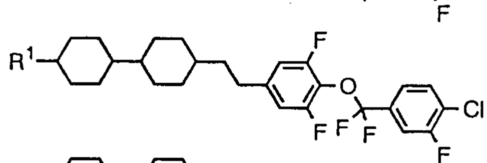 (1-c-78)

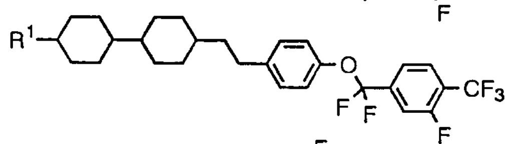 (1-c-79)

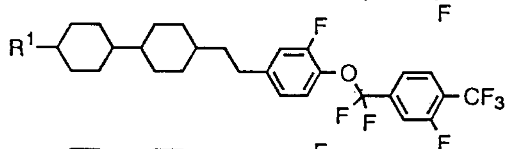 (1-c-80)

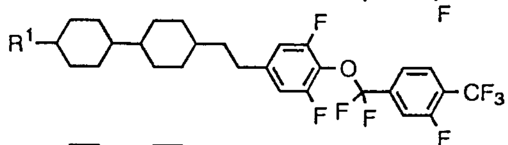 (1-c-81)

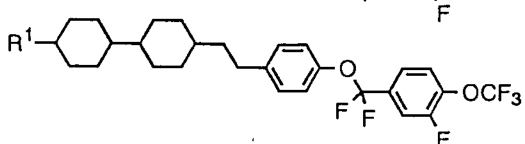 (1-c-82)

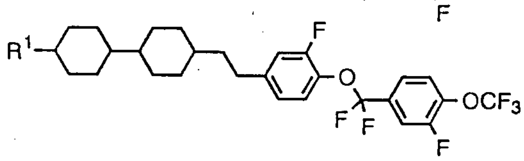 (1-c-83)

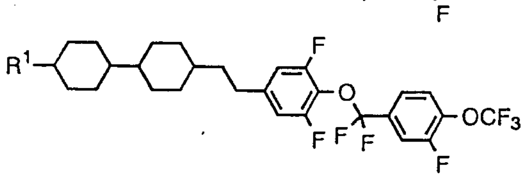 (1-c-84)

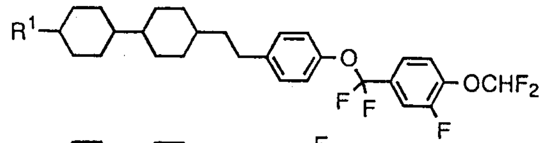 (1-c-85)

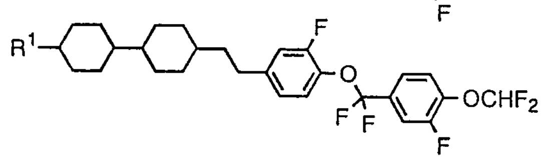 (1-c-86)

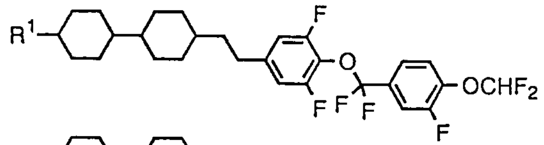 (1-c-87)

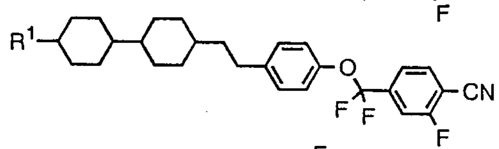 (1-c-88)

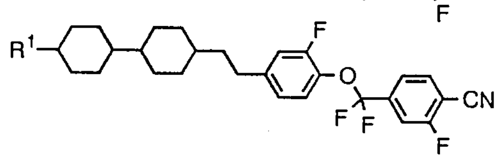 (1-c-89)

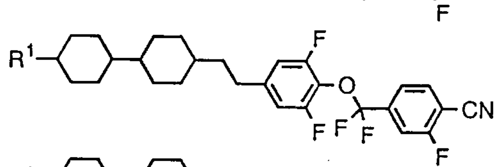 (1-c-90)

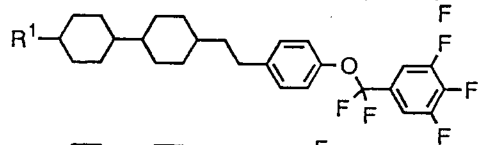 (1-c-91)

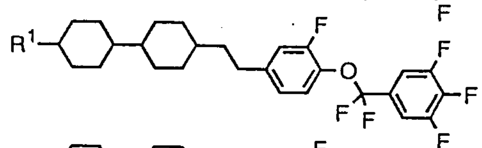 (1-c-92)

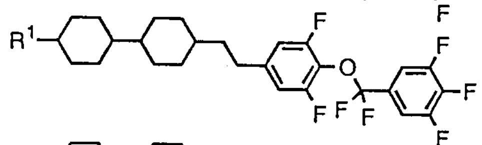 (1-c-93)

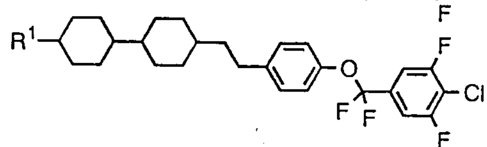 (1-c-94)

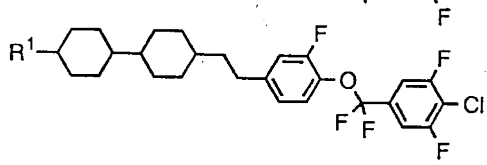 (1-c-95)

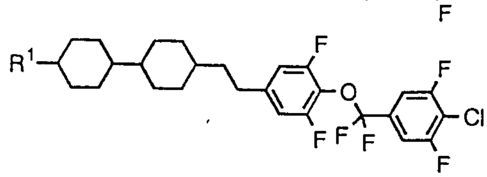 (1-c-96)

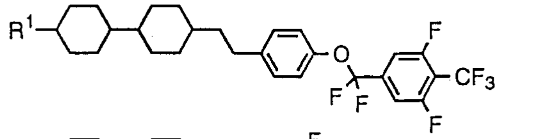
(1-c-97)

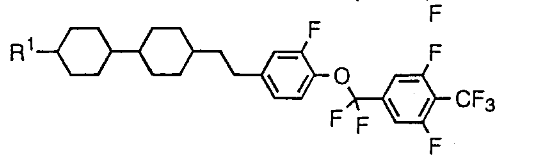
(1-c-98)

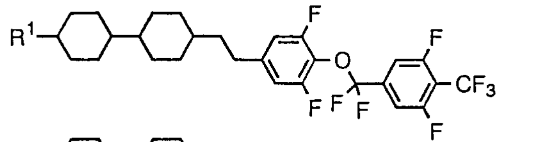
(1-c-99)

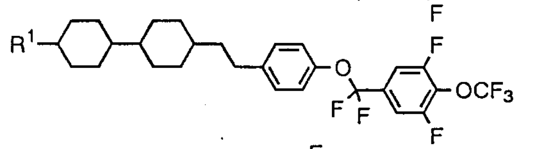
(1-c-100)

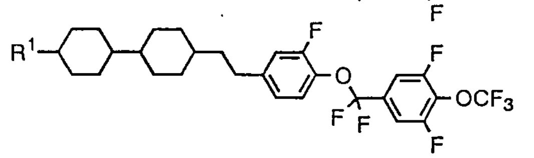
(1-c-101)

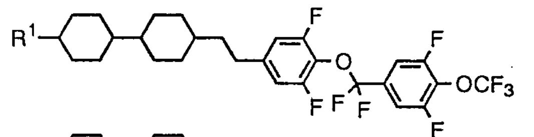
(1-c-102)

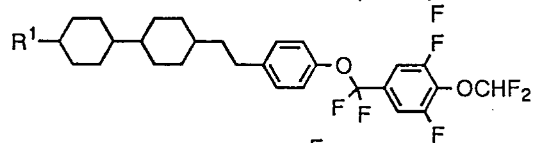
(1-c-103)

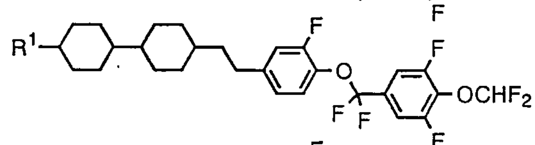
(1-c-104)

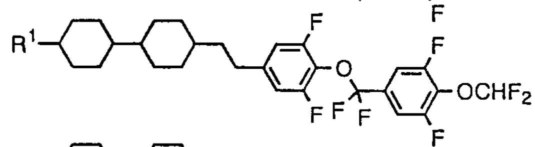
(1-c-105)

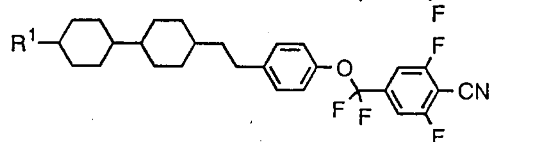
(1-c-106)

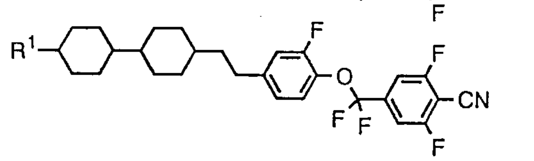
(1-c-107)

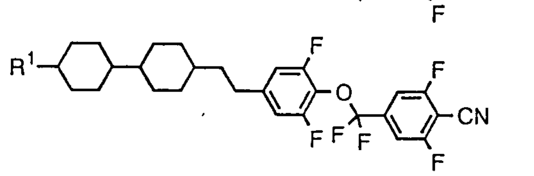
(1-c-108)

wherein $R^1$ has the same meaning as described above.

Any compound expressed by one of the general formulas (1-a-1) to (1-a-108), (1-b-1) to (1-b-144), and (1-c-1) to (1-c-108) recited above has a comparatively low viscosity and exhibits a medium degree (~7.0) of dielectric anisotropy. Among them, three-ring system compounds expressed by one of the general formulas (1-a-1) to (1-a-108) have

extremely low viscosity compared with known compounds having a medium degree (~7.0) of dielectric anisotropy, and are excellent in miscibility at low temperatures. Since the compounds exhibit low threshold voltage which is considered to be caused by their small elastic constant value, when the compounds are added as a component to liquid crystal compositions having high dielectric anisotropy and used for a low voltage driving, dielectric anisotropy and viscosity can remarkably be reduced without raising threshold voltage.

Since the four-ring system compounds expressed by one of the general formulas (1-b-1) to (1-b-144) and (1-c-1) to (1-c-108) have a comparatively wide temperature range of nematic phase, have low viscosity, and exhibit comparatively low threshold voltage, when the compounds are added as a component to liquid crystal compositions, only clearing point can be raised without raising threshold voltage or viscosity.

As described above, it is possible to provide liquid crystal compositions and liquid crystal display devices which are small in the intake of ionic impurities, are very stable against the changes in the outside environment, can be driven at a low voltage, and are capable of response at a high speed, by producing the compositions from three-ring system or four-ring system compounds, or by using those compounds together with known liquid crystalline compounds.

Liquid crystal compositions of the present invention comprise at least one compound expressed by the general formula (1) preferably in the ratio of 0.1 to 99 % by weight to develop excellent characteristics.

Further, the liquid crystal compositions of the present invention preferably comprise, as a second component, at least one compound selected from the group consisting of the compounds expressed by any one of the general formulas (2), (3), and (4) (hereinafter referred to as second component A) and/or at least one compound selected from the group of the compounds expressed by any one of the general formulas (5), (6), (7), (8), and (9) (hereinafter referred to as second component B) in addition to the liquid crystalline compound expressed by the general formula (1). Besies, a known compound may be mixed as a third component for the purpose of adjusting threshold voltage, temperature range of liquid crystal phase, optical anisotropy, dielectric anisotropy, and viscosity.

As preferable examples of the compounds included in the general formulas (2), (3), or (4) among the second component A, the compounds expressed by one of the formulas (2-1) to (2-15), (3-1) to (3-48), and (4-1) to (4-55) can be mentioned.

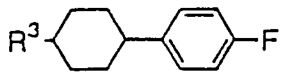
(2-1)

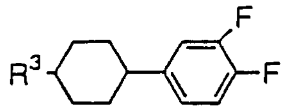
(2-2)

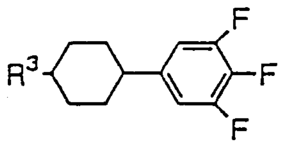
(2-3)

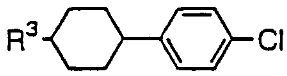
(2-4)

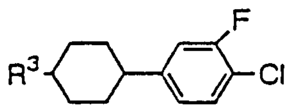
(2-5)

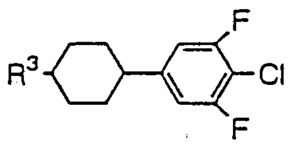
(2-6)

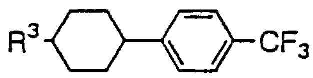
(2-7)

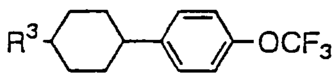
(2-8)

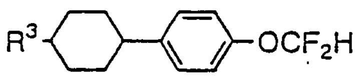
(2-9)

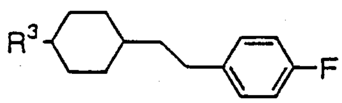
(2-10)

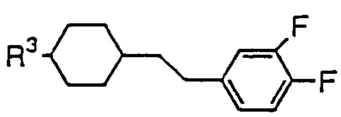
(2-11)

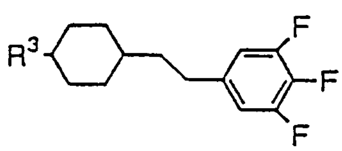
(2-12)

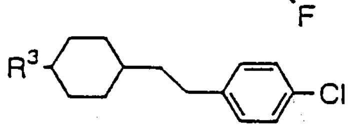
(2-13)

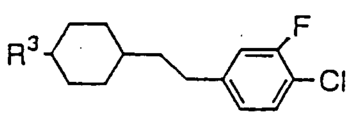
(2-14)

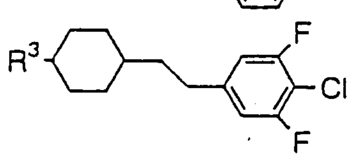
(2-15)

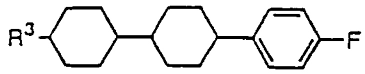 (3-1)

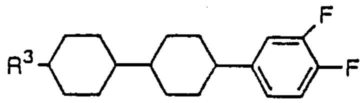 (3-2)

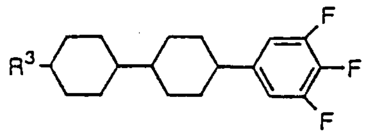 (3-3)

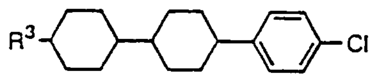 (3-4)

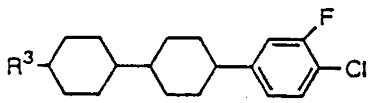 (3-5)

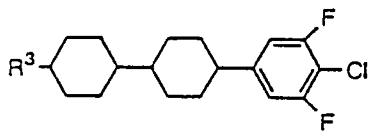 (3-6)

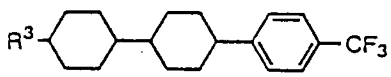 (3-7)

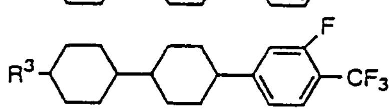 (3-8)

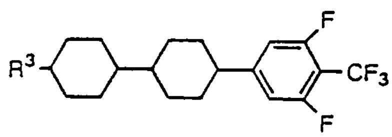 (3-9)

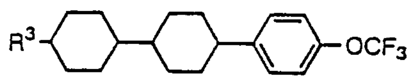 (3-10)

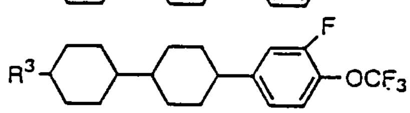 (3-11)

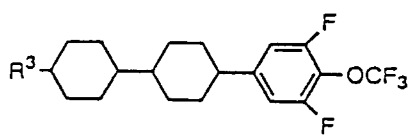 (3-12)

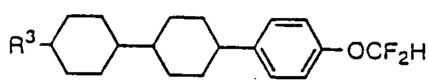 (3-13)

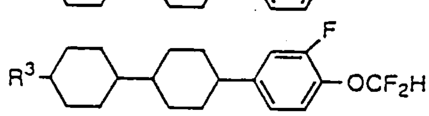 (3-14)

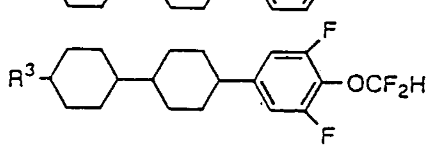 (3-15)

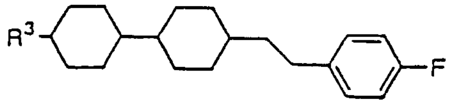
(3-16)

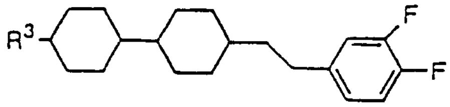
(3-17)

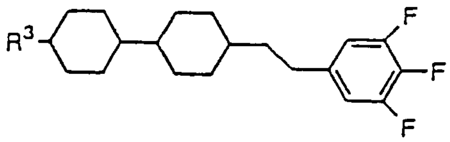
(3-18)

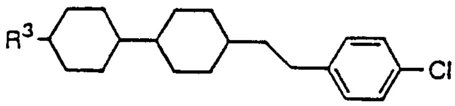
(3-19)

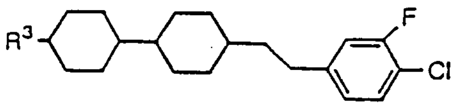
(3-20)

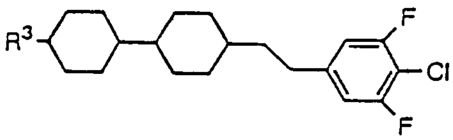
(3-21)

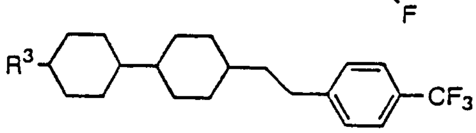
(3-22)

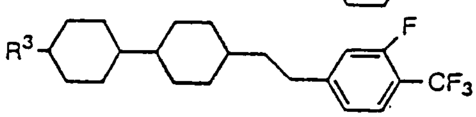
(3-23)

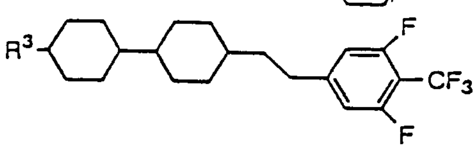
(3-24)

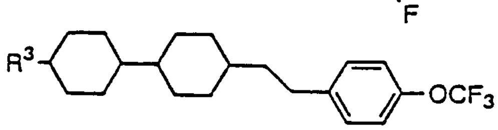
(3-25)

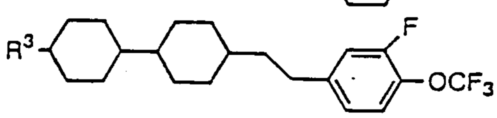
(3-26)

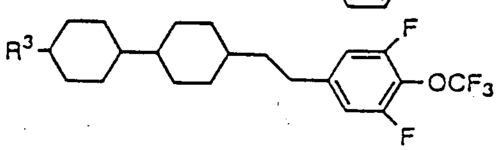
(3-27)

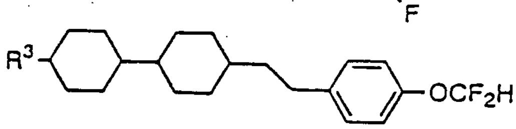
(3-28)

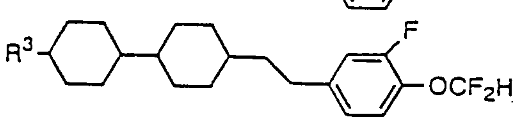
(3-29)

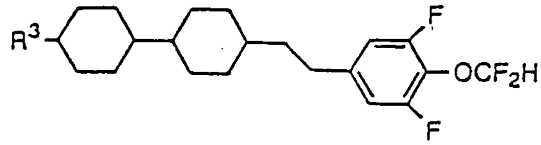 (3-30)

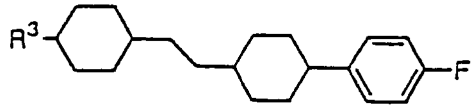 (3-31)

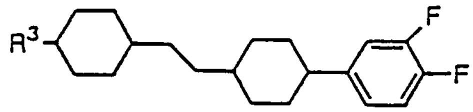 (3-32)

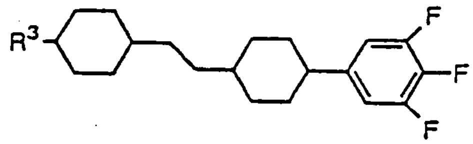 (3-33)

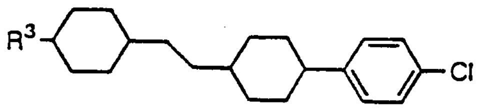 (3-34)

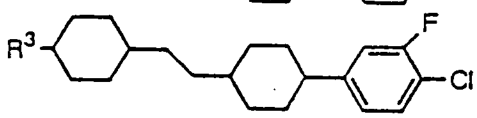 (3-35)

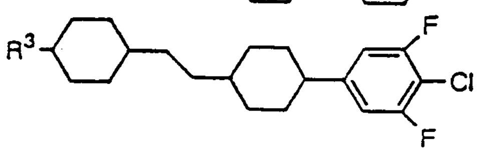 (3-36)

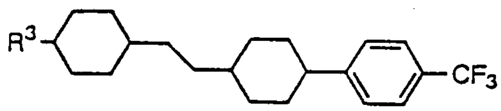 (3-37)

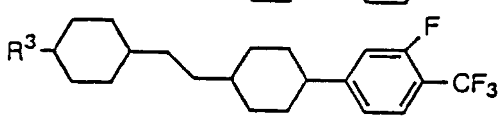 (3-38)

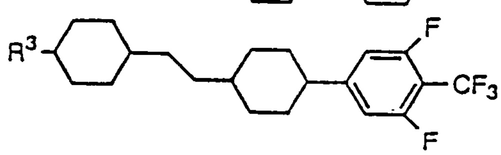 (3-39)

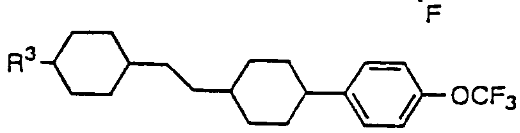 (3-40)

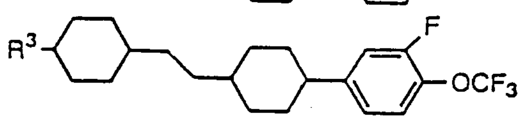 (3-41)

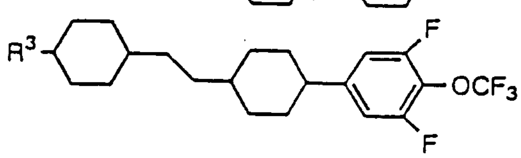 (3-42)

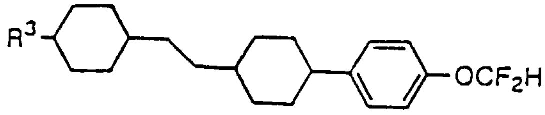

(3-43)

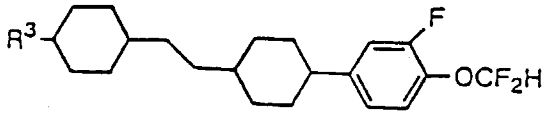

(3-44)

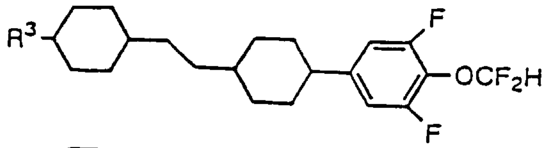

(3-45)

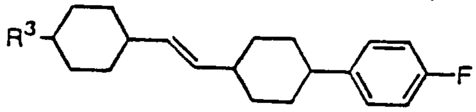

(3-46)

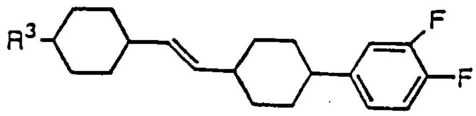

(3-47)

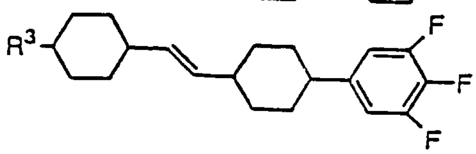

(3-48)

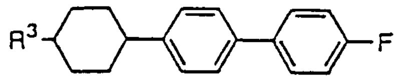
(4-1)

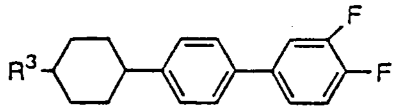
(4-2)

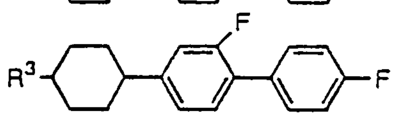
(4-3)

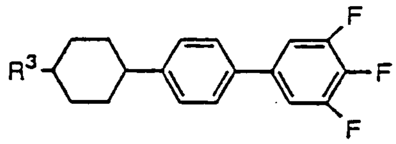
(4-4)

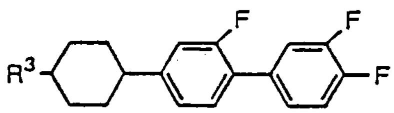
(4-5)

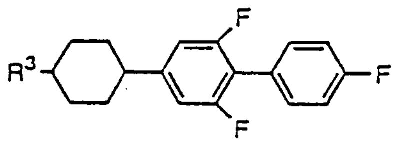
(4-6)

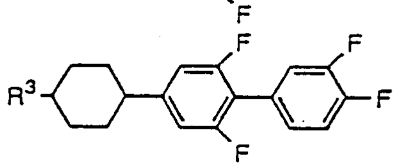
(4-7)

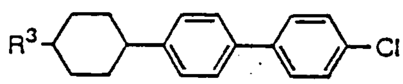
(4-8)

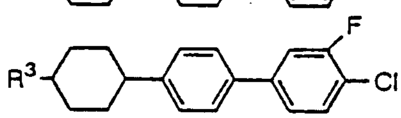
(4-9)

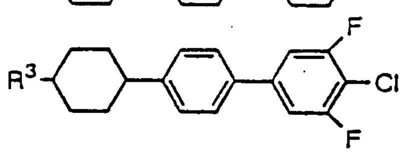
(4-10)

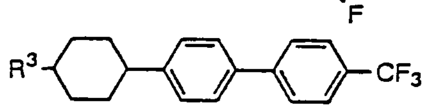
(4-11)

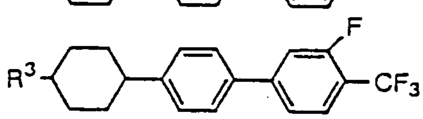
(4-12)

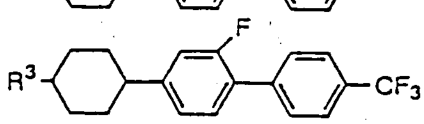
(4-13)

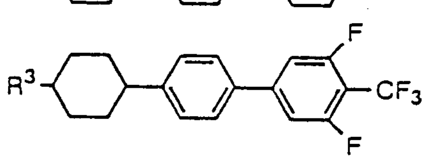
(4-14)

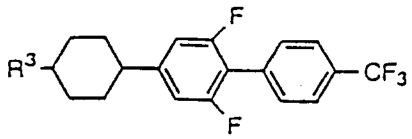
(4-15)

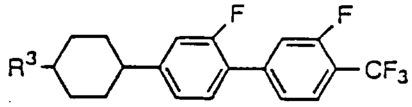
(4-16)

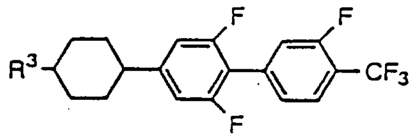
(4-17)

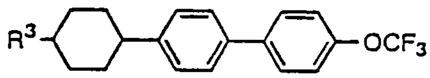
(4-18)

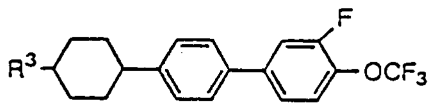
(4-19)

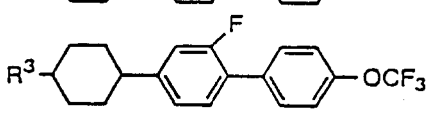
(4-20)

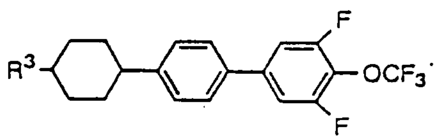
(4-21)

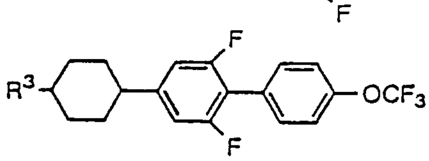
(4-22)

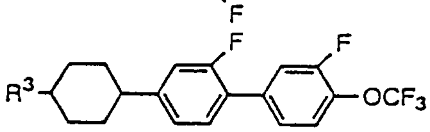
(4-23)

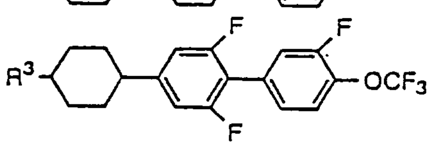
(4-24)

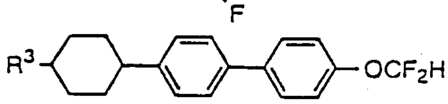
(4-25)

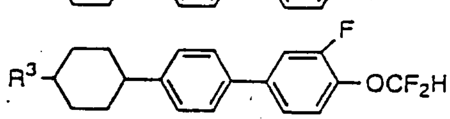
(4-26)

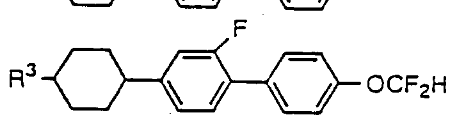
(4-27)

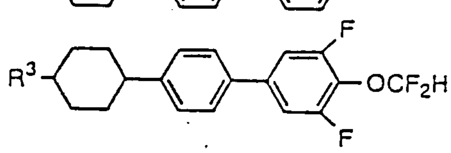
(4-28)

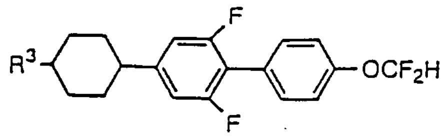

(4-29)

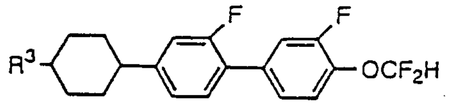

(4-30)

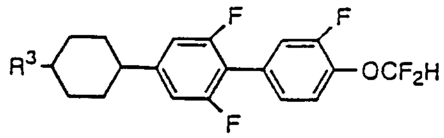

(4-31)

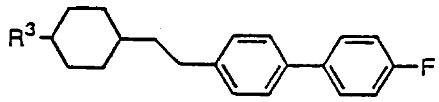

(4-32)

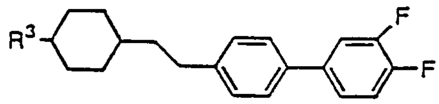

(4-33)

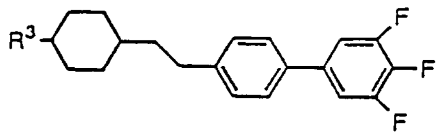

(4-34)

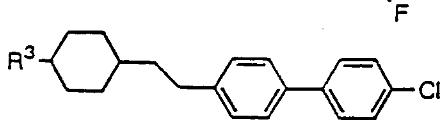

(4-35)

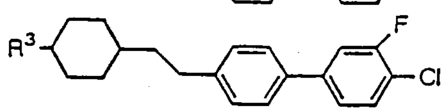

(4-36)

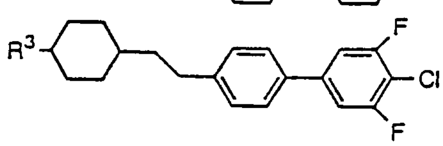

(4-37)

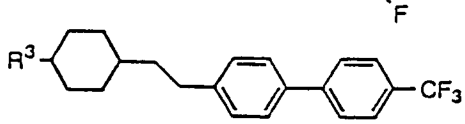

(4-38)

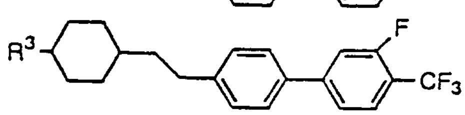

(4-39)

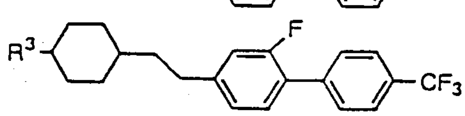

(4-40)

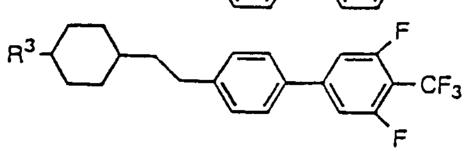

(4-41)

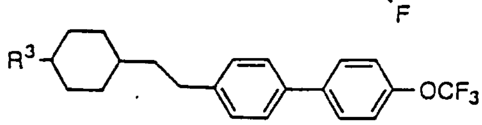

(4-42)

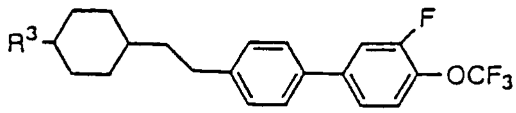

(4-43)

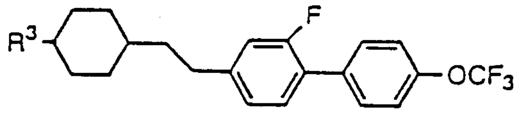

(4-44)

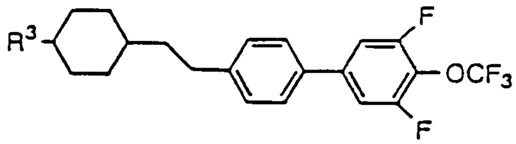

(4-45)

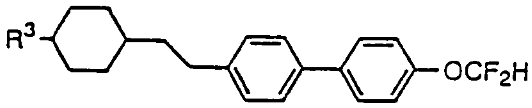

(4-46)

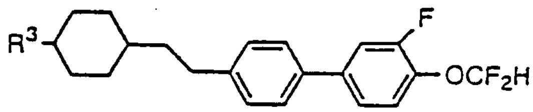

(4-47)

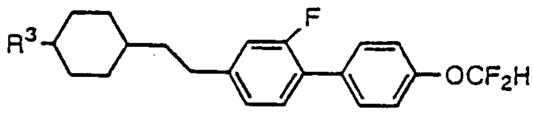

(4-48)

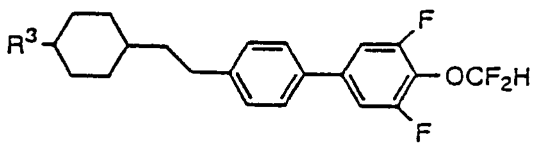

(4-49)

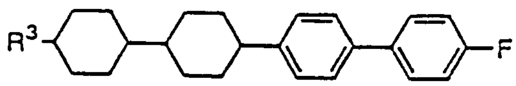

(4-50)

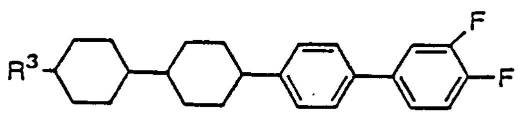

(4-51)

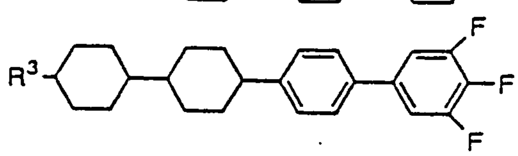

(4-52)

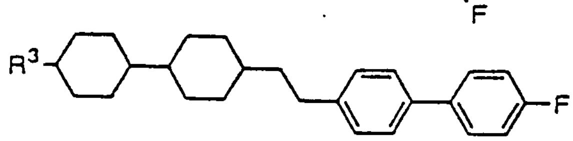

(4-53)

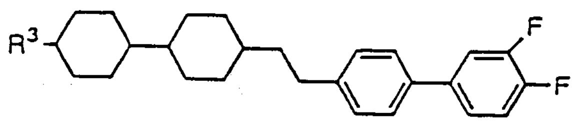

(4-54)

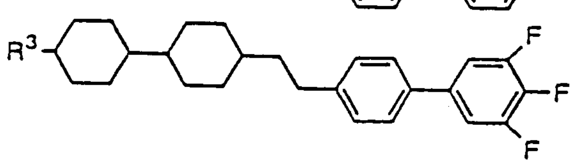

(4-55)

Compounds expressed by one of the general formulas (2) to (4) exhibit a positive dielectric anisotropy and are considerably excellent in heat stability and chemical stability.

Amount of the compounds to be used is suitably in the range of 1 to 99 % by weight, preferably 10 to 97 % by weight, and more desirably 40 to 95 % by weight based on the total amount of liquid crystal composition.

Among the compounds of the second component B described above, compounds expressed by one of the formulas (5-1) to (5-29), (6-1-) to (6-3), and (7-1) to (7-17) can be mentioned as preferable examples of the compounds included in the general formula (5), (6), or (7).

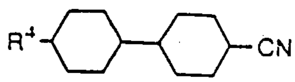 (5-1)

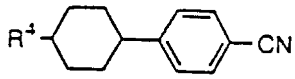 (5-2)

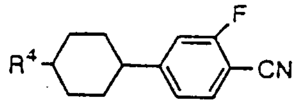 (5-3)

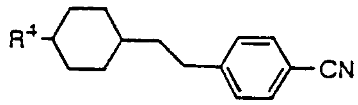 (5-4)

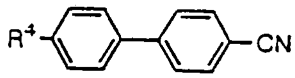 (5-5)

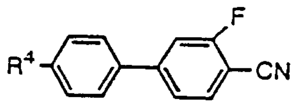 (5-6)

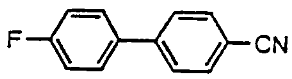 (5-7)

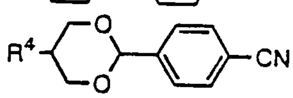 (5-8)

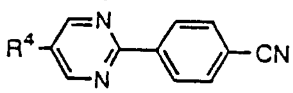 (5-9)

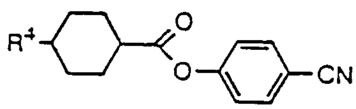 (5-10)

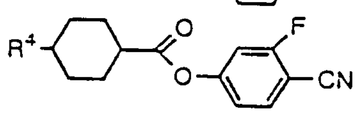 (5-11)

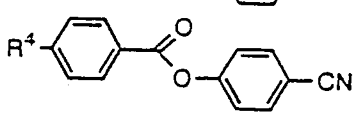 (5-12)

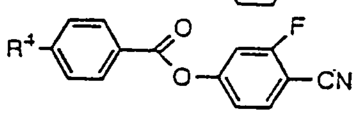 (5-13)

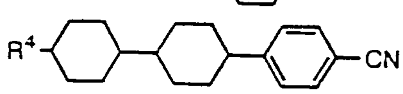 (5-14)

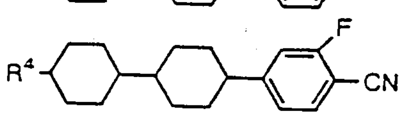 (5-15)

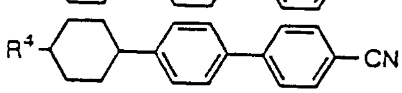 (5-16)

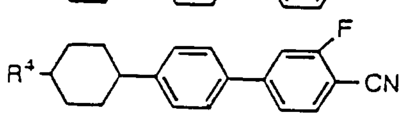 (5-17)

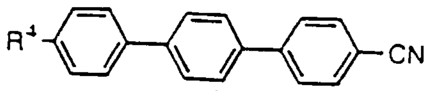
(5-18)

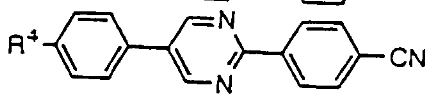
(5-19)

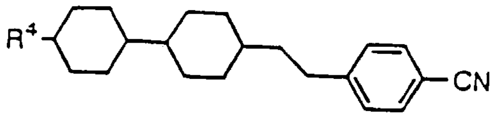
(5-20)

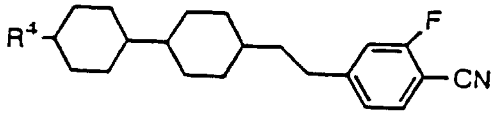
(5-21)

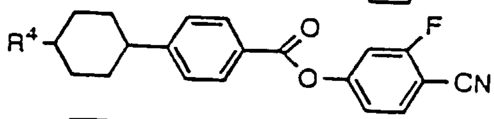
(5-22)

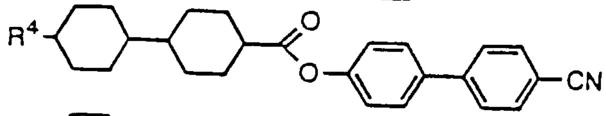
(5-23)

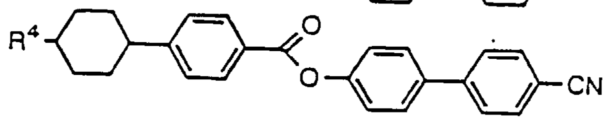
(5-24)

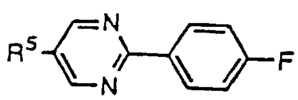
(6-1)

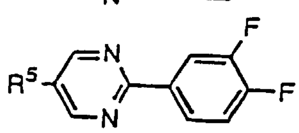
(6-2)

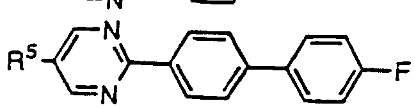
(6-3)

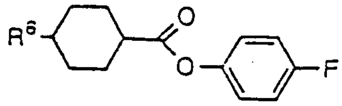
(7-1)

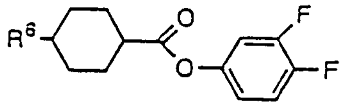
(7-2)

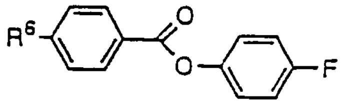
(7-3)

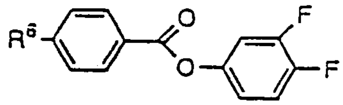
(7-4)

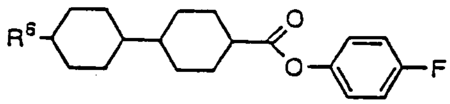
(7-5)

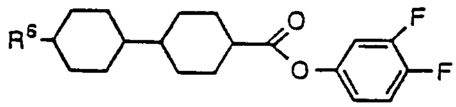
(7-6)

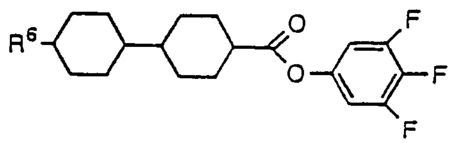
(7-7)

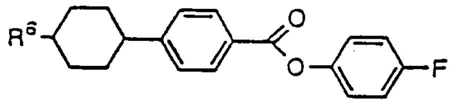
(7-8)

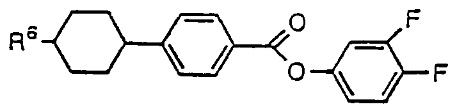
(7-9)

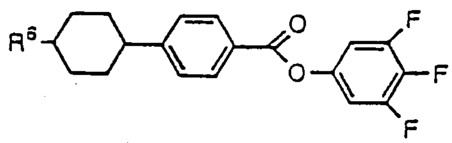
(7-10)

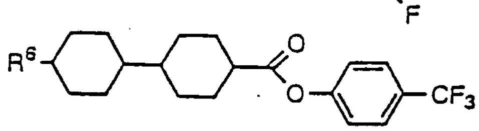
(7-11)

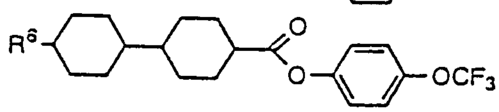
(7-12)

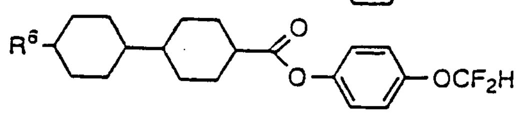
(7-13)

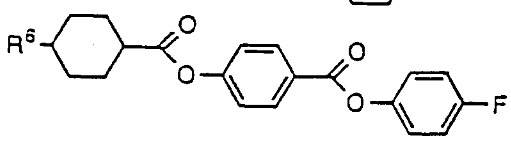
(7-14)

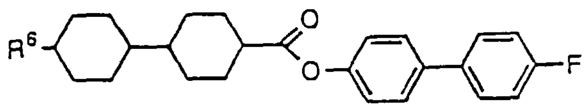

(7-15)

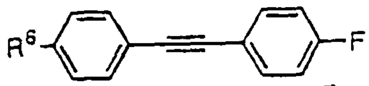

(7-16)

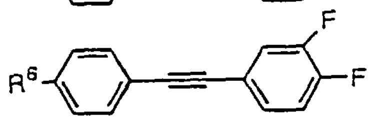

(7-17)

The compounds expressed by one of the general formulas (5) to (7) have a large positive value of dielectric anisotropy and are used, as a component of liquid crystal compositions, for the purpose of lowering threshold voltage. They are also used for the purpose of adjusting viscosity, adjusting optical anisotropy, and widening the temperature range of liquid crystal phase, and for the purpose of improving the steepness.

Among the second component B, the compounds expressed by one of the formulas (8-1) to (8-16) and (9-1) to (9-16) can be mentioned as preferable examples of the compounds included in the general formula (8) or (9).

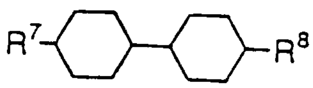

(8-1)

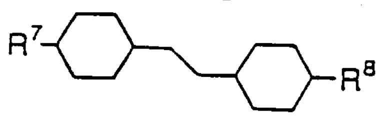

(8-2)

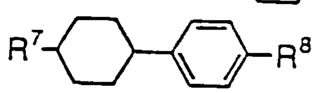

(8-3)

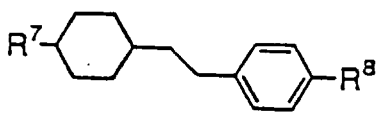

(8-4)

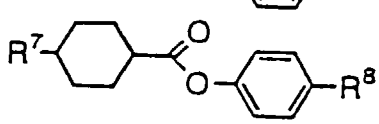

(8-5)

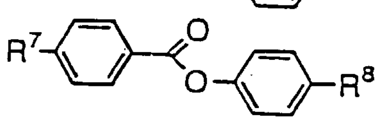

(8-6)

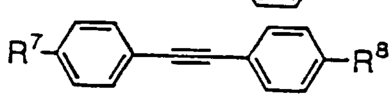

(8-7)

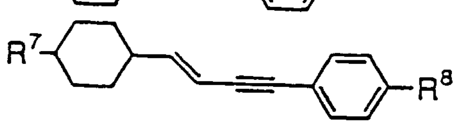

(8-8)

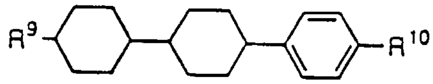
(9-1)

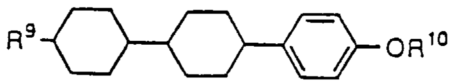
(9-2)

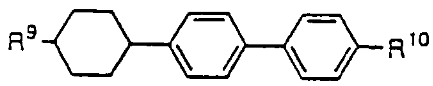
(9-3)

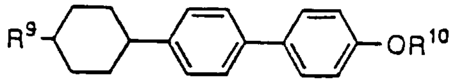
(9-4)

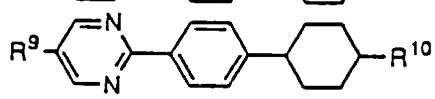
(9-5)

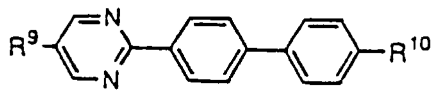
(9-6)

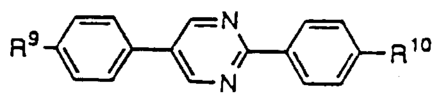
(9-7)

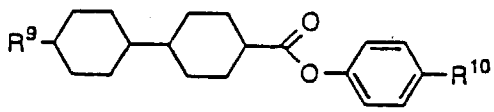
(9-8)

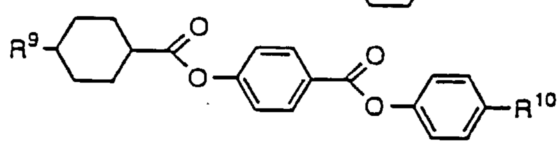
(9-9)

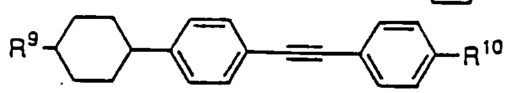
(9-10)

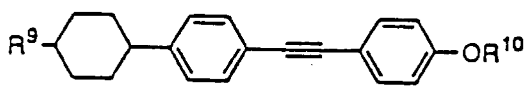
(9-11)

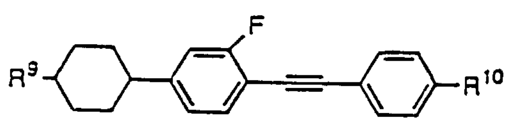
(9-12)

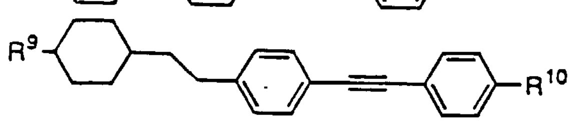
(9-13)

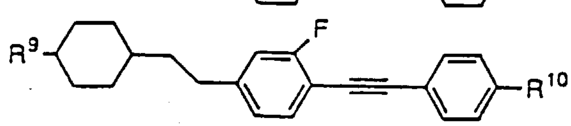
(9-14)

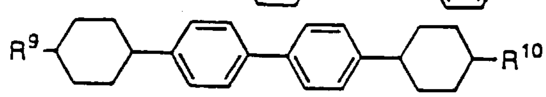
(9-15)

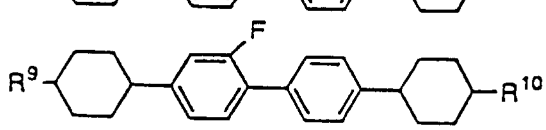
(9-16)

Amount of the compound of the second component B described above is suitably in the range of 1 to 99 % by weight, preferably 10 to 97 % by weight, and more desirably 40 to 95 % by weight based on the total amount of liquid

crystal composition.

Liquid crystal compositons used according to the present invention are produced by conventional methods which are known by themselves. Genarally, a method wherein various components are dissolved and mixed each other at a high temperature has been adopted. Moreover, the liquid crystal materials of the present invention are improved and optimized depending on intended uses by adding a suitable additive. Such an additive is well known in the art and described in detail in the literature. Usually, a chiral dopant or the like can be added to induce a helical structure of liquid crystals thereby adjust a required twisting angle and avoid a reverse-twist.

Liquid crystal compositions of the present invention can be used as ones for guest-host (GH) mode by adding a dichroic dye such as merocyanine type, styryl type, azo type, azomethine type, azoxy type, quinophthalone type, anthraquinone type, or tetrazine type dye. Liquid crystal compositions of the present invention can also be used as liquid crystal compositions for NCAP which is prepared by the microencapsulation of a nematic liquid crystal, or for a polymer dispersed liquid crystal display device (PDLCD) represented by a polymer network liquid crystal display device (PNLCD) prepared by forming polymers of three-dimensional reticulated structure in a liquid crystal. In addition, the liquid crystal compositions of the present invention can be used as ones for electrically controlled birefringence (ECB) mode or dynamic scattering (DS) mode.

As nematic liquid crystal compositions comprising the compound of the present invention, the following composition examples (Composition Examples 1 through 19) can be mentioned. In the composition examples, compounds are designated by the abbreviation according to the definitions shown in the following Table 1.

Table 1 Method for designating compounds by using symbols

$R--(-A_1-)--Z_1-- \cdots\cdots --Z_n--(-A_n-)--X$

| 1) Left side terminal group R- | Symbol | 3) Bonding group $-Z_1-$, $-Z_n-$ | Symbol |
|---|---|---|---|
| $C_nH_{2n+1}-$ | n- | $-C_2H_4-$ | 2 |
| $C_nH_{2n+1}O-$ | nO- | -COO- | E |
| $C_nH_{2n+1}OC_mH_{2m}-$ | nOm- | $-C\equiv C-$ | T |
| $CH_2=CH-$ | V- | -C=CH- | V |
| $CH_2=CHC_nH_{2n}-$ | Vn- | $-CF_2O-$ | CF2O |
| $C_nH_{2n+1}CH=CHC_mH_{2m}-$ | nVm- | $-OCF_2-$ | OCF2 |

| 2) Ring structure $-(-A_1-)-$, $-(-A_n-)-$ | Symbol | 4) Right side terminal group -X | Symbol |
|---|---|---|---|
| 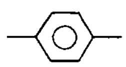 | B | -F | -F |
| | | -Cl | -CL |
| 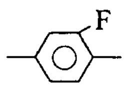 | B(F) | -CN | -C |
| | | $-CF_3$ | -CF3 |
| 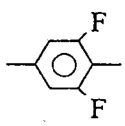 | B(F,F) | $-OCF_3$ | -OCF3 |
| | | $-OCF_2H$ | -OCF2H |
| 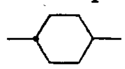 | H | $-C_nH_{2n+1}$ | -n |
| | | $-OC_nH_{2n+1}$ | -On |
| 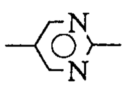 | Py | $-COOCH_3$ | -EMe |
| | | $-C_nH_{2n}CH=CH_2$ | -nV |
| 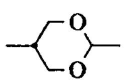 | D | $-C_mH_{2m}CH=CHC_nH_{2n+1}$ | -mVn |
| | | $-CH=CF_2$ | -VFF |

5) Examples of designation

Example 1 3-H2B(F,F)B(F)-F

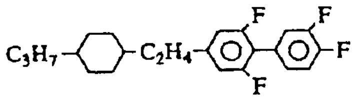

Example 3 1V2-BEB(F,F)-C

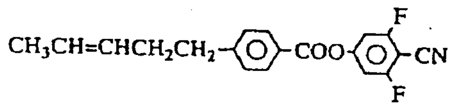

Example 2 3-HB(F)TB-2

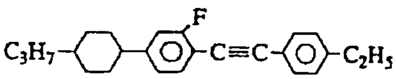

Composition Example 1

| | |
|---|---|
| 3-HBOCF2B(F)-OCF3 | 6.0 % |
| 7-HB(F)-F | 12.0 % |
| 2-HHB(F)-F | 13.0 % |
| 3-HHB(F)-F | 13.0 % |
| 5-HHB(F)-F | 13.0 % |
| 2-H2HB(F)-F | 6.0 % |
| 3-H2HB(F)-F | 3.0 % |
| 5-H2HB(F)-F | 6.0 % |
| 2-HBB(F)-F | 7.0 % |
| 3-HBB(F)-F | 7.0 % |
| 5-HBB(F)-F | 14.0 % |

Composition Example 2

| | |
|---|---|
| 3-HBOCF2B(F,F)-F | 5.0 % |
| 7-HB(F,F)-F | 5.0 % |
| 3-HHB(F,F)-F | 7.0 % |
| 3-H2HB(F,F)-F | 6.0 % |
| 3-HBB(F,F)-F | 15.0 % |

| | |
|---|---|
| 5-HBB(F,F)-F | 15.0 % |
| 3-H2BB(F,F)-F | 6.0 % |
| 4-H2BB(F,F)-F | 6.0 % |
| 5-H2BB(F,F)-F | 6.0 % |
| 3-HBEB(F,F)-F | 2.0 % |
| 5-HBEB(F,F)-F | 2.0 % |
| 3-HHEB(F,F)-F | 15.0 % |
| 4-HHEB(F,F)-F | 5.0 % |
| 5-HHEB(F,F)-F | 5.0 % |

Composition Example 3

| | |
|---|---|
| 3-HBOCF2B(F,F)-F | 10.0 % |
| 3-H2BOCF2B(F,F)-F | 10.0 % |
| 3-H2HB(F,F)-F | 5.0 % |
| 5-H2HB(F,F)-F | 4.0 % |
| 3-HHB(F,F)-F | 10.0 % |
| 4-HHB(F,F)-F | 6.0 % |
| 3-HH2B(F,F)-F | 12.0 % |
| 5-HH2B(F,F)-F | 8.0 % |
| 3-HBB(F,F)-F | 9.0 % |
| 5-HBB(F,F)-F | 9.0 % |
| 3-HHEB(F,F)-F | 9.0 % |
| 4-HHEB(F,F)-F | 2.0 % |
| 5-HHEB(F,F)-F | 2.0 % |
| 3-HHBB(F,F)-F | 2.0 % |
| 3-HH2BB(F,F)-F | 2.0 % |

Composition Example 4

| | |
|---|---|
| 3-HBOCF2B(F)-OCF3 | 8.0 % |

| | |
|---|---|
| 3-HBOCF2B(F,F)-CF3 | 8.0 % |
| 7-HB(F,F)-F | 4.0 % |
| 7-HB(F)-F | 7.0 % |
| 2-HHB(F)-F | 10.0 % |
| 3-HHB(F)-F | 10.0 % |
| 5-HHB(F)-F | 10.0 % |
| 2-H2HB(F)-F | 4.0 % |
| 3-H2HB(F)-F | 2.0 % |
| 5-H2HB(F)-F | 4.0 % |
| 4-H2HB(F,F)-F | 5.0 % |
| 5-H2HB(F,F)-F | 5.0 % |
| 3-HHB(F,F)-F | 8.0 % |
| 3-HH2B(F,F)-F | 8.0 % |
| 5-HH2B(F,F)-F | 7.0 % |

Composition Example 5

| | |
|---|---|
| 3-HBOCF2B(F)-OCF3 | 8.0 % |
| 5-HBOCF2B(F)-CL | 6.0 % |
| 3-HB-CL | 4.0 % |
| 5-HB-CL | 4.0 % |
| 7-HB-CL | 5.0 % |
| 2-HBB(F)-F | 5.5 % |
| 3-HBB(F)-F | 5.5 % |
| 5-HBB(F)-F | 11.0 % |
| 2-HHB-CL | 5.0 % |
| 4-HHB-CL | 5.0 % |
| 5-HHB-CL | 5.0 % |
| 3-HBB(F,F)-F | 16.0 % |

| | |
|---|---|
| 5-HBB(F,F)-F | 16.0 % |
| 3-HB(F)-TB-2 | 4.0 % |

Composition Example 6

| | |
|---|---|
| 3-HBOCF2B(F,F)-OCF3 | 4.0 % |
| 3-H2BOCF2B(F,F)-F | 9.0 % |
| 2-HHB(F)-F | 8.6 % |
| 3-HHB(F)-F | 8.7 % |
| 5-HHB(F)-F | 8.7 % |
| 3-HHB(F,F)-F | 7.0 % |
| 5-HHB(F,F)-F | 7.0 % |
| 3-H2HB(F,F)-F | 4.0 % |
| 4-H2HB(F,F)-F | 4.0 % |
| 5-H2HB(F,F)-F | 4.0 % |
| 3-HH2B(F,F)-F | 8.0 % |
| 5-HH2B(F,F)-F | 8.0 % |
| 2-HBB-F | 5.0 % |
| 3-HBB-F | 5.0 % |
| 5-HBB-F | 3.0 % |
| 3-HHB-1 | 6.0 % |

Composition Example 7

| | |
|---|---|
| 2-HBOCF2B(F)-F | 3.0 % |
| 3-HBOCF2B(F)-F | 3.0 % |
| 5-HBOCF2B(F)-F | 3.0 % |
| 7-HB(F)-F | 10.0 % |
| 7-HB(F,F)-F | 8.0 % |
| 5-H2B(F)-F | 3.0 % |
| 2-HHB(F)-F | 4.6 % |

| | |
|---|---|
| 3-HHB(F)-F | 4.7 % |
| 5-HHB(F)-F | 4.7 % |
| 2-HBB(F)-F | 7.5 % |
| 3-HBB(F)-F | 7.5 % |
| 5-HBB(F)-F | 15.0 % |
| 2-HBB-F | 4.0 % |
| 3-HBB-F | 4.0 % |
| 3-HB(F)-TB-2 | 6.0 % |
| 3-HB(F)-TB-3 | 6.0 % |
| 3-HB(F)-TB-4 | 6.0 % |

Composition Example 8

| | |
|---|---|
| 3-HBOCF2B(F)-OCF3 | 7.0 % |
| 3-HBOCF2B(F)-OCHF2 | 5.0 % |
| 5-HEB-F | 2.5 % |
| 7-HEB-F | 2.5 % |
| 2-HHB(F)-F | 9.0 % |
| 3-HHB(F)-F | 9.0 % |
| 5-HHB(F)-F | 9.0 % |
| 2-HBB(F)-F | 4.0 % |
| 3-HBB(F)-F | 4.0 % |
| 5-HBB(F)-F | 8.0 % |
| 3-H2HB(F,F)-F | 10.0 % |
| 3-HHB(F,F)-F | 10.0 % |
| 3-HH2B(F,F)-F | 10.0 % |
| 5-HH2B(F,F)-F | 10.0 % |

Composition Example 9

| | |
|---|---|
| 3-HBOCF2B(F)-OCF3 | 6.0 % |

| | |
|---|---|
| 3-H2BOCF2B(F,F)-F | 5.0 % |
| 3-HHBOCF2B(F,F)-F | 2.0 % |
| 2-HHB(F)-F | 10.0 % |
| 3-HHB(F)-F | 10.0 % |
| 5-HHB(F)-F | 10.0 % |
| 2-HBB(F)-F | 5.5 % |
| 3-HBB(F)-F | 5.5 % |
| 5-HBB(F)-F | 11.0 % |
| 3-HHB(F,F)-F | 7.0 % |
| 5-HHB(F,F)-F | 4.0 % |
| 3-HH2B(F,F)-F | 5.0 % |
| 5-HH2B(F,F)-F | 5.0 % |
| 5-H2HB(F,F)-F | 5.0 % |
| 5-HHEBB-F | 2.0 % |
| 3-HB-O2 | 5.0 % |
| 3-HHB-O1 | 2.0 % |

Composition Example 10

| | |
|---|---|
| 1V2-HBOCF2B(F)-C | 5.0 % |
| 3-H2BOCF2B(F)-C | 3.0 % |
| V2-HB-C | 10.0 % |
| 1V2-HB-C | 10.0 % |
| 3-HB-C | 20.0 % |
| 5-HB-C | 10.0 % |
| 3-HB(F)-C | 8.0 % |
| 2-BEB-C | 3.0 % |
| V2-HHB-1 | 8.0 % |
| 3-HHB-O1 | 4.0 % |

| | |
|---|---|
| 3-HHB-3 | 10.0 % |
| 3-H2BTB-2 | 3.0 % |
| 3-H2BTB-3 | 3.0 % |
| 3-H2BTB-4 | 3.0 % |

Composition Example 11

| | |
|---|---|
| 5-HBOCF2B(F,F)-C | 5.0 % |
| 2-HBOCF2B(F)-C | 5.0 % |
| 3-HBOCF2B(F)-C | 5.0 % |
| 1V2-BEB(F,F)-C | 5.0 % |
| 2O1-BEB(F)-C | 5.0 % |
| 3O1-BEB(F)-C | 9.0 % |
| 3-HB(F)-C | 15.0 % |
| 3-HH-4 | 3.0 % |
| 1O1-HH-3 | 3.0 % |
| 4-BTB-O2 | 5.0 % |
| 2-HHB(F)-C | 10.0 % |
| 3-HHB(F)-C | 10.0 % |
| 3-H2BTB-2 | 4.0 % |
| 3-H2BTB-3 | 3.0 % |
| 3-H2BTB-4 | 3.0 % |
| 2-BTB-1 | 1.0 % |
| 1-BTB-6 | 2.0 % |
| 4-BTB-4 | 1.0 % |
| 3-HH-2V | 3.0 % |
| 4-HH-V | 3.0 % |

Composition Example 12

| | |
|---|---|
| V-H2BOCF2B(F,F)-C | 6.0 % |

| | |
|---|---|
| 5-HBOCF2B(F)-C | 10.0 % |
| 2-BB-C | 5.0 % |
| 2O2O-BB-C | 4.0 % |
| 1O1-HB-C | 10.0 % |
| 2O1-HB-C | 6.0 % |
| 2-BEB-C | 10.0 % |
| 5-PyB-F | 8.0 % |
| 2-PyB-2 | 2.0 % |
| 3-PyB-2 | 2.0 % |
| 4-PyB-2 | 2.0 % |
| V-HHB-1 | 2.0 % |
| 3-HHB-1 | 3.0 % |
| 3-HHB-3 | 6.0 % |
| 2-PyBH-3 | 5.0 % |
| 3-PyBH-3 | 5.0 % |
| 4-PyBH-3 | 5.0 % |
| 3-PyBB-F | 3.0 % |
| 4-PyBB-F | 3.0 % |
| 6-PyBB-6 | 3.0 % |

Composition Example 13

| | |
|---|---|
| 3-HBOCF2B(F)-OCF3 | 3.0 % |
| 3-H2HBOCF2B(F)-C | 3.0 % |
| 3-PyB(F)-F | 4.5 % |
| 3O2O-BEB-C | 3.0 % |
| 3-BEB-C | 6.0 % |
| 3-DB-C | 10.0 % |
| 4-DB-C | 10.0 % |

| | |
|---|---|
| 3-HEB-O4 | 12.0 % |
| 4-HEB-O2 | 9.0 % |
| 5-HEB-O1 | 9.0 % |
| 3-HEB-O2 | 7.5 % |
| 5-HEB-O2 | 6.0 % |
| 3-HHB-1 | 5.0 % |
| 3-HHEBB-C | 2.0 % |
| 3-HBEBB-C | 2.0 % |
| 1O-BEB-2 | 2.0 % |
| 4-HEB-3 | 2.0 % |
| 5-HEB-1 | 2.0 % |
| 6-PyB-o2 | 2.0 % |

Composition Example 14

| | |
|---|---|
| 5-HH2BOCF2B(F)-C | 2.0 % |
| 3-HBOCF2BB(F)-C | 2.0 % |
| 3-HB-C | 20.0 % |
| 3-HHB-1 | 7.0 % |
| 3-HHB-3 | 8.0 % |
| 5-HEB-F | 3.0 % |
| 7-HEB-F | 3.0 % |
| 3-HHEB-F | 1.0 % |
| 5-HHEB-F | 1.0 % |
| 3-HEB-O4 | 4.0 % |
| 4-HEB-O2 | 3.0 % |
| 5-HEB-O1 | 3.0 % |
| 3-HEB-O2 | 2.5 % |
| 5-HEB-O2 | 2.0 % |

| | |
|---|---|
| 3-HB(F)TB-2 | 6.0 % |
| 3-HB(F)TB-3 | 6.0 % |
| 3-HB(F)VB-4 | 6.0 % |
| 3-H2BTB-2 | 4.0 % |
| 3-H2BTB-3 | 4.0 % |
| 3-H2BTB-4 | 4.0 % |
| 3-HHEBB-C | 3.0 % |
| 3-HEBEB-F | 3.0 % |
| 3-HH-EMe | 2.5 % |

Composition Example 15

| | |
|---|---|
| 2-HBOCF2B(F)-C | 8.0 % |
| 3-HBOCF2B(F)-C | 10.0 % |
| 5-HBOCF2B(F)-C | 10.0 % |
| 5-PyB(F)-F | 8.0 % |
| 2-HB(F)-C | 9.0 % |
| 3-HB(F)-C | 10.0 % |
| 3O-BB-C | 7.0 % |
| 2-HHB-C | 3.0 % |
| 3-HHB-C | 3.0 % |
| 4-HHB-C | 3.0 % |
| 5-HHB-C | 3.0 % |
| 2-HHB(F)-C | 5.0 % |
| 3-HHB(F)-C | 5.0 % |
| 3-PyBB-F | 3.0 % |
| 4-PyBB-F | 3.0 % |
| 5-HBB-C | 5.0 % |
| 3-HB(F)EB(F)-C | 5.0 % |

Composition Example 16

| | |
|---|---|
| 3-HBOCF2B(F)-OCF3 | 5.0 % |
| 3-HBOCF2B(F,F)-CF3 | 3.0 % |
| 5-HB-F | 9.0 % |
| 6-HB-F | 7.0 % |
| 7-HB-F | 7.0 % |
| 5-HB-3 | 5.0 % |
| 3-HB-O1 | 5.0 % |
| 2-HHB-OCF3 | 5.0 % |
| 4-HHB-OCF3 | 5.0 % |
| 5-HHB-OCF3 | 4.0 % |
| 3-HH2B-OCF3 | 2.0 % |
| 5-HH2B-OCF3 | 3.0 % |
| 3-HH2B-F | 3.0 % |
| 5-HH2B-F | 3.0 % |
| 3-HBB(F)-F | 6.0 % |
| 5-HBB(F)-F | 5.0 % |
| 3-HH2B(F)-F | 7.0 % |
| 5-HH2B(F)-F | 9.0 % |
| 3-HB(F)BH-3 | 3.0 % |
| 5-HB(F)BH-3 | 2.0 % |
| 5-HB(F)BH-5 | 2.0 % |

Composition Example 17

| | |
|---|---|
| 3-HBOCF2B(F,F)-OCHF2 | 8.0 % |
| 5-H2BOCF2B(F)-F | 2.0 % |
| 5-HB-F | 6.0 % |
| 7-HB-F | 6.0 % |

| | |
|---|---|
| 2-HHB-OCHF2 | 4.0 % |
| 3-HHB-OCHF2 | 5.0 % |
| 3-HHB(F,F)-OCHF2 | 7.0 % |
| 5-HHB(F,F)-OCHF2 | 5.0 % |
| 2-HHB-OCF3 | 8.0 % |
| 3-HHB-OCF3 | 9.0 % |
| 5-HHB-OCF3 | 10.0 % |
| 3-HH2B(F)-F | 10.0 % |
| 5-HH2B(F)-F | 10.0 % |
| 3-HHEB(F)-F | 5.0 % |
| 5-HHEB(F)-F | 5.0 % |

Composition Example 18

| | |
|---|---|
| 3-HBOCF2B(F)-C | 5.0 % |
| 5-HBOCF2B(F)-F | 3.0 % |
| 4-HEB(F)-F | 10.0 % |
| 5-HEB(F)-F | 10.0 % |
| 2-BEB(F)-C | 5.0 % |
| 3-BEB(F)-C | 5.0 % |
| 4-BEB(F)-C | 8.0 % |
| 5-BEB(F)-C | 8.0 % |
| 1O3-HB(F)-C | 6.0 % |
| 3-HHEB(F)-F | 4.0 % |
| 5-HHEB(F)-F | 3.0 % |
| 2-HBEB(F)-C | 5.0 % |
| 4-HBEB(F)-C | 5.0 % |
| 5-HBEB(F)-C | 5.0 % |
| 3-HBTB-2 | 10.0 % |

| | |
|---|---|
| V2-HH-3 | 4.0 % |
| V2-HHB-1 | 4.0 % |

Composition Example 19

| | |
|---|---|
| 2-HBOCF2B(F,F)-F | 10.0 % |
| 3-HBOCF2B(F,F)-F | 10.0 % |
| 5-HBOCF2B(F,F)-F | 10.0 % |
| 2-H2BOCF2B(F,F)-F | 10.0 % |
| 3-H2BOCF2B(F,F)-F | 10.0 % |
| 5-H2BOCF2B(F,F)-F | 10.0 % |
| 3-HBOCF2B(F)-OCF3 | 10.0 % |
| 3-HBOCF2B(F,F)-CF3 | 10.0 % |
| 3-HBOCF2B(F)-OCHF2 | 10.0 % |
| 3-HBOCF2BB(F)-OCF3 | 10.0 % |

[Methods for producing compounds]

The compounds of the present invention of the formula (1) can readily be produced by using ordinary chemical procedures of organic synthesis. For instance, the compounds can readily be synthesized by selecting suitable procedures described in Organic Synthesis, Organic Reaction, or Jikken Kagaku Kouza (Course of Chemical Experiments) (published by Maruzen Co., Ltd.) and using them in combination.

That is, the compounds expressed by the general formula (1) can be produced through the following procedures:

First, a Grignard reagent is prepared from bromobenzene derivative (10) according to a method of M. S. Kharasch et al., "Grignard Reactions of Nonmetallic Substances", Prentice-Hall (1954). Next, the prepared Grignard reagent is reacted with carbon disulfide according to a method described in A. S. Wheeler et al., J. Am. Chem. Soc., 50, 3106 (1928) to prepare dithiocarboxylic acid derivative (11). The derivative (11) is converted into thioncarboxylic acid chloride by heating to reflux together with thionyl chloride in diethyl ether and then converted into thioncarboxylic acid-O-ester derivative (13) by reacting with phenol derivative (12) in the presence of a base such as pyridine. Then, the derivative (13) is reacted with diethylaminosulfur trifluoride (hereinafter abbreviated to DAST), or reacted with quarternary ammonium dihydrogentrifluoride in the presence of N-bromosuccinic acid imide according to a method described in Laid-open Japanese Patent Publication No. Hei 5-255165 to produce the compounds of the formula (1).

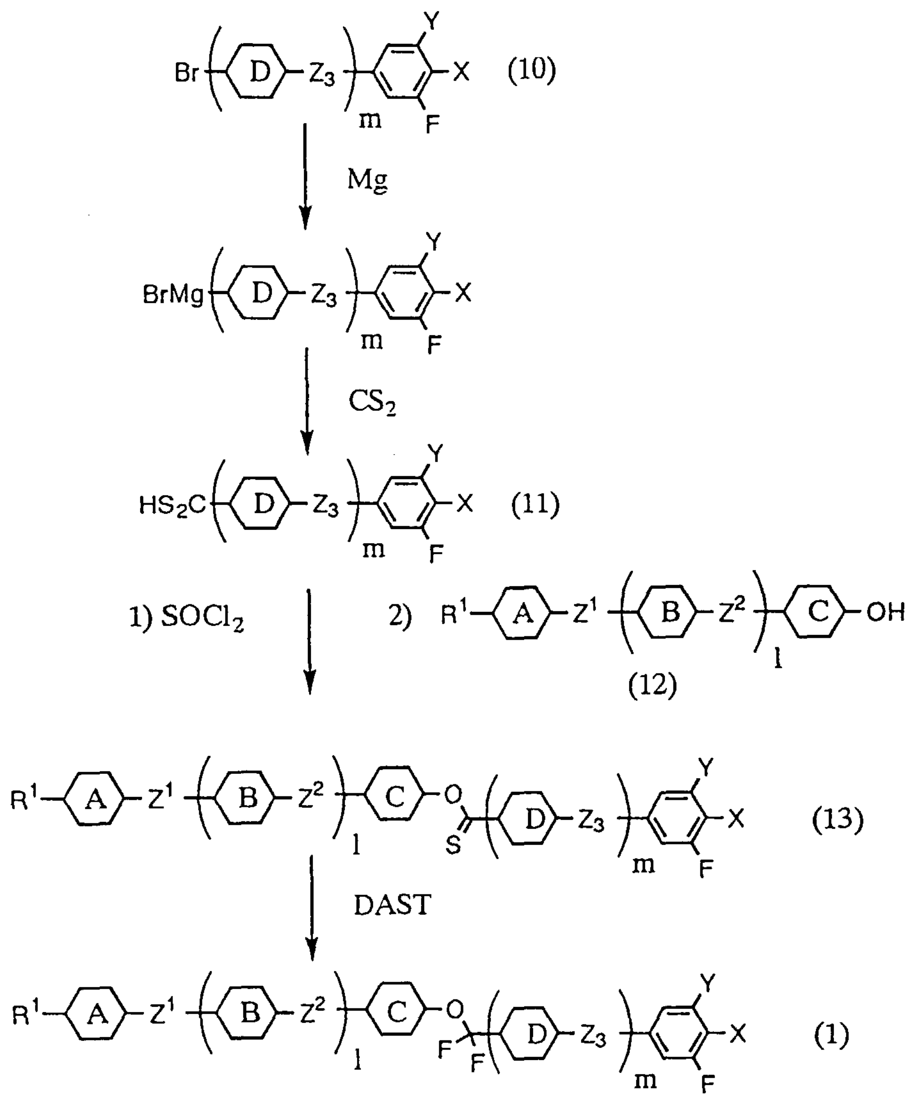


wherein $R^1$, rings A, B, C, and D, $Z^1$, $Z^2$, $Z^3$, X, Y, $\ell$, and m have the same meaning as described above.

While bromobenzene derivative (10) expressed by the general formula and used in the production method described above can be produced by known procedures of organic synthesis, the derivative can preferably be produced by the following method. That is, in the case of the derivative wherein bonding group $Z^3$ is single bond, first, iodobenzene derivative (14) is lithiated with butyl lithium or the like, reacted with zinc chloride to prepare organic zinc compound, and then subjected to cross coupling with bromobenzene derivative (15) to prepare biphenyl derivative (16). Next, the derivative (16) is reacted with bromine in the presence of a catalyst such as ferric chloride to produce bromobenzene derivative (10-1).

In the case of the derivative (10) wherein bonding group $Z^3$ is ethenylene or ethylene, first, acid chloride (17) is reacted with the Grignard reagent prepared from the derivative (15) by using iron=acetylacetonate as catalyst to prepare ketone derivative (18). Next, the carbonyl group of the derivative (18) is reduced down to alcohol with sodium boron hydride, and then dehydrated by using a mineral acid such as sulfuric acid and hydrochloric acid, an organic acid such as p-toluenesulfonic acid, or a non-aqueous acidic ion exchange resin as catalyst to prepare styrene derivative (19). The derivative (19) is reacted with bromine in the presence of a catalyst such as ferric chloride to produce bromobenzene derivative (10-2) wherein $Z^3$ is ethenyl group. Bromobenzene derivative (10-3) wherein $Z^3$ is ethylene group can be produced by further subjecting the derivative (19) to hydrogen reduction in the presence of a catalyst such as palladium-carbon and then brominating in the same manner as described above.

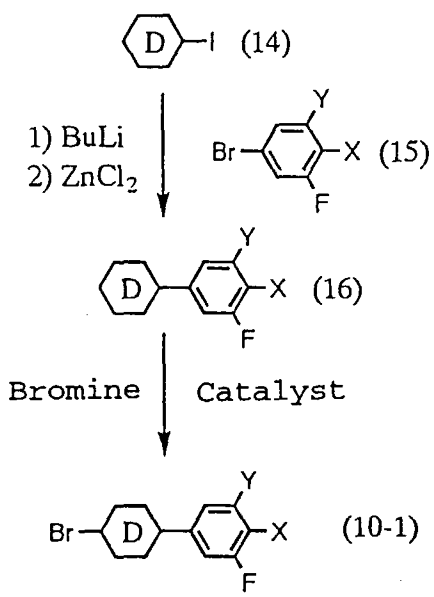


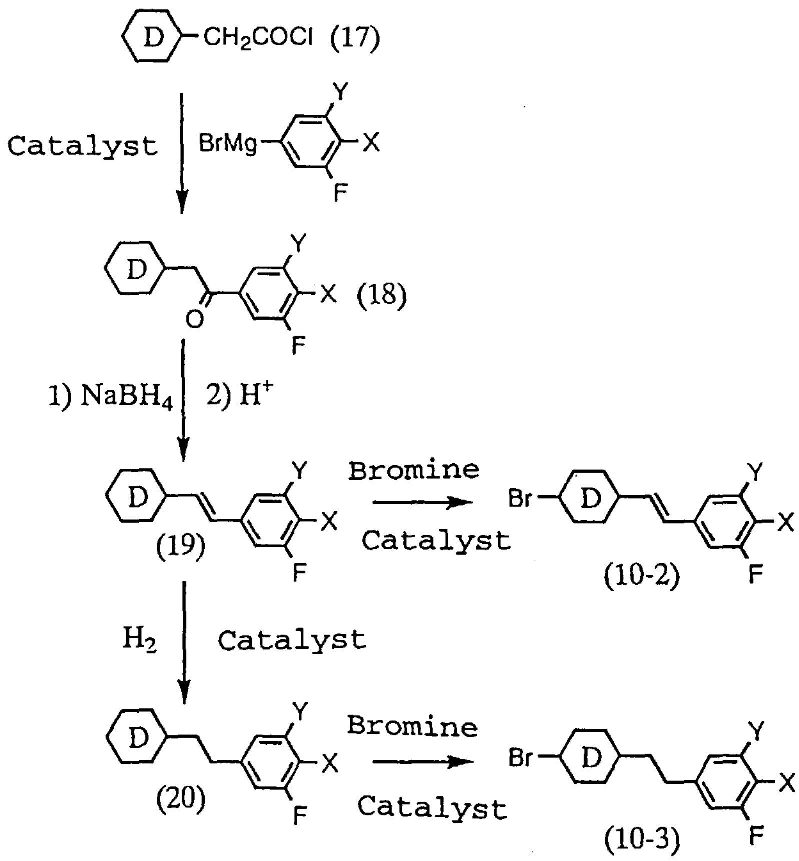


wherein ring D, X, and Y have the same meaning as described above.

While the phenol derivative (12) expressed by the general formula and used in the production method described above can be produced through known procedures of organic chemistry, the derivative can preferably be produced by the following method. That is, the phenol derivative (12) can be produced by reacting anisole derivative (21) preparation method of which is described in Japanese Patent Publication No. Sho 62-39136, Japanese Patent Publication No. Hei 3-3643, Laid-open Japanese Patent Publication No. Sho 57-159725, Laid-open Japanese Patent Publication No. Sho

60-84230, and WO Japanese Patent Publication (Tokuhyo) No. Hei 1-502274, with boron tribromide in a halogen type solvent.

In the case of the derivative (12) wherein bonding group $Z^2$ is 1,2-ethenylene or 1,2-ethylene, first, acid chloride (22) is reacted with the Grignard reagent prepared from bromoanisole derivative, by using iron=acetylacetonate as catalyst to prepare ketone derivative (23). Next, the carbonyl group of the derivative (23) is reduced down to alcohol with sodium boron hydride and then dehydrated by using a mineral acid such as sulfuric acid and hydrochloric acid, an organic acid such as p-toluenesulfonic acid, or a non-aqueous acidic ion exchange resin as catalyst to prepare 4(E)-vinylanisole derivative (24). The derivative (24) is reacted with bromine in the presence of a catalyst such as ferric chloride to produce phenol derivative (12-1) wherein $Z^2$ is 1,2-ethenylene group. Phenol derivative (12-2) wherein $Z^2$ is 1,2-ethylene can be produced by further subjecting the derivative (24) to hydrogen reduction in the presence of a catalyst such as palladium-carbon and then brominating in the same manner as described above.

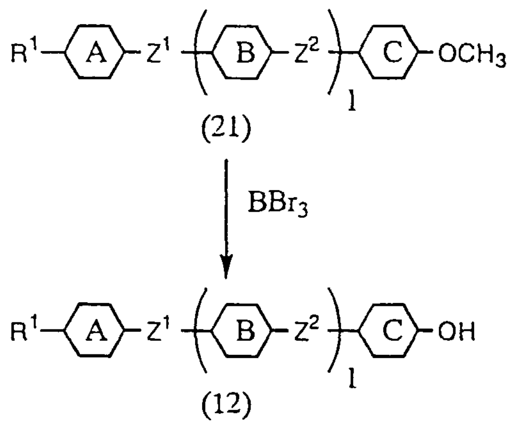


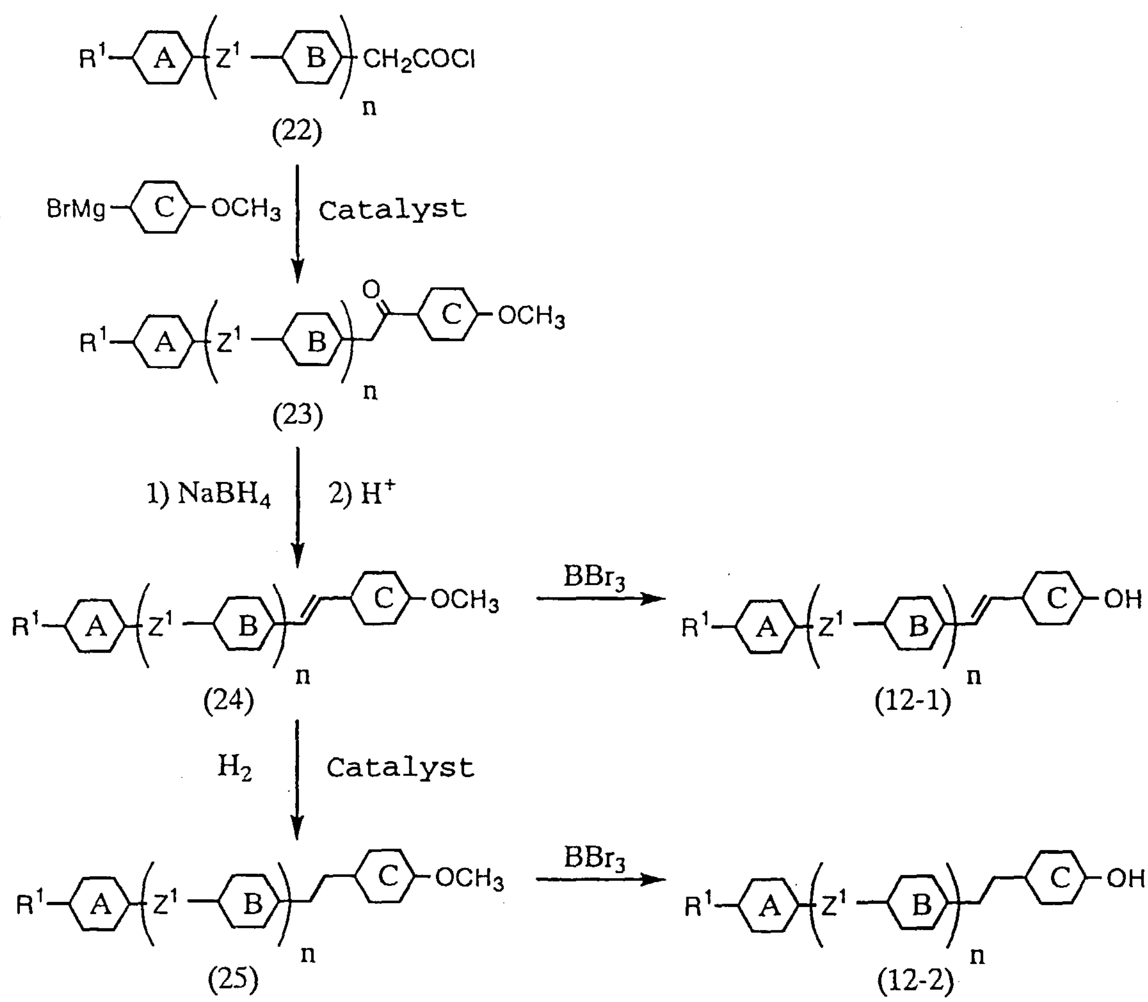


wherein rings A, B, and C, and $\ell$ have the same meaning as described above, and n is 0 or 1.

While the derivative wherein $R^1$ is an alkenyl group can be produced by various known procedures of organic synthesis, the derivative can preferably produced by the following method. That is, 1,4-cyclohexanedione monoethyleneketal (26) is reacted with the Grignard reagent prepared from bromoanisole derivative, the alcohol derivative thus formed is dehydrated by using a mineral acid such as sulfuric acid and hydrochloric acid, an organic acid such as p-toluenesulfonic acid, or a non-aqueous acidic ino exchange resin as catalyst, and then the cyclohexene derivative thus obtained is subjected to hydrogen reduction in the presence of a catalyst such as palladium-carbon and Raney nickel.

Subsequently, the product is subjected to deprotection of ketal with formic acid to obtain cyclohexanone derivative (28), and further the derivative (28) is reacted with boron tribromide to obtain ketophenol derivative (12-3). Ketophenol derivative expressed by the general formula (12-4) can be produced by using cyclohexanone derivative (27-1) or aldehyde derivative (27-2) in place of 1,4-cyclohexanedione monoethyleneketal.

Next, ketophenol derivative (12-3) is subjected to esterification with thioncarboxylic acid chloride, and the thiocarbonyl group is subjected to the geminal fluorination to produce cyclohexanone intermediate (30) in the same manner as in the case of the production of compounds of the formula (1) described above. In the same manner, derivative (31) can be produced from the derivative (12-4).

Derivative wherein $R^1$ is ethenyl group or 1 (E)-propenyl group can preferably be produced by the following method by using the cyclohexanone intermediate (30) as intermediate of the synthesis. That is, the ylide which is prepared by reacting methoxymethyltriphenylphosphonium chloride with a base such as a sodium alkoxide and alkyl lithium in tetrahydrofuran (hereinafter abbreviated as THF) according to a method described in Organic Reactions, Vol. 14, Chapter 3 is reacted with cyclohexanone intermediate (30), and then the product is heated to reflux in the co-presence of formic acid to produce aldehyde intermediate (32). Subsequently, the aldehyde intermediate (32) is reacted with the ylide which is prepared by reacting methyltriphenylphosphonium bromide or ethyltriphenylphosphonium bromide with a base such as a sodium alkoxide and alkyl lithium to produce derivative (1-1) wherein $R^1$ is ethenyl group or derivative (1-3) wherein $R^1$ is 1-propenyl group. Derivative (1-3) is isomerized by reacting benzenesulfinic acid or p-toluenesulfinic acid thereto according to a method described in Japanese Patent Publication No. Hei 4-30382 or subjected to inversion of olefin according to a method described in Japanese Patent Publication No. Hei 6-62462 to form a derivative wherein $R^1$ is 1(E)-propenyl group, when necessary.

According to the production methods described above, alkenyl derivatives expressed by the general formula (1-2) or (1-4) can be produced from cyclohexanone intermediate (31).

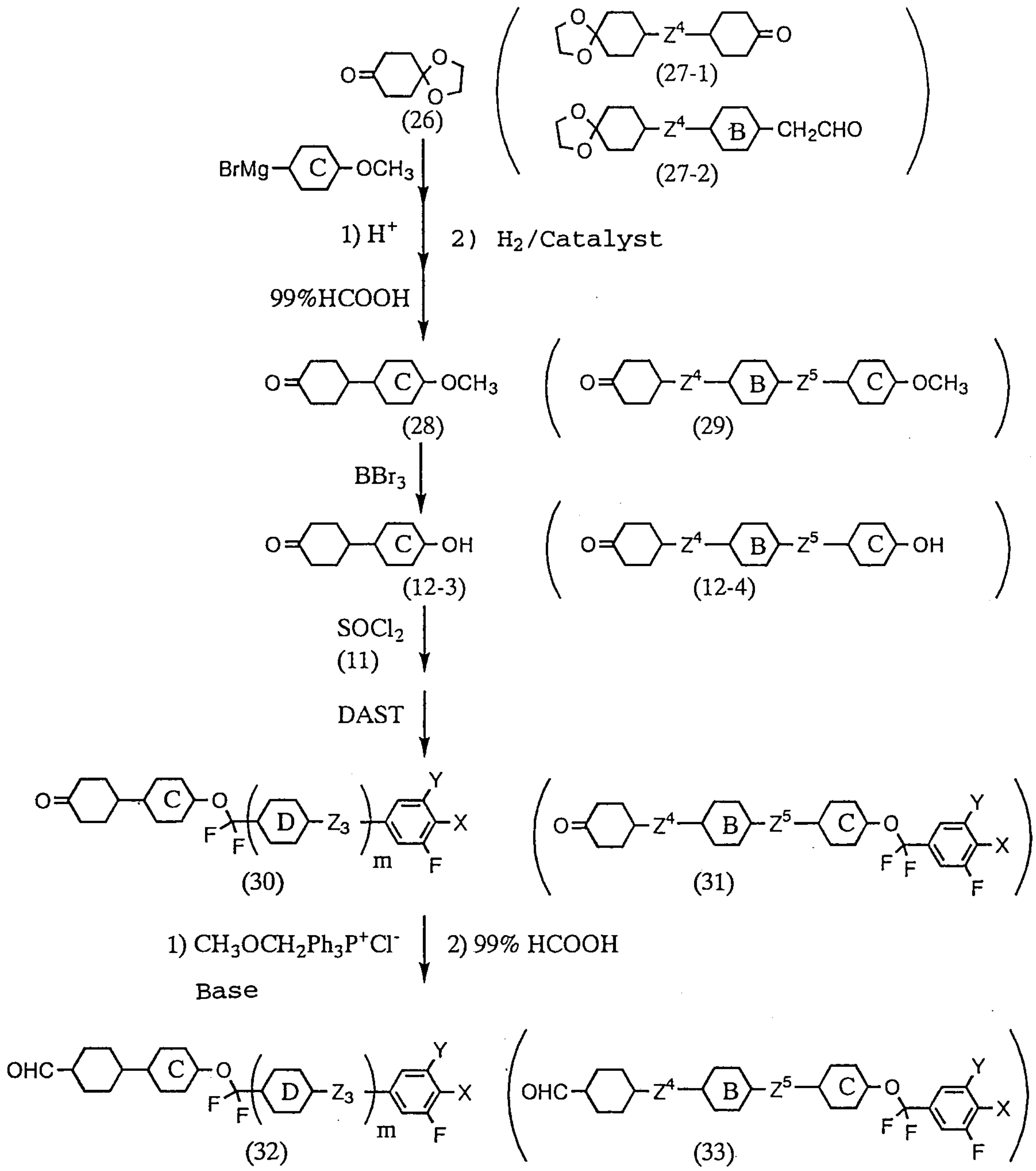
(26)
(27-1)
(27-2)
1) $H^+$
2) $H_2$/Catalyst
99%HCOOH
(28)
(29)
$BBr_3$
(12-3)
(12-4)
$SOCl_2$
(11)
DAST
(30)
(31)
1) $CH_3OCH_2Ph_3P^+Cl^-$
Base
2) 99% HCOOH
(32)
(33)

(32)((33))

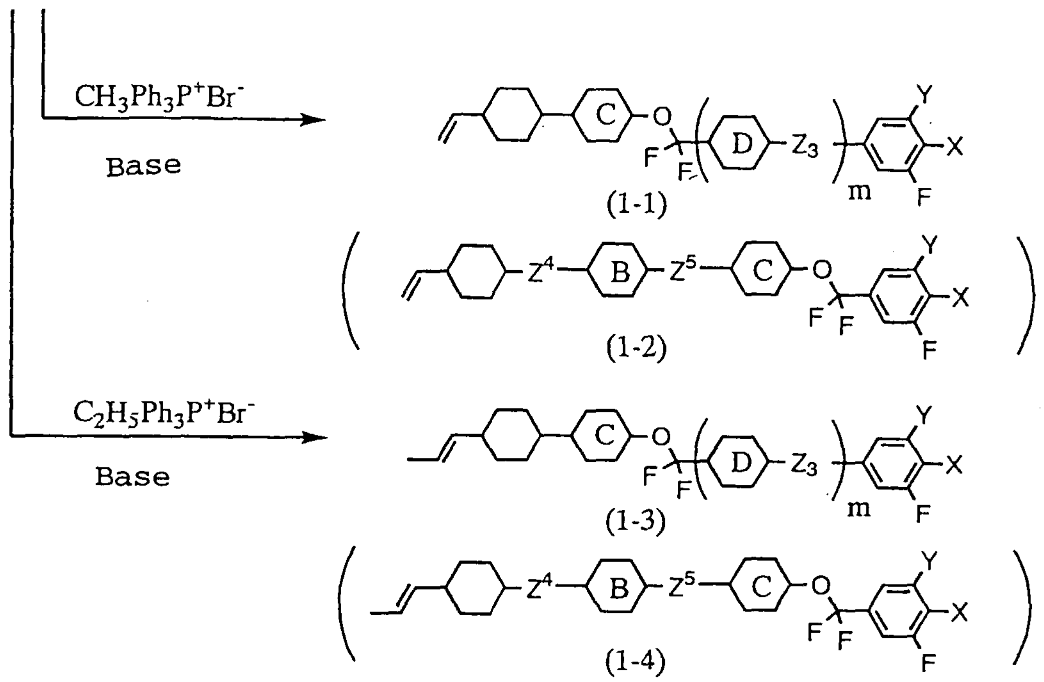


(1-1)

(1-2)

(1-3)

(1-4)

wherein rings B, C, and D, X, Y, and m have the same meaning as described above, and $Z^4$ and $Z^5$ independently represent $-CH_2CH_2-$ or single bond.

Derivative wherein $R^1$ is 3-butenyl group or 3(E)-pentenyl group can also preferably be produced by the following production method. That is, the ylide which is prepared by reacting 2-(1,3-dioxane-2-yl)ethyltriphenylphosphonium bromide with a base such as a sodium alkoxide or alkyl lithium, is reacted with the cyclohexanone intermediate (30) production method of which is described above, and then the product is subjected to hydrogen reduction in the presence of palladium-carbon catalyst and continuously heated to reflux together with formic acid to perform deprotection of ketal thereby produce aldehyde intermediate (33). Subsequently, aldehyde intermediate (33) is reacted with ylide which is prepared by reacting methyltriphenylphosphonium bromide or ethyltriphenylphosphonium bromide with a base such as a sodium alokoxide or alkyl lithium, to produce derivative (1-5) wherein $R^1$ is 3-butenyl group or derivative (1-7) wherein $R^1$ is 3-pentenyl group. Derivative (1-7) is isomerized by reacting benzenesulfinic acid or p-toluenesulfinic acid thereto according to a method described in Japanese Patent Publication No. Hei 4-30382, or subjected to inversion of olefin according to a method described in Japanese Patent Publication No. Hei 6-62462 to form a derivative wherein $R^1$ is 3(E)-pentenyl group, when necessary.

According to the production methods described above, alkenyl derivatives expressed by the general formula (1-6) or (1-8) can be produced from cyclohexanone derivative (31).

(30)((31))

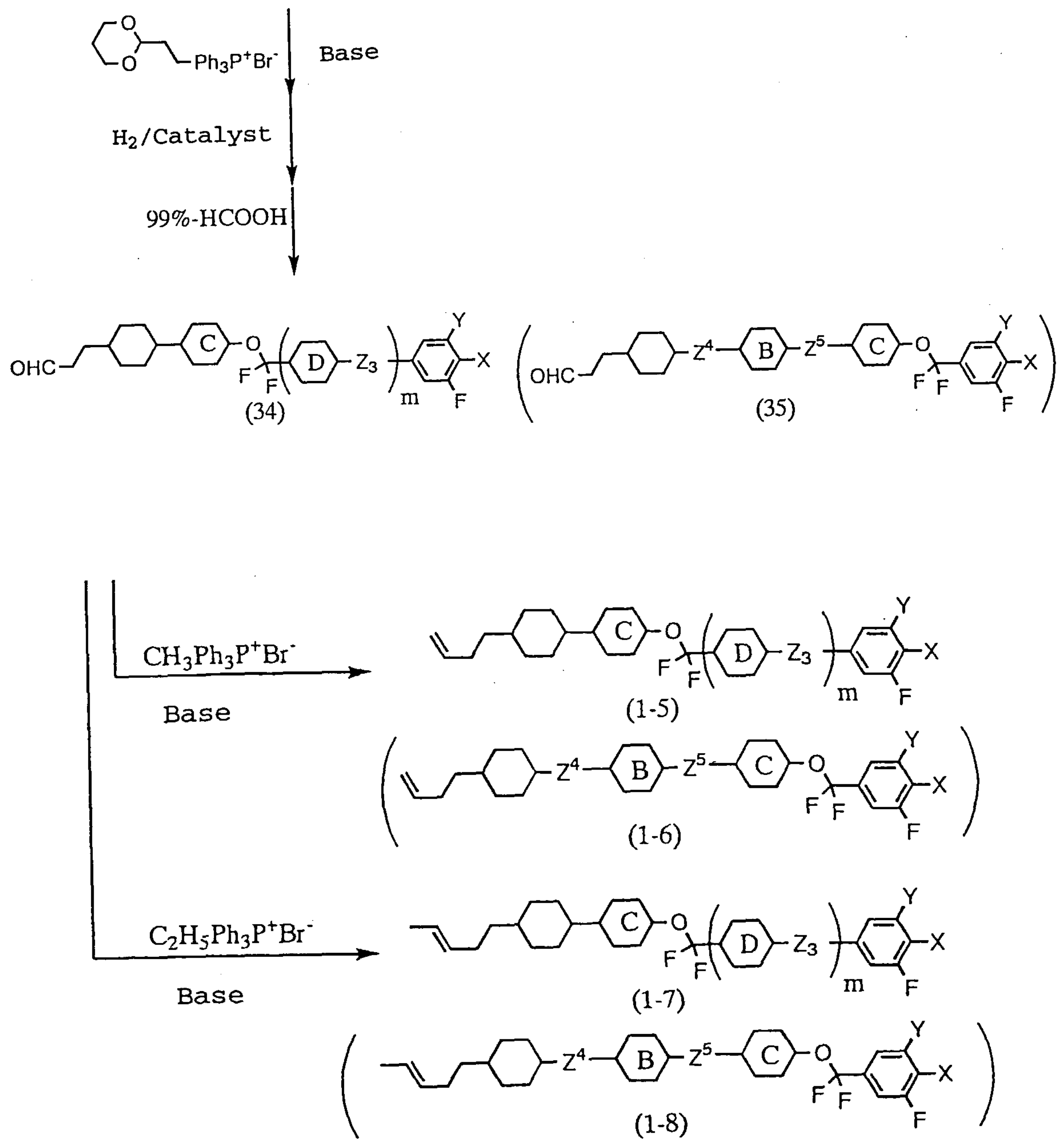


(34) (35)

(1-5)

(1-6)

(1-7)

(1-8)

wherein rings B, C, and D, X, Y, and m have the same meaning as described above, and $Z^4$ and $Z^5$ independently represent $-CH_2CH_2-$ or single bond.

[Examples]

Now, methods for producing the compounds of the present invention and use examples thereof will be described in more detail with reference to Examples. However, it should be understood that the scope of the present invention is by no means restricted by such specific Examples. In each of the Examples, Cr indicates crystal; N, nematic phase; S, smectic phase, and Iso, isotropic liquid; and the unit of all phase transition temperatures is °C.

Example 1

Preparation of difluoro-(4-(trans-propylcyclohexyl) phenyloxy) (3,4,5-trifluorophenyl)methane (Compound expressed by the general formula (1) wherein ring A represents 1,4-cyclohexylene group, ring C represents 1,4-phenylene group, ℓ and m are 0, $R^1$ represents n-$C_3H_7$, $Z^1$ represents single bond, and X and Y are F; Compound No. 1)

Process for the preparation are broadly divided into three steps, and each of the preparation steps are described in detail separately from one another below.

First step

In a 500 ml three-neck flask provided with a stirrer, thermometer, and nitrogen gas introducing tube, 4.8 g (199.1 mmol) of magnesium turnings were suspended in 100 ml of THF while stirring under atomsphere of nitrogen gas, and then 100 ml of a solution of 40 g (189.7 mmol) of 3,4,5-trifluorobromobenzene in THF was added dropwise thereto in 1.5 hours so that interior temperature did not exceed 50°C. The reaction solution was stirred for 2 hours while heating at 50°C with a hot water bath to age. Then, the flask was cooled with an ice water bath so that the interior temperature lowered down to 5°C, and 57.8 g (758.8 mmol) of carbon disulfide was added dropwise in 40 min so that the interior temperature did not exceed 10°C. The reaction solution was stirred for 30 min while keeping a temperature at lower than 10°C, raised up to room temperature, and then stirred for 2 hours. The reaction solution was cooled down to a temperature lower than 5°C again, and then 80 ml of 6N hydrochloric acid was added thereto to terminate the reaction. The reaction solution was extracted with 800 ml of diethyl ether, washed with 1,000 ml of water, and then dried over anhydrous magnesium sulfate. The diethyl ether was distilled off and the residue was concentrated to obtain 36.3 g of a deep murex solid. This solid was 3,4,5-trifluorophenyldithiocarboxylic acid.

Second step

In a 500 ml egg-plant type flask, 36.3 g of the 3,4,5-trifluorophenylditriocarboxylic acid obtained by the procedures described above was dissolved in 300 ml of diethyl ether, 103.8 g of thionyl chloride was added thereto at room temperature, and then the product was heated to reflux on a hot water bath for 8 hours. The diethyl ether and unreacted thionyl chloride were distilled off under a reduced pressure with an aspirator and the residue was concentrated to obtain 32.3 g of a deep murex oily product. This product was 3,4,5-trifluorophenylthioncarboxylic acid chloride. Then, in a 500 ml three-neck flask provided with a stirrer, thermometer, and nitrogen gas introducing tube, 27.9 g (127.9 mmol) of 4-(trans-4-propylcyclohexyl)phenol and 12.1 g (153.4 mmol) of pyridine were dissolved in 100 ml of toluene under nitrogen gas atmosphere, and 90 ml of a solution of 32.3 g of the 3,4,5-trifluorophenylthioncarboxylic acid chloride obtained by the procedures described above in toluene was added dropwise thereto while stirring at room temperature in 40 min. After finishing of the addition, the reaction solution was heated on a hot water bath so that the internal temperature reached 60°C and stirred for 3 hours to age. The reaction solution was cooled down to room temperature, 300 ml of water and 60 ml of 6N hydrochloric acid were added thereto, the toluene layer was separated, and then the water layer was extracted with 400 ml of toluene. The extract layer was washed with 500 ml of water, 100 ml of 2N aqueous solution of sodium hydroxide, and 500 ml of water in turn, and then dried over anhydrous magnesium sulfate. The toluene was distilled off under a reduced pressure and the residue was concentrated to obtain 45.2 g of a deep murex paste like product. The reaction product was purified by column chromatography using a silica gel as filler and heptane as eluent, and then recrystallized from heptane to obtain 20.3 g of pale yellow needle-like crystals. These crystals were thioncarboxylic acid-O-ester derivative (36).

Cr 88.0 Iso

$^1$H-NMR (δ ppm) 0.8-2.1 (16H, m), 2.5 (1H, m), 6.9 (2H, d, J=9.0 Hz), 7.3 (2H, d, J=9.0 Hz), and 7.9 (2H, m)

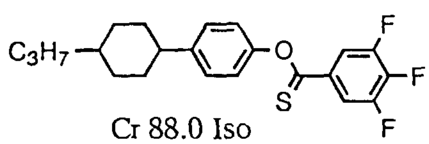

Cr 88.0 Iso

(36)

Third step

In a 300 ml egg-plant type flask, 10 g (25.5 mmol) of the thioncarboxylic acid-O-ester derivative (36) obtained by the procedures described above was dissolved in 60 ml of dichloromethane under nitrogen gas stream, 14.4 g (89.2 mmol) of DAST (diethylaminosulfur trifluoride) was added thereto at room temperature, and the mixture was stirred at room temperature for 25 hours. The reaction solution was added to 200 ml of an ice water to terminate the reaction, the dichloromethane layer was separated, and then the water layer was extracted with 100 ml of dichloromethane. The extract layer was washed with 200 ml of water, 50 ml of 2N aqueous solution of sodium hydroxide, and 200 ml of water in turn, and then dried over anhydrous magnesium sulfate. The dichloromethane was distilled off and the residue was concentrated to obtain 9.1 g of a pale yellow crystalline mixture. The reaction product was purified by column chromatography using a silica gel as filler and heptane as eluent, and then recrystallized from heptane to obtain 3.7 g of colorless needle-like crystals. These crystals were objective difluoro-(4-(trans-4-propylcyclohexyl)phenyloxy) (3,4,5-trifluorophenyl)methane (Compound No. 1).

Cr 59.2 Iso
$^{1}$H-NMR (δ ppm) 0.8-2.0 (16H, m), 2.5 (1H, m), 6.9-7.2 (4H, m), and 7.4 (2H, m)

Measured chart of $^{19}$F-NMR spectrum of Compound No. 1 is shown in Fig. 1.

Following compounds can be prepared according to the preparation method described above with the exception that, instead of 4-(trans-4-propylcyclohexyl)phenol, another 4-(trans-4-alkylcyclohexyl)phenol having a different chain length of alkyl group is used.

(Compound No. 2)

Difluoro-(4-(trans-4-methylcyclohexyl)phenyloxy) (3,4,5-trifluorophenyl)methane

(Compound No. 3)

Difluoro-(4-(trans-4-ethylcyclohexyl)phenyloxy) (3,4,5-trifluorophenyl)methane

(Compound No. 4)

Difluoro-(4-(trans-4-butylcyclohexyl)phenyloxy) (3,4,5-trifluorophenyl)methane

(Compound No. 5)

Difluoro-(4-(trans-4-pentylcyclohexyl)phenyloxy) (3,4,5-trifluorophenyl)methane

(Compound No. 6)

Difluoro-(4-(trans-4-hexylcyclohexyl)phenyloxy) (3,4,5-trifluorophenyl)methane

(Compound No. 7)

Difluoro-(4-(trans-4-heptylcyclohexyl)phenyloxy) (3,4,5-trifluorophenyl)methane

(Compound No. 8)

Difluoro-(4-(trans-4-octylcyclohexyl)phenyloxy) (3,4,5-trifluorophenyl)methane

8Compound No. 9)

Difluoro-(4-(trans-4-nonylcyclohexyl)phenyloxy) (3,4,5-trifluorophenyl)methane

(Compound No. 10)

Difluoro-(4-(trans-4-decylcyclohexyl)phenyloxy) (3,4,5-trifluorophenyl)methane

Following compounds can be prepared according to the preparation method described above with the exception that 2-fluoro-4-(trans-4-alkylcyclohexyl)phenol or 2,6-difluoro-4-(trans-4-alkylcyclohexyl)phenol is used in place of 4-(trans-4-propylcyclohexyl)phenol.

(Compound No. 11)

Difluoro-(2-fluoro-4-(trans-4-ethylcyclohexyl)phenyloxy) (3,4,5-trifluorophenyl)methane

Cr 26.1 Iso

(Compound No. 12)

Difluoro-(2-fluoro-4-(trans-4-propylcyclohexyl)phenyloxy) (3,4,5-trifluorophenyl)methane

(Compound No. 13)

Difluoro-(2-fluoro-4-(trans-4-pentylcyclohexyl)phenyloxy) (3,4,5-trifluorophenyl)methane

(Compound No. 14)

Difluoro-(2,6-difluoro-4-(trans-4-ethylcyclohexyl)phenyloxy) (3,4,5-trifluorophenyl)methane

(Compound No. 15)

Difluoro-(2,6-difluoro-4-(trans-4-propylcyclohexyl) phenyloxy) (3,4,5-trifluorophenyl)methane

(Compound No. 16)

Difluoro-(2,6-difluoro-4-(trans-4-pentylcyclohexyl) phenyloxy) (3,4,5-trifluorophenyl)methane

Following compounds can be prepared according to the preparation method described above with the exception that, in stead of 3,4,5-trifluorobromobenzene, one of various other known bromobenzene derivatives is used.

(Compound No. 17)

Difluoro-(4-(trans-4-ethylcyclohexyl)phenyloxy) (3,4-difluorophenyl)methane

(Compound No. 18)

Difluoro-(4-(trans-4-propylcyclohexyl)phenyloxy) (3,4-difluorophenyl)methane

(Compound No. 19)

Difluoro-(4-(trans-4-pentylcyclohexyl)phenyloxy) (3,4-difluorophenyl)methane

(Compound No. 20)

Difluoro(4-(trans-4-ethylcyclohexyl)phenyloxy) (3-fluoro-4-chlorophenyl)methane

(Compound No. 21)

Difluoro(4-(trans-4-propylcyclohexyl)phenyloxy) (3-fluoro-4-chlorophenyl)methane

(Compound No. 22)

Difluoro(4-(trans-4-pentylcyclohexyl)phenyloxy) (3-fluoro-4-chlorophenyl)methane

(Compound No. 23)

Difluoro-(4-(trans-4-ethylcyclohexyl)phenyloxy) (3-fluoro-4-trifluoromethylphenyl)methane

(Compound No. 24)

Difluoro-(4-(trans-4-propylcyclohexyl)phenyloxy) (3-fluoro-4-trifluoromethylphenyl)methane

(Compound No. 25)

Difluoro-(4-(trans-4-pentylcyclohexyl)phenyloxy) (3-fluoro-4-trifluoromethylphenyl)methane

(Compound No. 26)

Difluoro-(4-(trans-4-ethylcyclohexyl)phenyloxy) (3-fluoro-4-trifluoromethoxyphenyl)methane

(Compound No. 27)

Difluoro-(4-(trans-4-propylcyclohexyl)phenyloxy) (3-fluoro-4-trifluoromethoxyphenyl)methane

Cr 15.2 SB 66.3 Iso

(Compound No. 28)

Difluoro-(4-(trans-4-pentylcyclohexyl)phenyloxy) (3-fluoro-4-trifluoromethoxyphenyl)methane

(Compound No. 29)

Difluoro-(4-(trans-4-ethylcyclohexyl)phenyloxy) (3-fluoro-4-difluoromethoxyphenyl)methane

(Compound No. 30)

Difluoro-(4-(trans-4-propylcyclohexyl)phenyloxy) (3-fluoro-4-difluoromethoxyphenyl)methane

(Compound No. 31)

Difluoro-(4-(trans-4-pentylcyclohexyl)phenyloxy) (3-fluoro-4-difluoromethoxyphenyl)methane

(Compound No. 32)

Difluoro-(4-(trans-4-ethylcyclohexyl)phenyloxy) (3-fluoro-4-cyanophenyl)methane

(Compound No. 33)

Difluoro-(4-(trans-4-propylcyclohexyl)phenyloxy) (3-fluoro-4-cyanophenyl)methane

(Compound No. 34)

Difluoro-(4-(trans-4-pentylcyclohexyl)phenyloxy) (3-fluoro-4-cyanophenyl)methane

(Compound No. 35)

Difluoro-(4-(trans-4-ethylcyclohexyl)phenyloxy) (3,5-

difluoro-4-trifluoromethylphenyl)methane

(Compound No. 36)

Difluoro-(4-(trans-4-propylcyclohexyl)phenyloxy) (3,5-difluoro-4-trifluoromethylphenyl)methane

(Compound No. 37)

Difluoro-(4-(trans-4-pentylcyclohexyl)phenyloxy) (3,5-difluoro-4-trifluoromethylphenyl)methane

(Compound No. 38)

Difluoro-(4-(trans-4-ethylcyclohexyl)phenyloxy) (3,5-difluoro-4-trifluoromethoxyphenyl)methane

(Compound No. 39)

Difluoro-(4-(trans-4-propylcyclohexyl)phenyloxy) (3,5-difluoro-4-trifluoromethoxyphenyl)methane

(Compound No. 40)

Difluoro-(4-(trans-4-pentylcyclohexyl)phenyloxy) (3,5-difluoro-4-trifluoromethoxyphenyl)methane

(Compound No. 41)

Difluoro-(4-(trans-4-ethylcyclohexyl)phenyloxy) (3,5-difluoro-4-difluoromethoxyphenyl)methane

(Compound No. 42)

Difluoro-(4-(trans-4-propylcyclohexyl)phenyloxy) (3,5-difluoro-4-difluoromethoxyphenyl)methane

(Compound No. 43)

Difluoro-(4-(trans-4-pentylcyclohexyl)phenyloxy) (3,5-difluoro-4-difluoromethoxyphenyl)methane

(Compound No. 44)

Difluoro-(4-(trans-4-ethylcyclohexyl)phenyloxy) (3,5-

difluoro-4-cyanophenyl)methane

(Compound No. 45)

Difluoro-(4-(trans-4-propylcyclohexyl)phenyloxy) (3,5-difluoro-4-cyanophenyl)methane

(Compound No. 46)

Difluoro-(4-(trans-4-pentylcyclohexyl)phenyloxy) (3,5-difluoro-4-cyanophenyl)methane

Example 2

Preparation of difluoro-(4-(2-(trans-4-propylcyclohexyl) ethyl)phenyloxy) (3,4,5-trifluorophenyl)methane (Compound expressed by the general formula (1) wherein ring A represents 1,4-cyclohexylene group, ring C represents 1,4-phenylene group, ℓ and m are 0, $R^1$ represents n-$C_3H_7$, $Z^1$ represents 1,2-ethylene, and X and Y represent F; Compound No. 47)

Objective difluoro-(4-(2-(trans-4-propylcyclohexyl)ethyl) phenyloxy) (3,4,5-trifluorophenyl)methane was synthesized by repeating Example 1 with the exception that 4-(2-(trans-4-propylcyclohexyl)ethyl)phenol was used in place of 4-(trans-4-propylcyclohexyl)phenol in its second step.

Following compounds can be prepared according the preparation method described above with the exception that, instead of 4-(2-(trans-4-propylcyclohexyl)ethyl)phenol, another 4-(2-(trans-4-alkylcyclohexyl)ethyl)phenol having a different chain length of alkyl group is used.

(Compound No. 48)

Difluoro-(4-(2-(trans-4-methylcyclohexyl)ethyl)phenyloxy) (3,4,5-trifluorophenyl)methane

(Compound No. 49)

Difluoro-(4-(2-(trans-4-ethylcyclohexyl)ethyl)phenyloxy)(3,4,5-trifluorophenyl)methane

(Compound No. 50)

Difluoro-(4-(2-(trans-4-butylcyclohexyl)ethyl)phenyloxy)(3,4,5-trifluorophenyl)methane

(Compound No. 51)

Difluoro-(4-(2-(trans-4-pentylcyclohexyl)ethyl)phenyloxy)(3,4,5-trifluorophenyl)methane

(Compound No. 52)

Difluoro-(4-(2-(trans-4-hexylcyclohexyl)ethyl)phenyloxy)(3,4,5-trifluorophenyl)methane

(Compound No. 53)

Difluoro-(4-(2-(trans-4-heptylcyclohexyl)ethyl)phenyloxy)(3,4,5-trifluorophenyl)methane

(Compound No. 54)

Difluoro-(4-(2-(trans-4-octylcyclohexyl)ethyl)phenyloxy)(3,4,5-trifluorophenyl)methane

(Compound No. 55)

Difluoro-(4-(2-(trans-4-nonylcyclohexyl)ethyl)phenyloxy)(3,4,5-trifluorophenyl)methane

(Compound No. 56)

Difluoro-(4-(2-(trans-4-decylcyclohexyl)ethyl)phenyloxy)(3,4,5-trifluorophenyl)methane

Following compounds can be prepared according to the preparation method described above and in Example 1 with the exception that, instead of 3,4,5-trifluorobromobenzene, one of other known bromobenzene derivatives is used.

(Compound No. 57)

Difluoro-(4-(2-(trans-4-ethylcyclohexyl)ethyl)phenyloxy)(3,4-difluorophenyl)methane

(Compound No. 58)

Difluoro-(4-(2-(trans-4-propylcyclohexyl)ethyl)phenyloxy)(3,4-difluorophenyl)methane

(Compound No. 59)

Difluoro-(4-(2-(trans-4-pentylcyclohexyl)ethyl)phenyloxy)(3,4-difluorophenyl)methane

(Compound No. 60)

Difluoro-(4-(2-(trans-4-ethylcyclohexyl)ethyl)phenyloxy) (3-fluoro-4-chlorophenyl)methane

(Compound No. 61)

Difluoro-(4-(2-(trans-4-propylcyclohexyl)ethyl)phenyloxy)(3-fluoro-4-chlorophenyl)methane

(Compound No. 62)

Difluoro-(4-(2-(trans-4-pentylcyclohexyl)ethyl)phenyloxy)(3-fluoro-4-chlorophenyl)methane

(Compound No. 63)

Difluoro-(4-(2-(trans-4-ethylcyclohexyl)ethyl)phenyloxy) (3-fluoro-4-trifluoromethylphenyl)methane

(Compound No. 64)

Difluoro-(4-(2-(trans-4-propylcyclohexyl)ethyl)phenyloxy)(3-fluoro-4-trifluoromethylphenyl)methane

(Compound No. 65)

Difluoro-(4-(2-(trans-4-pentylcyclohexyl)ethyl)phenyloxy)

(3-fluoro-4-trifluoromethylphenyl)methane

(Compound No. 66)

Difluoro-(4-(2-(trans-4-ethylcyclohexyl)ethyl)phenyloxy) (3-fluoro-4-trifluoromethoxyphenyl)methane

(Compound No. 67)

Difluoro-(4-(2-(trans-4-propylcyclohexyl)ethyl)phenyloxy) (3-fluoro-4-trifluoromethoxyphenyl)methane

(Compound No. 68)

Difluoro-(4-(2-(trans-4-pentylcyclohexyl)ethyl)phenyloxy) (3-fluoro-4-trifluoromethoxyphenyl)methane

(Compound No. 69)

Difluoro-(4-(2-(trans-4-ethylcyclohexyl)ethyl)phenyloxy) (3-fluoro-4-difluoromethoxyphenyl)methane

(Compound No. 70)

Difluoro-(4-(2-(trans-4-propylcyclohexyl)ethyl)phenyloxy) (3-fluoro-4-difluoromethoxyphenyl)methane

(Compound No. 71)

Difluoro-(4-(2-(trans-4-pentylcyclohexyl)ethyl)phenyloxy) (3-fluoro-4-difluoromethoxyphenyl)methane

(Compound No. 72)

Difluoro-(4-(2-(trans-4-ethylcyclohexyl)ethyl)phenyloxy) (3-fluoro-4-cyanophenyl)methane

(Compound No. 73)

Difluoro-(4-(2-(trans-4-propylcyclohexyl)ethyl)phenyloxy) (3-fluoro-4-cyanophenyl)methane

(Compound No. 74)

Difluoro-(4-(2-(trans-4-pentylcyclohexyl)ethyl)phenyloxy)

(3-fluoro-4-cyanophenyl)methane

(Compound No. 75)

Difluoro-(4-(2-(trans-4-ethylcyclohexyl)ethyl)phenyloxy)(3,5-difluoro-4-trifluoromethylphenyl)methane

(Compound No. 76)

Difluoro-(4-(2-(trans-4-propylcyclohexyl)ethyl)phenyloxy)(3,5-difluoro-4-trifluoromethylphenyl)methane

(Compound No. 77)

Difluoro-(4-(2-(trans-4-pentylcyclohexyl)ethyl)phenyloxy)(3,5-difluoro-4-trifluoromethylphenyl)methane

(Compound No. 78)

Difluoro-(4-(2-(trans-4-ethylcycloheyxl)ethyl)phenyloxy)(3,5-difluoro-4-trifluoromethoxyphenyl)methane

(Compound No. 79)

Difluoro-(4-(2-(trans-4-propylcycloheyxl)ethyl)phenyloxy)(3,5-difluoro-4-trifluoromethoxyphenyl)methane

(Compound No. 80)

Difluoro-(4-(2-(trans-4-pentylcycloheyxl)ethyl)phenyloxy)(3,5-difluoro-4-trifluoromethoxyphenyl)methane

(Compound No. 81)

Difluoro-(4-(2-(trans-4-ethylcyclohexyl)ethyl)phenyloxy)(3,5-difluoro-4-difluoromethoxyphenyl)methane

(Compound No. 82)

Difluoro-(4-(2-(trans-4-propylcyclohexyl)ethyl)phenyloxy)(3,5-difluoro-4-difluoromethoxyphenyl)methane

(Compound No. 83)

Difluoro-(4-(2-(trans-4-pentylcyclohexyl)ethyl)phenyloxy)

(3,5-difluoro-4-difluoromethoxyphenyl)methane

(Compound No. 84)

Difluoro-(4-(2-(trans-4-ethylcyclohexyl)ethyl)phenyloxy)

(3,5-difluoro-4-cyanophenyl)methane

(Compound No. 85)

Difluoro-(4-(2-(trans-4-propylcyclohexyl)ethyl)phenyloxy)

(3,5-difluoro-4-cyanophenyl)methane

(Compound No. 86)

Difluoro-(4-(2-(trans-4-pentylcyclohexyl)ethyl)phenyloxy)

(3,5-difluoro-4-cyanophenyl)methane

Example 3

Preparation of difluoro-(4-(trans-4-ethenylcyclohexyl) phenyloxy) (3,4,5-trifluorophenyl)methane (Compound expressed by the general formula (1) wherein ring A represents 1,4-cyclohexylene group, ring C represents 1,4-phenylene group, ℓ and m are 0, $R^1$ represents ethenyl group, $Z^1$ represents a covalent bond, and X and Y are F; Compound No. 87)

Preparation process is broadly divided into three steps of

1) Synthesis of 4-(4-hydroxyphenyl)cyclohexanone (12-3),
2) Synthesis of an intermediate of cyclohexanone (30-1), and
3) Preparation of difluoro-(4-(trans-4-ethenylcyclohexyl)phenyloxy) (3,4,5-trifluorophenyl)methane.

The steps are described in detail separately from one another.

[Synthesis of 4-(4-hydroxyphenyl)cyclohexanone (12-3)]

First step

In a 1 ℓ three-neck flask provided with a stirrer, thermometer, and nitrogen gas introducing tube, 5.9 g (242.2 mmol) of magnesium turnings were suspended in 100 ml of THF while stirring under nitrogen gas atmosphere, and 200 ml of a solution of 43.1 g (230.7 mmol) of 4-bromoanisole in THF was added dropwise in 80 min so that the internal temperature did not exceed 50°C. The reaction solution was stirred for 2 hours while heating at 50°C on a hot water bath to age. Subsequently, 30.0 g (192.2 mmol) of 1,4-cyclohexanedionemonoethylene ketal was added dropwise thereto at room temperature in 40 min so that the internal temperature did not exceed 60°C. The reaction solution was stirred on a hot water bath while maintaining 50°C for 2 hours. The reaction solution was cooled with an ice water and then 100 ml of a saturated aqueous solution of ammonium chloride was added thereto to terminate the reaction. The reaction solution was extracted with 400 ml of toluene, washed with 1,000 ml of water, and then dried over anhydrous magnesium sulfate. The toluene was distilled off under a reduced pressure and the residue was concentrated to obtain 48.2 g of a brown solid. Then, the reaction product was dissolved in 240 ml of toluene in a 1 ℓ egg-plant type flask provided with a Dean and Stark dehydrating tube, 2.4 g of a non-aqueous acidic ion exchange resin (Amberlist) was added thereto as acid catalyst, and then the reaction product was heated to reflux while stirring for 3 hours. The catalyst was filtered off, the toluene was distilled off under a reduced pressure, and the residue was concentrated, purified by column chromatography using a silica gel as filler and toluene-ethyl acetate mixed solvent as eluent, and then recrystallized

from toluene to obtain 35.5 g of crystals.

In a 1 ℓ egg-plant type flask, 35.5 g of the reaction product obtained by the procedures described above was dissolved in 200 ml of toluene/ethanol (1/1) mixed solvent, 1.8 g of 5 % palladium-carbon catalyst was added thereto, and the reaction product was subjected to hydrogenation reaction at room temperature under a hydrogen gas pressure of 1 to 2 kg/cm$^2$ for 7 hours. From the reaction solution, the catalyst was filtered off and then the solution was concentrated to obtain 32.6 g of a reaction product. The reaction product was dissolved as it was in 100 ml of toluene in a 300 ml three-neck flask provided with a stirrer and thermometer, 24.4 g of 99 % formic acid was added thereto, and then heated to reflux while stirring for 2 hours. Water in an amount of 300 ml was added to the reaction product, the toluene layer was separated and then the water layer was extracted with 200 ml of toluene. The extract layer was washed with 800 ml of water and then dried over anhydrous magnesium sulfate. The toluene was distilled off under a reduced pressure, and the residue was concentrated to obtain 21.2 g of a reaction product. This product was 4-(methoxyphenyl) cyclohexanone.

Dichloromethane in an amount of 500 ml was added to a 1 ℓ egg-plant type flask provided with a stirrer, thermometer, and nitrogen gas introducing tube, and cooled down to a temperature lower than -50°C with a dry ice-acetone bath, 52.0 g (207.6 mmol) of boron tribromide was added dropwise thereto while maintaining a temperature lower than -50°C in 20 min, and then 100 ml of a solution of 21.2 g (103.8 mmol) of the 4-(methoxyphenyl)cyclohexanone obtained by the procedures described above in dichloromethane was added dropwise while maintaining the same temperature in 30 min. Then, the solution was stirred at the same temperature for 1 hour, raised up to room temperature, and was further stirred for 3 hours. The reaction solution was gradually added dropwise in 1 ℓ of water. After termination of the reaction, the dichloromethane layer was separated, and the water layer was extracted with 500 ml of dichloromethane. The extract layer was washed with 500 ml of water, 200 ml of 2N aqueous solution of sodium hydroxide, and 500 ml of water in turn, and then dried over anhydrous magnesium sulfate. From the reaction solution, the dichloromethane was distilled off and the residue was concentrated to obtain 19.5 g of a reaction product. The reaction product was recrystallized from a mixed solvent of toluene-heptane to obtain 14.8 g of colorless crystals. These crystals were 4-(4-hydroxyphenyl)cyclohexanone (12-3).

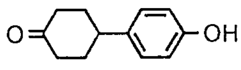

(12-3)

[Synthesis of cyclohexanone intermediate (30-1)]

Third step

In a 300 ml three-neck flask provided with a stirrer, thermometer, and nitrogen gas introducing tube, 14.8 g (78.0 mmol) of 4-(4-hydroxyphenyl)cyclohexanone (12-3) was dissolved in 30 ml of toluene under nitrogen gas atmosphere, 8.0 g (101.4 mmol) of pyridine was added thereto, and then 50 ml of a solution of 21.3 g (101.4 mmol) of the 3,4,5-trifluorophenylthioncarboxylic acid chloride which was synthesized by the procedures similar to the methods described in the first and second steps of Example 1, in toluene was added dropwise in 15 min. After finishing of the dropping, the reaction solution was heated on a hot water bath so that the internal temperature reached 60°C, and stirred for 3 hours to age. The reaction solution was cooled down to room temperature, 100 ml of water and 50 ml of 6N hydrochloric aicd were added thereto to separate a toluene layer, and then the water layer was extracted with 200 ml of toluene. The extract layer was washed with 200 ml of water, 50 ml of 2N aqueous solution of sodium hydroxide, and 300 ml of water in turn, and then dried over anhydrous magnesium sulfate. The toluene was distilled off under a reduced pressure and the residue was concentrated to obtain 28.4 g of a deep murex paste like product. The reaction product was purified by column chromatography using a silica gel as filler and a mixed solvent of heptane/toluene as eluent, and then recrystallized from heptane to obtain 20.4 g of pale yellow needle like crystals. These crystals were thioncarboxylic acid-O-ester derivative (37).

Fourth step

In a 300 ml egg-plant type flask provided with a nitrogen gas introducing tube, 10.0 g (27.5 mmol) of the thioncarboxylic acid-O-ester derivative (37) obtained by the procedures described above was dissolved in 50 ml of dichloromethane under nitrogen gas atmosphere, 17.7 g (109.8 mmol) of PAST was added thereto at room temperature, and then the contents were stirred at room temperature for 25 hours. The reaction solution was added to 200 ml of an ice

water to terminate the reaction, the dichloromethane layer was separated, and then the water layer was extracted with 100 ml of dichloromethane. The extract layer was washed with 200 ml of water, 50 ml of 2N aqueous solution of sodium hydroxide, and 200 ml of water in turn, and then dried over anhydrous magnesium sulfate. The dichloromethane was distilled off and the residue was concentrated to obtain 9.9 g of a pale yellow crystalline mixture. The reaction product was purified by column chromatograhy using a silica gel as filler and a mixed solvent of heptane and toluene as eluent, and further recrystallized from heptane to obtain 6.4 g of colorless needle like crystals. These crystals were cyclohexanone intermediate (30-1).

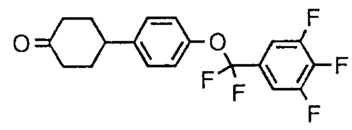 (30-1)

[Synthesis of difluoro(4-(trans-4-ethenylcyclohexyl)phenyloxy) (3,4, 5-trifluorophenyl)methane]

Fifth step

In a 300 ml three-neck flask provided with a stirrer, thermometer, and nitrogen gas introducing tube, 7.8 g (22.6 mmol) of methoxymethyltriphenylphosphonium chloride was dissolved in 40 ml of THF under nitrogen gas atmosphere, cooled down to a temperature lower than -50°C in a dry ice-acetone bath, 2.7 g (23.7 mmol) of potassium-t-butoxide was added thereto, and then the mixture was stirred while maintaining the same temperature of lower than -50°C for 2 hours to prepare a ylide. Subsequently, 20 ml of a solution of 6.4 g (17.4 mmol) of the cyclohexanone intermediate (30-1) obtained by the procedures described above in THF was added dropwise thereto at the same temperature in 10 min, stirred at the same temperature for 1 hour, raised up to room temperature, and then further stirred at room temperature for 8 hours. Water in an amount of 200 ml was added to the reaction solution to terminate the reaction, the THF layer was separated, and then the water layer was extracted with 100 ml of toluene. The extract layer was washed with 500 ml of water and dried over anhydrous magnesium sulfate, the solvent was distilled off under a reduced pressure, and then the residue was concentrated to obtain 13.0 g of a reaction product. The reaction product was purified by column chromatography using a silica gel as filler and toluene as eluent to obtain 6.2 g of a yellow brown product. Next, the reaction product was dissolved in 50 ml of toluene in a 200 ml egg-plant type flask, 2.9 g (62.8mmol) of 99 % formic acid was added thereto, and then heated to reflux for 2 hours. Water in an amount of 50 ml was added to the reaction solution and extracted with 50 ml of toluene. The extract layer was washed with 100 ml of water, 30 ml of 2N aqueous solution of sodium hydroxide, and 100 ml of water in turn, and dried over anhydrous magnesium sulfate. The solvent was distilled off under a reduced pressure and the residue was concentrated to obtain a reaction product. The reaction product was purified by column chromatography using a silica gel as filler and toluene as eluent to obtain 5.4 g of colorless crystals. These crystals were cyclohexanecarbaldehyde derivative (38).

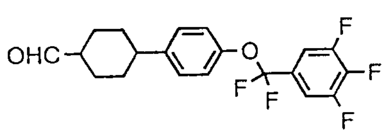 (38)

Sixth step

In a 100 ml three-neck flask provided with a stirrer, thermometer, and nitrogen gas introducing tube, 7.3 g (18.2 mmol) of methyltriphenylphosphonium iodide was suspended in 30 ml of THF under nitrogen gas atmosphere, cooled down to a temperature lower than -50°C in a dry ice-acetone bath while stirring, 2.1 g (18.7 mmol) of potassium-t-butoxide was added thereto, and then the mixture was stirred while maintaining the same temperature of lower than -50°C for 2 hours to prepare a ylide. Next, 15 ml of a solution of 5.4 g (13.9 mmol) of the cyclohexanecarbaldehyde derivative (38) obtained by the procedures described above in THF was added dropwise at the same temperature to the ylide over 15 min, stirred at the same temperature for 1 hour, raised up to room temperatue, and then further stirred at

room temperature for 8 hours. Water in an amount of 50 ml was added to the reaction solution to terminate the reaction, the THF layer was separated, and then the water layer was extracted with 50 ml of toluene. The extract layer was washed with 80 ml of water and then dried over anhydrous magnesium sulfate. The solvent was distilled off under a reduced pressure, and the residue was concentrated to obtain 4.8 g of a reaction product. The reaction product was purified by column chromatography using a silica gel as filler and heptane as eluent, and then recrystallized from heptane to obtain 2.3 g of colorless needle like compound. This was the objective difluoro-(4-(trans-4-ethenylcyclohexyl)phenyloxy) (3,4,5-trifluorophenyl)methane.

Following compounds can be prepared according to the preparation method described above with the exception that a thioncarboxylic acid chloride derivative which can be prepared from one of various known bromobenzene derivatives by the procedures described in the first and second steps of Example 1 is used in place of 3,4,5-trifluorophenylthioncarboxylic acid chloride in its third step.

(Compound No. 88)

Difluoro-(4-(trans-4-ethenylcyclohexyl)phenyloxy) (3,4-difluorophenyl)methane

(Compound No. 89)

Difuloro-(4-(trans-4-ethenylcyclohexyl)phenyloxy) (3-fluoro-4-chlorophenyl)methane

(Compound No. 90)

Difuloro-(4-(trans-4-ethenylcyclohexyl)phenyloxy) (3-fluoro-4-trifluoromethylphenyl)methane

(Compound No. 91)

Difuloro-(4-(trans-4-ethenylcyclohexyl)phenyloxy) (3-fluoro-4-trifluoromethoxyphenyl)methane

(Compound No. 92)

Difuloro-(4-(trans-4-ethenylcyclohexyl)phenyloxy) (3-fluoro-4-difluoromethoxyphenyl)methane

(Compound No. 93)

Difuloro-(4-(trans-4-ethenylcyclohexyl)phenyloxy) (3-fluoro-4-cyanophenyl)methane

(Compound No. 94)

Difluoro-(4-(trans-4-ethenylcyclohexyl)phenyloxy) (3,5-difluoro-4-trifluoromethylphenyl)methane

(Compound No. 95)

Difluoro-(4-(trans-4-ethenylcyclohexyl)phenyloxy) (3,5-difluoro-4-trifluoromethoxyphenyl)methane

(Compound No. 96)

Difluoro-(4-(trans-4-ethenylcyclohexyl)phenyloxy) (3,5-difluoro-4-difluoromethoxyphenyl)methane

(Compound No. 97)

Difluoro-(4-(trans-4-ethenylcyclohexyl)phenyloxy) (3,5-difluoro-4-4-cyanophenyl)methane

Following compounds can be prepared first by repeating the procedures in the preparation method described above with the exception that ethyltriphenylphosphonium iodide is used in place of methyltriphenylphosphonium iodide in the sixth step, and then by isomerize the resultant product by reacting it with benzenesulfinic acid or p-toluenesulfinic acid according to a method described in Japanese Patent Publication No. Hei 4-30382 or performing the steric inversion of olefin according to a method described in Japanese Patent Publication No. Hei 6-62462.

(Compound No. 98)

Difluoro-(4-(trans-4-((E)-1-propenyl)cyclohexyl)phenyloxy)(3,4,5-trifluorophenyl)methane

(Compound No. 99)

Difluoro-(4-(trans-4-((E)-1-propenyl)cyclohexyl)phenyloxy)(3,4-difluorophenyl)methane

(Compound No. 100)

Difluoro-(4-(trans-4-((E)-1-propenyl)cyclohexyl)phenyloxy)(3-fluoro-4-chlorophenyl)methane

(Compound No. 101)

Difluoro-(4-(trans-4-((E)-1-propenyl)cyclohexyl)phenyloxy)(3-fluoro-4-trifluoromethylphenyl)methane

(Compound No. 102)

Difluoro-(4-(trans-4-((E)-1-propenyl)cyclohexyl)phenyloxy)(3-fluoro-4-trifluoromethoxyphenyl)methane

(Compound No. 103)

Difluoro-(4-(trans-4-((E)-1-propenyl)cyclohexyl)phenyloxy)(3-fluoro-4-difluoromethoxyphenyl)methane

(Compound No. 104)

Difluoro-(4-(trans-4-((E)-1-propenyl)cyclohexyl)phenyloxy)(3-fluoro-4-cyanophenyl)methane

(Compound No. 105)

Difluoro-(4-(trans-4-((E)-1-propenyl)cyclohexyl)phenyloxy)(3,5-difluoro-4-trifluoromethylphenyl)methane

(Compound No. 106)

Difluoro-(4-(trans-4-((E)-1-propenyl)cyclohexyl)phenyloxy)(3,5-difluoro-4-trifluoromethoxyphenyl)methane

(Compound No. 107)

Difluoro-(4-(trans-4-((E)-1-propenyl)cyclohexyl)phenyloxy)(3,5-difluoro-4-difluoromethoxyphenyl)methane

(Compound No. 108)

Difluoro-(4-(trans-4-((E)-1-propenyl)cyclohexyl)phenyloxy)(3,5-difluoro-4-cyanophenyl)methane

Example 4

Preparation of difluoro-(4-(trans-4-(3-butenyl)cyclohexyl) phenyloxy) (3,4,5-trifluorophenyl)methane (Compound expressed by the general formula (1) wherein ring A represents 1,4-cycohexylene group, ring C represents 1,4-phenylene group, ℓ and m are 0, $R^1$ represents 3-butenyl group, $Z^1$ represents a covalent bond, and X and Y represent F; Compound No. 109)

Preparation process is broadly divided into two steps, and the steps are described in detail separately from each other.

First step

In a 300 ml three-neck flask provided with a stirrer, thermometer, and nitrogen gas introducing tube, 16.8 g (37.8 mmol) of 2-(1,3-dioxane-2-yl)ethyltriphenylphosphonium bromide was dissolved in 50 ml of THF under nitrogen gas atmosphere, and cooled down to a temperature lower than -50°C in a dry ice-acetone bath, 4.5 g (39.8 mmol) of potassium-t-butoxide was added thereto, and then the mixture was stirred while maintaing the same temperature of lower than -50°C for 2 hours to prepare a ylide. Then, 30 ml of a solution of 10 g (27.0 mmol) of the cyclohexanone intermediate (30-1) obtained by the procedures described in the fourth step of Example 3 in THF was added dropwise thereto at the same temperature in 10 min, stirred at the same temperature for 1 hour, raised up to room temperature, and then further stirred at room temperature for 8 hours. Water in an amount of 200 ml was added to the reaction solution to terminate the reaction, the THF layer was separated, and then the water layer was extracted with 100 ml of toluene. The extract layer was washed with 500 ml of water and then dried over anhydrous magnesium sulfate. The solvent was distilled off and the residue was concentrated to obtain 23.1 g of a reaction product. The reaction product was purified by column chromatography using a silica gel as filler and toluene as eluent to obtain 11.1 g of a yellow brown reaction product. In a 300 ml egg-plant type flask, the reaction product obtained by the procedures described above in an amount of 11.1 g was dissolved in 100 ml of a 1/1 mixed solvent of toluene/ethanol, 0.6 g of 5 % palladium-carbon catalyst was added thereto, and then the product was subjected to a hydrogenation reaction at room temperature under a hydrogen gas pressure of 1 to 2 $kg/cm^2$ for 6 hours. From the reaction solution, the catalyst was filtered off, and the filtrate (filtered solution) was concentrated to obtain 11.1 g of a reactioin product. In a 300 ml three-neck flask, the reaction product was dissolved as it was in 50 ml of toluene, 4.4 g (95.2 mmol) of 99 % formic acid was added thereto, and then the mixture was heated to reflux for 2 hours. Water in an amount of 100 ml was added to the reaction product, the toluene layer was separated, and then the water layer was extracted with 100 ml of toluene. The extract layer was washed with 400 ml of water and then dried over anhydrous magnesium sulfate. The toluene was distilled off under a reduced pressure and the residue was concentrated to obtain 8.5 g of a reaction product. This product was aldehyde derivative (39).

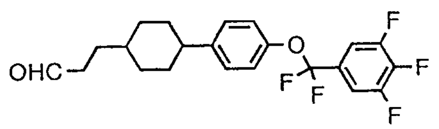

(39)

Second step

In a 100 ml three-neck flask provided with a stirrer, thermometer, and nitrogen gas introducing tube, 10.9 g (26.9 mmol) of methyltriphenylphosphonium iodide was suspended in 50 ml of THF under nitrogen gas atmosphere, and cooled down to a temperature lower than -50°C in a dry ice-acetone bath while stirring, 3.2 g (28.2 mmol) of potassium-t-butoxide was added thereto, and then the mixture was stirred while maintaining the same temperature of lower than -50°C to prepare a ylide. Subsequently, 25 ml of a solution of 8.5 g (20.7 mmol) of the aldehyde derivative (39) obtained by the procedures described above was added dropwise thereto at the same temperature in 10 min, and the contents were stirred at the same temperature for 1 hour, raised up to room temperature, and then further stirred at room temperature for 8 hours. Water in an amount of 50 ml was added to the reaction solution to terminate the reaction, the THF layer was separated, and then the water layer was extracted with 50 ml of toluene. The extract layer was washed with 80 ml of water and then dried over anhydrous magnesium sulfate. The solvent was distilled off under a reduced pressure, and the residue was concentrated to obtain 15.4 g of a reaction product. The reaction product was purified by column chromatography using a silica gel as filler and heptane as eluent, and further recrystallized from heptane to obtain 3.6 g of a colorless needle like compound. This compound was the objective difluoro-(4-(trans-4-(3-butenyl)cyclohexyl)phenyloxy) (3,4,5-trifluorophenyl)methane.

Following compounds can be prepared according to the ppreparation method described above with the exception that cyclohexanone derivative (30) prepared from one of various known bromobenzene derivatives is used in place of cyclohexanone derivative (30-1)

(Compound No. 110)

Difluoro-(4-(trans-4-(3-butenyl)cyclohexyl)phenyloxy) (3,4-difluorophenyl)methane

(Compound No. 111)

Difluoro-(4-(trans-4-(3-butenyl)cyclohexyl)phenyloxy) (3-fluoro-4-chlorophenyl)methane

(Compound No. 112)

Difluoro-(4-(trans-4-(3-butenyl)cyclohexyl)phenyloxy) (3-fluoro-4-trifluoromethylphenyl)methane

(Compound No. 113)

Difluoro-(4-(trans-4-(3-butenyl)cyclohexyl)phenyloxy) (3-fluoro-4-trifluoromethoxyphenyl)methane

(Compound No. 114)

Difluoro-(4-(trans-4-(3-butenyl)cyclohexyl)phenyloxy) (3-fluoro-4-difluoromethoxyphenyl)methane

(Compound No. 115)

Difluoro-(4-(trans-4-(3-butenyl)cyclohexyl)phenyloxy) (3-fluoro-4-cyanophenyl)methane

(Compound No. 116)

Difluoro-(4-(trans-4-(3-butenyl)cyclohexyl)phenyloxy) (3,5-difluoro-4-trifluoromethylphenyl)methane

(Compound No. 117)

Difluoro-(4-(trans-4-(3-butenyl)cyclohexyl)phenyloxy) (3,5-difluoro-4-trifluoromethoxyphenyl)methane

(Compound No. 118)

Difluoro-(4-(trans-4-(3-butenyl)cyclohexyl)phenyloxy) (3,5-difluoro-4-difluoromethoxyphenyl)methane

(Compound No. 119)

Difluoro-(4-(trans-4-(3-butenyl)cyclohexyl)phenyloxy) (3,5-difluoro-4-cyanophenyl)methane

Following compounds can be prepared first by repeating the procedures in the preparation method described above with the exception that ethyltriphenylphosphonium iodide is used in place of methyltriphenylphosphonium iodide in the second step, and then by isomerize the resultant product by reacting it with benzenesulfinic acid or p-toluenesulfinic acid according to a method described in Japanese Patent Publication No. Hei 4-30382 or performing the steric inversion of olefin according to a method described in Japanese Patent Publication No. 6-62462.

(Compound No. 120)

Difluoro-(4-(trans-4-((E)-3-pentenyl)cyclohexyl)phenyloxy)(3,4,5-trifluorophenyl)methane

(Compound No. 121)

Difluoro-(4-(trans-4-((E)-3-pentenyl)cyclohexyl)phenyloxy)(3,4-difluorophenyl)methane

(Compound No. 122)

Difluoro-(4-(trans-4-((E)-3-pentenyl)cyclohexyl)phenyloxy)(3-fluoro-4-chlorophenyl)methane

(Compound No. 123)

Difluoro-(4-(trans-4-((E)-3-pentenyl)cyclohexyl)phenyloxy)(3-fluoro-4-trifluoromethylphenyl)methane

(Compound No. 124)

Difluoro-(4-(trans-4-((E)-3-pentenyl)cyclohexyl)phenyloxy)(3-fluoro-4-trifluoromethoxyphenyl)methane

(Compound No. 125)

Difluoro-(4-(trans-4-((E)-3-pentenyl)cyclohexyl)phenyloxy)(3-fluoro-4-difluoromethoxyphenyl)methane

(Compound No. 126)

Difluoro-(4-(trans-4-((E)-3-pentenyl)cyclohexyl)phenyloxy)(3-fluoro-4-cyanophenyl)methane

(Compound No. 127)

Difluoro-(4-(trans-4-((E)-3-pentenyl)cyclohexyl)phenyloxy)(3,5-difluoro-4-trifluoromethylphenyl)methane

(Compound No. 128)

Difluoro-(4-(trans-4-((E)-3-pentenyl)cyclohexyl)phenyloxy)(3,5-difluoro-4-trifluoromethoxyphenyl)methane

(Compound No. 129)

Difluoro-(4-(trans-4-((E)-3-pentenyl)cyclohexyl)phenyloxy)(3,5-difluoro-4-difluoromethoxyphenyl)methane

(Compound No. 130)

Difluoro-(4-(trans-4-((E)-3-pentenyl)cyclohexyl)phenyloxy)(3,5-difluoro-4-cyanophenyl)methane

Example 5

Preparation of difluoro-(4-(trans-4-propylcyclohexyl) phenyloxy) (4'-(3,4,5-trifluoro)biphenyl)methane (Compound expressed by the general formula (1) wherein ring A represents 1,4-cyclohexylene group, ring C and ring D represent 1,4-phenylene group, $R^1$ represents n-$C_3H_7$, $Z^1$ and $Z^3$ represent a covalent bond, $\ell$ is 0, n is 1, and X and Y are F; Compound No. 131)

Difluoro-(4-(trans-4-propylcyclohexyl)phenyloxy) (4'-(3,4,5-trifluoro)biphenyl)methane was synthesized by carrying out the same procedures as described in Example 1 with the exception that 3,4,5-trifluoro-4'-bromobiphenyl was used in place of 3,4,5-trifluorobromobenzene in the first step of Example 1.

Following compounds can be prepared according to the preparation method described above and the preparation method shown in Example 1 with the exception that, instead of 4-(trans-4-propylcyclohexyl)phenol, amother 4-(trans-4-alkylcyclohexyl)phenol having a different alkyl chain is used, or that one of various known 3-fluoro-4'-bromobiphenyl derivatives is used in place of 3,4,5-trifluoro-4'-bromobiphenyl.

(Compound No. 132)

Difluoro-(4-(trans-4-methylcyclohexyl)phenyloxy) (4'-(3,4,5-trifluoro)biphenyl)methane

(Compound No. 133)

Difluoro-(4-(trans-4-ethylcyclohexyl)phenyloxy) (4'-(3,4,5-trifluoro)biphenyl)methane

(Compound No. 134)

Difluoro-(4-(trans-4-butylcyclohexyl)phenyloxy) (4'-(3,4,5-trifluoro)biphenyl)methane

(Compound No. 135)

Difluoro-(4-(trans-4-pentylcyclohexyl)phenyloxy) (4'-(3,4,5-trifluoro)biphenyl)methane

(Compound No. 136)

Difluoro-(4-(trans-4-hexylcyclohexyl)phenyloxy) (4'-(3,4,5-trifluoro)biphenyl)methane

(Compound No. 137)

Difluoro-(4-(trans-4-heptylcyclohexyl)phenyloxy) (4'-(3,4,5-trifluoro)biphenyl)methane

(Compound No. 138)

Difluoro-(4-(trans-4-octylcyclohexyl)phenyloxy) (4'-(3,4,5-trifluoro)biphenyl)methane

(Compound No. 139)

Difluoro-(4-(trans-4-nonylcyclohexyl)phenyloxy) (4'-(3,4,5-trifluoro)biphenyl)methane

(Compound No. 140)

Difluoro-(4-(trans-4-decylcyclohexyl)phenyloxy) (4'-(3,4,5-trifluoro)biphenyl)methane

(Compound No. 141)

Difluoro-(4-(trans-4-ethylcyclohexyl)phenyloxy) (4'-(3,4-difluoro)biphenyl)methane

(Compound No. 142)

Difluoro-(4-(trans-4-propylcyclohexyl)phenyloxy) (4'-(3,4-difluoro)biphenyl)methane

(Compound No. 143)

Difluoro-(4-(trans-4-pentylcyclohexyl)phenyloxy) (4'-(3,4-difluoro)biphenyl)methane

(Compound No. 144)

Difluoro-(4-(trans-4-ethylcyclohexyl)phenyloxy) (4'-(3-fluoro-4-chloro)biphenyl)methane

(Compound No. 145)

Difluoro-(4-(trans-4-propylcyclohexyl)phenyloxy) (4'-(3-fluoro-4-chloro)biphenyl)methane

(Compound No. 146)

Difluoro-(4-(trans-4-pentylcyclohexyl)phenyloxy) (4'-(3-fluoro-4-chloro)biphenyl)methane

(Compound No. 147)

Difluoro-(4-(trans-4-ethylcyclohexyl)phenyloxy) (4'-(3-fluoro-4-trifluoromethyl)biphenyl)methane

Compound No. 148)

Difluoro-(4-(trans-4-propylcyclohexyl)phenyloxy) (4'-(3-fluoro-4-trifluoromethyl)biphenyl)methane

(Compound No. 149)

Difluoro-(4-(trans-4-pentylcyclohexyl)phenyloxy) (4'-(3-fluoro-4-trifluoromethyl)biphenyl)methane

(Compound No. 150)

Difluoro-(4-(trans-4-ethylcyclohexyl)phenyloxy) (4'-(3-fluoro-4-trifluoromethoxy)biphenyl)methane

(Compound No. 151)

Difluoro-(4-(trans-4-propylcyclohexyl)phenyloxy) (4'-(3-fluoro-4-trifluoromethoxy)biphenyl)methane

(Compound No. 152)

Difluoro-(4-(trans-4-pentylcyclohexyl)phenyloxy) (4'-(3-fluoro-4-trifluoromethoxy)biphenyl)methane

(Compound No. 153)

Difluoro-(4-(trans-4-ethylcyclohexyl)phenyloxy) (4'-(3-fluoro-4-difluoromethoxy)biphenyl)methane

(Compound No. 154)

Difluoro-(4-(trans-4-propylcyclohexyl)phenyloxy) (4'-(3-fluoro-4-difluoromethoxy)biphenyl)methane

(Compound No. 155)

Difluoro-(4-(trans-4-pentylcyclohexyl)phenyloxy) (4'-(3-fluoro-4-difluoromethoxy)biphenyl)methane

(Compound No. 156)

Difluoro-(4-(trans-4-ethylcyclohexyl)phenyloxy) (4'-(3-fluoro-4-cyano)biphenyl)methane

(Compound No. 157)

Difluoro-(4-(trans-4-propylcyclohexyl)phenyloxy) (4'-(3-fluoro-4-cyano)biphenyl)methane

(Compound No. 158)

Difluoro-(4-(trans-4-pentylcyclohexyl)phenyloxy) (4'-(3-fluoro-4-cyano)biphenyl)methane

(Compound No. 159)

Difluoro-(4-(trans-4-ethylcyclohexyl)phenyloxy) (4'-(3,5-difluoro-4-trifluoromethyl)biphenyl)methane

(Compound No. 160)

Difluoro-(4-(trans-4-propylcyclohexyl)phenyloxy) (4'-(3,5-difluoro-4-trifluoromethyl)biphenyl)methane

(Compound No. 161)

Difluoro-(4-(trans-4-pentylcyclohexyl)phenyloxy) (4'-(3,5-difluoro-4-trifluoromethyl)biphenyl)methane

(Compound No. 162)

Difluoro-(4-(trans-4-ethylcyclohexyl)phenyloxy) (4'-(3,5-difluoro-4-trifluoromethoxy)biphenyl)methane

(Compound No. 163)

Difluoro-(4-(trans-4-propylcyclohexyl)phenyloxy) (4'-(3,5-difluoro-4-trifluoromethoxy)biphenyl)methane

(Compound No. 164)

Difluoro-(4-(trans-4-pentylcyclohexyl)phenyloxy) (4'-(3,5-difluoro-4-trifluoromethoxy)biphenyl)methane

(Compound No. 165)

Difluoro-(4-(trans-4-ethylcyclohexyl)phenyloxy) (4'-(3,5-difluoro-4-difluoromethoxy)biphenyl)methane

(Compound No. 166)

Difluoro-(4-(trans-4-propylcyclohexyl)phenyloxy) (4'-(3,5-difluoro-4-difluoromethoxy)biphenyl)methane

(Compound No. 167)

Difluoro-(4-(trans-4-pentylcyclohexyl)phenyloxy) (4'-(3,5-difluoro-4-difluoromethoxy)biphenyl)methane

(Compound No. 168)

Difluoro-(4-(trans-4-ethylcyclohexyl)phenyloxy) (4'-(3,5-difluoro-4-cyano)biphenyl)methane

(Compound No. 169)

Difluoro-(4-(trans-4-propylcyclohexyl)phenyloxy) (4'-(3,5-difluoro-4-cyano)biphenyl)methane

(Compound No. 170)

Difluoro-(4-(trans-4-pentylcyclohexyl)phenyloxy) (4'-(3,5-difluoro-4-cyano)biphenyl)methane

Following compounds can be prepared according to the preparation method described above and the preparation method shown in Example 1 with the exception that a 4-(2-(trans-4-alkylcyclohexyl)ethyl)phenol is used in place of 4-(trans-4-propylcyclohexyl)phenol, and 3,4,5-trifluoro-4'-bromobiphenyl or one of various known 3-fluoro-4'-bromobiphenyl derivatives is used.

(Compound No. 171)

Difluoro-(4-(2-(trans-4-ethylcyclohexyl)ethyl)phenyloxy) (4'-(3,4,5-trifluoro)biphenyl)methane

(Compound No. 172)

Difluoro-(4-(2-(trans-4-propylcyclohexyl)ethyl)phenyloxy) (4'-(3,4,5-trifluoro)biphenyl)methane

(Compound No. 173)

Difluoro-(4-(2-(trans-4-pentylcyclohexyl)ethyl)phenyloxy) (4'-(3,4,5-trifluoro)biphenyl)methane

(Compound No. 174)

Difluoro-(4-(2-(trans-4-ethylcyclohexyl)ethyl)phenyloxy)

(4'-(3,4-difluoro)biphenyl)methane

(Compound No. 175)

Difluoro-(4-(2-(trans-4-propylcyclohexyl)ethyl)phenyloxy)(4'-(3,4-difluoro)biphenyl)methane

(Compound No. 176)

Difluoro-(4-(2-(trans-4-pentylcyclohexyl)ethyl)phenyloxy)(4'-(3,4-difluoro)biphenyl)methane

(Compound No. 177)

Difluoro-(4-(2-(trans-4-ethylcyclohexyl)ethyl)phenyloxy)(4'-(3-fluoro-4-chloro)biphenyl)methane

(Compound No. 178)

Difluoro-(4-(2-(trans-4-propylcyclohexyl)ethyl)phenyloxy)(4'-(3-fluoro-4-chloro)biphenyl)methane

(Compound No. 179)

Difluoro-(4-(2-(trans-4-pentylcyclohexyl)ethyl)phenyloxy)(4'-(3-fluoro-4-chloro)biphenyl)methane

(Compound No. 180)

Difluoro-(4-(2-(trans-4-ethylcyclohexyl)ethyl)phenyloxy)(4'-(3-fluoro-4-trifluoromethyl)biphenyl)methane

(Compound No. 181)

Difluoro-(4-(2-(trans-4-propylcyclohexyl)ethyl)phenyloxy)(4'-(3-fluoro-4-trifluoromethyl)biphenyl)methane

(Compound No. 182)

Difluoro-(4-(2-(trans-4-pentylcyclohexyl)ethyl)phenyloxy)(4'-(3-fluoro-4-trifluoromethyl)biphenyl)methane

(Compound No. 183)

Difluoro-(4-(2-(trans-4-ethylcyclohexyl)ethyl)phenyloxy)

(4'-(3-fluoro-4-trifluoromethoxy)biphenyl)methane

(Compound No. 184)

Difluoro-(4-(2-(trans-4-propylcyclohexyl)ethyl)phenyloxy)(4'-(3-fluoro-4-trifluoromethoxy)biphenyl)methane

(Compound No. 185)

Difluoro-(4-(2-(trans-4-pentylcyclohexyl)ethyl)phenyloxy)(4'-(3-fluoro-4-trifluoromethoxy)biphenyl)methane

(Compound No. 186)

Difluoro-(4-(2-(trans-4-ethylcyclohexyl)ethyl)phenyloxy)(4'-(3-fluoro-4-difluoromethoxy)biphenyl)methane

(Compound No. 187)

Difluoro-(4-(2-(trans-4-propylcyclohexyl)ethyl)phenyloxy)(4'-(3-fluoro-4-difluoromethoxy)biphenyl)methane

(Compound No. 188)

Difluoro-(4-(2-(trans-4-pentylcyclohexyl)ethyl)phenyloxy)(4'-(3-fluoro-4-difluoromethoxy)biphenyl)methane

(Compound No. 189)

Difluoro-(4-(2-(trans-4-ethylcyclohexyl)ethyl)phenyloxy)(4'-(3-fluoro-4-cyano)biphenyl)methane

(Compound No. 190)

Difluoro-(4-(2-(trans-4-propylcyclohexyl)ethyl)phenyloxy)(4'-(3-fluoro-4-cyano)biphenyl)methane

(Compound No. 191)

Difluoro-(4-(2-(trans-4-pentylcyclohexyl)ethyl)phenyloxy)(4'-(3-fluoro-4-cyano)biphenyl)methane

(Compound No. 192)

Difluoro-(4-(2-(trans-4-ethylcyclohexyl)ethyl)phenyloxy)

(4'-(3,5-difluoro-4-trifluoromethyl)biphenyl)methane

(Compound No. 193)

Difluoro-(4-(2-(trans-4-propylcyclohexyl)ethyl)phenyloxy)(4'-(3,5-difluoro-4-trifluoromethyl)biphenyl)methane

(Compound No. 194)

Difluoro-(4-(2-(trans-4-pentylcyclohexyl)ethyl)phenyloxy)(4'-(3,5-difluoro-4-trifluoromethyl)biphenyl)methane

(Compound No. 195)

Difluoro-(4-(2-(trans-4-ethylcyclohexyl)ethyl)phenyloxy)(4'-(3,5-difluoro-4-trifluoromethoxy)biphenyl)methane

(Compound No. 196)

Difluoro-(4-(2-(trans-4-propylcyclohexyl)ethyl)phenyloxy)(4'-(3,5-difluoro-4-trifluoromethoxy)biphenyl)methane

(Compound No. 197)

Difluoro-(4-(2-(trans-4-pentylcyclohexyl)ethyl)phenyloxy)(4'-(3,5-difluoro-4-trifluoromethoxy)biphenyl)methane

(Compound No. 198

Difluoro-(4-(2-(trans-4-ethylcyclohexyl)ethyl)phenyloxy)(4'-(3,5-difluoro-4-difluoromethoxy)biphenyl)methane

(Compound No. 199)

Difluoro-(4-(2-(trans-4-propylcyclohexyl)ethyl)phenyloxy)(4'-(3,5-difluoro-4-difluoromethoxy)biphenyl)methane

(Compound No. 200)

Difluoro-(4-(2-(trans-4-pentylcyclohexyl)ethyl)phenyloxy)(4'-(3,5-difluoro-4-difluoromethoxy)biphenyl)methane

(Compound No. 201)

Difluoro-(4-(2-(trans-4-ethylcyclohexyl)ethyl)phenyloxy)

(4'-(3,5-difluoro-4-cyano)biphenyl)methane

(Compound No. 202)

Difluoro-(4-(2-(trans-4-propylcyclohexyl)ethyl)phenyloxy)

(4'-(3,5-difluoro-4-cyano)biphenyl)methane

(Compound No. 203)

Difluoro-(4-(2-(trans-4-pentylcyclohexyl)ethyl)phenyloxy)

(4'-(3,5-difluoro-4-cyano)biphenyl)methane

Example 6

Preparation of difluoro-(4-(trans-4-(trans-4-propylcyclohexyl)cyclohexyl)phenyloxy) (3,4,5-trifluorophenyl)methane (Compound expressed by the general formula (1) wherein ring A and ring B represent 1,4-cyclohexylene group, ring C represents 1,4-phenylene group, $R^1$ representsts n-$C_3H_7$, $Z^1$ and $Z^2$ represent a covalent bond, $\ell$ is 1, m is 0, and X and Y are F; Compound No. 204)

Difluoro-(4-(trans-4-(trans-4-propylcyclohexyl)cyclohexyl) phenyloxy) (3,4,5-trifluorophenyl)methane was synthesized by conducting the same procedures described in Example 1 with the exception that 4-(trans-4-(trans-4-propylcyclohexyl)cyclohexyl) phenol was used in place of 4-(trans-4-propylcyclohexyl)phenol in the second step of Example 1.

Following compounds can be prepared according to the preparation method described above and the preparation method shown in Example 1 with the exception that, instead of 4-(trans-4-(trans-4-propylcyclohexyl) cyclohexyl)phenol, another 4-(trans-4-(trans-4-alkylcyclohexyl)cyclohexyl)phenol having a different alkyl chain is used, and 3,4,5-trifluorobromobenzene or one of various known 3-fluorobromobenzene derivatives is used.

(Compound No. 205)

Difluoro-(4-(trans-4-(trans-4-methylcyclohexyl)cyclohexyl) phenyloxy) (3,4,5-trifluorophenyl)methane

(Compound No. 206)

Difluoro-(4-(trans-4-(trans-4-ethylcyclohexyl)cyclohexyl) phenyloxy) (3,4,5-trifluorophenyl)methane

(Compound No. 207)

Difluoro-(4-(trans-4-(trans-4-butylcyclohexyl)cyclohexyl) phenyloxy) (3,4,5-trifluorophenyl)methane

(Compound No. 208)

Difluoro-(4-(trans-4-(trans-4-pentylcyclohexyl)cyclohexyl) phenyloxy) (3,4,5-trifluorophenyl)methane

(Compound No. 209)

Difluoro-(4-(trans-4-(trans-4-hexylcyclohexyl)cyclohexyl) phenyloxy) (3,4,5-trifluorophenyl)methane

(Compound No. 210)

Difluoro-(4-(trans-4-(trans-4-heptylcyclohexyl)cyclohexyl) phenyloxy) (3,4,5-trifluorophenyl)methane

(Compound No. 211)

Difluoro-(4-(trans-4-(trans-4-octylcyclohexyl)cyclohexyl) phenyloxy) (3,4,5-trifluorophenyl)methane

(Compound No. 212)

Difluoro-(4-(trans-4-(trans-4-nonylcyclohexyl)cyclohexyl) phenyloxy) (3,4,5-trifluorophenyl)methane

(Compound No. 213)

Difluoro-(4-(trans-4-(trans-4-decylcyclohexyl)cyclohexyl)phenyloxy) (3,4,5-trifluorophenyl)methane

(Compound No. 214)

Difluoro-(4-(trans-4-(trans-4-ethylcyclohexyl)cyclohexyl)phenyloxy) (3,4-difluorophenyl)methane

(Compound No. 215)

Difluoro-(4-(trans-4-(trans-4-propylcyclohexyl)cyclohexyl)phenyloxy) (3,4-difluorophenyl)methane

(Compound No. 216)

Difluoro-(4-(trans-4-(trans-4-pentylcyclohexyl)cyclohexyl)phenyloxy) (3,4-difluorophenyl)methane

(Compound No. 217)

Difluoro-(4-(trans-4-(trans-4-ethylcyclohexyl)cyclohexyl)phenyloxy) (3-fluoro-4-chlorophenyl)methane

(Compound No. 218)

Difluoro-(4-(trans-4-(trans-4-propylcyclohexyl)cyclohexyl)phenyloxy) (3-fluoro-4-chlorophenyl)methane

(Compound No. 219)

Difluoro-(4-(trans-4-(trans-4-pentylcyclohexyl)cyclohexyl)phenyloxy) (3-fluoro-4-chlorophenyl)methane

(Compound No. 220)

Difluoro-(4-(trans-4-(trans-4-ethylcyclohexyl)cyclohexyl)phenyloxy) (3-fluoro-4-trifluoromethylphenyl)methane

(Compound No. 221)

Difluoro-(4-(trans-4-(trans-4-propylcyclohexyl)cyclohexyl)phenyloxy) (3-fluoro-4-trifluoromethylphenyl)methane

(Compound No. 222)

Difluoro-(4-(trans-4-(trans-4-pentylcyclohexyl)cyclohexyl) phenyloxy) (3-fluoro-4-trifluoromethylphenyl)methane (Compound No. 223)

Difluoro-(4-(trans-4-(trans-4-ethylcylohexyl)cyclohexyl) phenyloxy) (3-fluoro-4-trifluoromethoxyphenyl)methane (Compound No. 224)

Difluoro-(4-(trans-4-(trans-4-propylcylohexyl)cyclohexyl) phenyloxy) (3-fluoro-4-trifluoromethoxyphenyl)methane (Compound No. 225)

Difluoro-(4-(trans-4-(trans-4-pentylcylohexyl)cyclohexyl) phenyloxy) (3-fluoro-4-trifluoromethoxyphenyl)methane (Compound No. 226)

Difluoro-(4-(trans-4-(trans-4-ethylcycloheyxl)cyclohexyl) phenyloxy) (3-fluoro-4-difluoromethoxyphenyl)methane (Compound No. 227)

Difluoro-(4-(trans-4-(trans-4-propylcycloheyxl)cyclohexyl) phenyloxy) (3-fluoro-4-difluoromethoxyphenyl)methane (Compound No. 228)

Difluoro-(4-(trans-4-(trans-4-pentpylcycloheyxl)cyclohexyl) phenyloxy) (3-fluoro-4-difluoromethoxyphenyl)methane (Compound No. 229)

Difluoro-(4-(trans-4-(trans-4-ethylcycloheyxl)cyclohexyl) phenyloxy) (3-fluoro-4-cyanophenyl)methane (Compound No. 230)

Difluoro-(4-(trans-4-(trans-4-propylcycloheyxl)cyclohexyl) phenyloxy) (3-fluoro-4-cyanophenyl)methane (Compound No. 231)

Difluoro-(4-(trans-4-(trans-4-pentylcycloheyxl)cyclohexyl) phenyloxy) (3-fluoro-4-cyanophenyl)methane

(Compound No. 232)

Difluoro-(4-(trans-4-(trans-4-ethylcycloheyxl)cyclohexyl) phenyloxy) (3,5-difluoro-4-trifluoromethylphenyl)methane

(Compound No. 233)

Difluoro-(4-(trans-4-(trans-4-propylcycloheyxl)cyclohexyl) phenyloxy) (3,5-difluoro-4-trifluoromethylphenyl)methane

(Compound No. 234)

Difluoro-(4-(trans-4-(trans-4-pentylcycloheyxl)cyclohexyl) phenyloxy) (3,5-difluoro-4-trifluoromethylphenyl)methane

(Compound No. 235)

Difluoro-(4-(trans-4-(trans-4-ethylcycloheyxl)cyclohexyl) phenyloxy) (3,5-difluoro-4-trifluoromethoxyphenyl)methane

(Compound No. 236)

Difluoro-(4-(trans-4-(trans-4-propylcycloheyxl)cyclohexyl) phenyloxy) (3,5-difluoro-4-trifluoromethoxyphenyl)methane

(Compound No. 237)

Difluoro-(4-(trans-4-(trans-4-pentylcycloheyxl)cyclohexyl) phenyloxy) (3,5-difluoro-4-trifluoromethoxyphenyl)methane

(Compound No. 238)

Difluoro-(4-(trans-4-(trans-4-ethylcycloheyxl)cyclohexyl) phenyloxy) (3,5-difluoro-4-difluoromethoxyphenyl)methane

(Compound No. 239)

Difluoro-(4-(trans-4-(trans-4-propylcycloheyxl)cyclohexyl) phenyloxy) (3,5-difluoro-4-difluoromethoxyphenyl)methane

(Compound No. 240)

Difluoro-(4-(trans-4-(trans-4-pentylcycloheyxl)cyclohexyl) phenyloxy) (3,5-difluoro-4-difluoromethoxyphenyl)methane

(Compound No. 241)

Difluoro-(4-(trans-4-(trans-4-ethylcycloheyxl)cyclohexyl) phenyloxy) (3,5-difluoro-4-cyanophenyl)methane

(Compound No. 242)

Difluoro-(4-(trans-4-(trans-4-propylcycloheyxl)cyclohexyl) phenyloxy) (3,5-difluoro-4-cyanophenyl)methane

(Compound No. 243)

Difluoro-(4-(trans-4-(trans-4-pentylcycloheyxl)cyclohexyl) phenyloxy) (3,5-difluoro-4-cyanophenyl)methane

Following compounds can be prepared according to the preparation method described above and the preparation method shown in Example 1 with the exception that a 4-(trans-4-(2-(trans-4-alkylcyclohexyl)ethyl)cyclohexyl)phenol or a 4-(2-(trans-4-(trans-4-alkylcyclohexyl)cyclohexyl)ethyl)phenol is used in place of 4-(trans-4-(trans-4-propylcyclohexyl)cyclohexyl)phenol is used, and 3,4,5-trifluorobromobenzene or one of various known 3-fluorobromobenzene derivatives is used.

(Compound No.244)

Difluoro-(4-(trans-4-(2-(trans-4-ethylcyclohexyl)ethyl) cyclohexyl)phenyloxy) (3,4,5-trifluorophenyl)methane

(Compound No. 245)

Difluoro-(4-(trans-4-(2-(trans-4-propylcyclohexyl)ethyl) cyclohexyl)phenyloxy) (3,4,5-trifluorophenyl)methane

(Compound No. 246)

Difluoro-(4-(trans-4-(2-(trans-4-pentylcyclohexyl)ethyl) cyclohexyl)phenyloxy) (3,4,5-trifluorophenyl)methane (Compound No. 247)

Difluoro-(4-(trans-4-(2-(trans-4-ethylcyclohexyl)ethyl) cycloheyxl)phenyloxy) (3,4-difluorophenyl)methane (Compound No. 248)

Difluoro-(4-(trans-4-(2-(trans-4-propylcyclohexyl)ethyl) cycloheyxl)phenyloxy) (3,4-difluorophenyl)methane (Compound No. 249)

Difluoro-(4-(trans-4-(2-(trans-4-pentylcyclohexyl)ethyl) cycloheyxl)phenyloxy) (3,4-difluorophenyl)methane (Compound No. 250)

Difluoro-(4-(trans-4-(2-(trans-4-ethylcyclohexyl)ethyl) cyclohexyl)phenyloxy) (3-fluoro-4-chlorophenyl)methane (Compound No. 251)

Difluoro-(4-(trans-4-(2-(trans-4-propylcyclohexyl)ethyl) cyclohexyl)phenyloxy) (3-fluoro-4-chlorophenyl)methane (Compound No. 252)

Difluoro-(4-(trans-4-(2-(trans-4-pentylcyclohexyl)ethyl) cyclohexyl)phenyloxy) (3-fluoro-4-chlorophenyl)methane (Compound No. 253)

Difluoro-(4-(trans-4-(2-(trans-4-ethylcyclohexyl)ethyl) cyclohexyl)phenyloxy) (3-fluoro-4-trifluoromethylphenyl)methane (Compound No. 254)

Difluoro-(4-(trans-4-(2-(trans-4-propylcyclohexyl)ethyl) cyclohexyl)phenyloxy) (3-fluoro-4-trifluoromethylphenyl)methane (Compound No. 255)

Difluoro-(4-(trans-4-(2-(trans-4-pentylcyclohexyl)ethyl) cyclohexyl)phenyloxy) (3-fluoro-4-trifluoromethylphenyl)methane (Compound No. 256)

Difluoro-(4-(trans-4-(2-(trans-4-ethylcyclohexyl)ethyl) cyclohexyl)phenyloxy) (3-fluoro-4-trifluoromethoxyphenyl)methane (Compound No. 257)

Difluoro-(4-(trans-4-(2-(trans-4-propylcyclohexyl)ethyl) cyclohexyl)phenyloxy) (3-fluoro-4-trifluoromethoxyphenyl)methane (Compound No. 258)

Difluoro-(4-(trans-4-(2-(trans-4-pentylcyclohexyl)ethyl) cyclohexyl)phenyloxy) (3-fluoro-4-trifluoromethoxyphenyl)methane (Compound No. 259)

Difluoro-(4-(trans-4-(2-(trans-4-ethylcycloheyxl)ethyl) cyclohexyl)phenyloxy) (3-fluoro-4-difluoromethoxyphenyl)methane (Compound No. 260)

Difluoro-(4-(trans-4-(2-(trans-4-propylcycloheyxl)ethyl) cyclohexyl)phenyloxy) (3-fluoro-4-difluoromethoxyphenyl)methane (Compound No. 261)

Difluoro-(4-(trans-4-(2-(trans-4-pentylcycloheyxl)ethyl) cyclohexyl)phenyloxy) (3-fluoro-4-difluoromethoxyphenyl)methane (Compound No. 262)

Difluoro-(4-(trans-4-(2-(trans-4-ethylcyclohexyl)ethyl) cyclohexyl)phenyloxy) (3-fluoro-4-cyanophenyl)methane (Compound No. 263)

Difluoro-(4-(trans-4-(2-(trans-4-propylcyclohexyl)ethyl) cyclohexyl)phenyloxy) (3-fluoro-4-cyanophenyl)methane (Compound No. 264)

Difluoro-(4-(trans-4-(2-(trans-4-pentylcyclohexyl)ethyl) cyclohexyl)phenyloxy) (3-fluoro-4-cyanophenyl)methane

(Compound No. 265)

Difluoro-(4-(trans-4-(2-(trans-4-ethylcylohexyl)ethyl) cyclohexyl)phenyloxy) (3,5-difluoro-4-trifluoromethylphenyl)methane

(Compound No. 266)

Difluoro-(4-(trans-4-(2-(trans-4-propylcylohexyl)ethyl) cyclohexyl)phenyloxy) (3,5-difluoro-4-trifluoromethylphenyl)methane

(Compound No. 267)

Difluoro-(4-(trans-4-(2-(trans-4-pentylcylohexyl)ethyl) cyclohexyl)phenyloxy) (3,5-difluoro-4-trifluoromethylphenyl)methane

(Compound No. 268)

Difluoro-(4-(trans-4-(2-(trans-4-ethylcyclohexyl)ethyl) cyclohexyl)phenyloxy) (3,5-difluoro-4-trifluoromethoxyphenyl)methane

(Compound No. 269)

Difluoro-(4-(trans-4-(2-(trans-4-propylcyclohexyl)ethyl) cyclohexyl)phenyloxy) (3,5-difluoro-4-trifluoromethoxyphenyl)methane

(Compound No. 270)

Difluoro-(4-(trans-4-(2-(trans-4-pentylcyclohexyl)ethyl) cyclohexyl)phenyloxy) (3,5-difluoro-4-trifluoromethoxyphenyl)methane

(Compound No. 271)

Difluoro-(4-(trans-4-(2-(trans-4-ethylcyclohexyl)ethyl) cyclohexyl)phenyloxy) (3,5-difluoro-4-difluoromethoxyphenyl)methane

(Compound No. 272)

Difluoro-(4-(trans-4-(2-(trans-4-propylcyclohexyl)ethyl) cyclohexyl)phenyloxy) (3,5-difluoro-4-difluoromethoxyphenyl)methane

(Compound No. 273)

Difluoro-(4-(trans-4-(2-(trans-4-pentylcyclohexyl)ethyl) cyclohexyl)phenyloxy) (3,5-difluoro-4-difluoromethoxyphenyl)methane

(Compound No. 274)

Difluoro-(4-(trans-4-(2-(trans-4-ethylcyclohexyl)ethyl) cyclohexyl)phenyloxy) (3,5-difluoro-4-cyanophenyl)methane

(Compound No. 275)

Difluoro-(4-(trans-4-(2-(trans-4-propylcyclohexyl)ethyl) cyclohexyl)phenyloxy) (3,5-difluoro-4-cyanophenyl)methane

(Compound No. 276)

Difluoro-(4-(trans-4-(2-(trans-4-pentylcyclohexyl)ethyl) cyclohexyl)phenyloxy) (3,5-difluoro-4-cyanophenyl)methane

(Compound No. 277)

Difluoro-(4-(2-(trans-4-(trans-4-ethylcyclohexyl)cyclohexyl) ethyl)phenyloxy) (3,4,5-trifluorophenyl)methane

(Compound No. 278)

Difluoro-(4-(2-(trans-4-(trans-4-propylcyclohexyl) cyclohexyl)ethyl)phenyloxy) (3,4,5-trifluorophenyl)methane

(Compound No. 279)

Difluoro-(4-(2-(trans-4-(trans-4-pentylcyclohexyl) cyclohexyl)ethyl)phenyloxy) (3,4,5-trifluorophenyl)methane

(Compound No. 280)

Difluoro-(4-(2-(trans-4-(trans-4-ethylcyclohexyl)cyclohexyl) ethyl)phenyloxy) (3,4-difluorophenyl)methane

(Compound No. 281)

Difluoro-(4-(2-(trans-4-(trans-4-propylcyclohexyl) cyclohexyl)ethyl)phenyloxy) (3,4-difluorophenyl)methane

(Compound No. 282)

Difluoro-(4-(2-(trans-4-(trans-4-pentylcyclohexyl) cyclohexyl)ethyl)phenyloxy) (3,4-difluorophenyl)methane

(Compound No. 283)

Difluoro-(4-(2-(trans-4-(trans-4-ethylcyclohexyl)cyclohexyl) ethyl)phenyloxy) (3-fluoro-4-chlorophenyl)methane

(Compound No. 284)

Difluoro-(4-(2-(trans-4-(trans-4-propylcyclohexyl) cyclohexyl)ethyl)phenyloxy) (3-fluoro-4-chlorophenyl)methane

(Compound No. 285)

Difluoro-(4-(2-(trans-4-(trans-4-pentylcyclohexyl) cyclohexyl)ethyl)phenyloxy) (3-fluoro-4-chlorophenyl)methane

(Compound No. 286)

Difluoro-(4-(2-(trans-4-(trans-4-ethylcyclohexyl)cyclohexyl) ethyl)phenyloxy) (3-fluoro-4-trifluoromethylphenyl)methane

(Compound No. 287)

Difluoro-(4-(2-(trans-4-(trans-4-propylcyclohexyl) cyclohexyl)ethyl)phenyloxy) (3-fluoro-4-trifluoromethylphenyl)methane

(Compound No. 288)

Difluoro-(4-(2-(trans-4-(trans-4-pentylcyclohexyl) cyclohexyl)ethyl)phenyloxy) (3-fluoro-4-trifluoromethylphenyl)methane

(Compound No. 289)

Difluoro-(4-(2-(trans-4-(trans-4-ethylcyclohexyl)cyclohexyl) ethyl)phenyloxy) (3-fluoro-4-trifluoromethoxyphenyl)methane

(Compound No. 290)

Difluoro-(4-(2-(trans-4-(trans-4-propylcyclohexyl) cyclohexyl)ethyl)phenyloxy) (3-fluoro-4-trifluoromethoxyphenyl)methane

(Compound No. 291)

Difluoro-(4-(2-(trans-4-(trans-4-pentylcyclohexyl) cyclohexyl)ethyl)phenyloxy) (3-fluoro-4-trifluoromethoxyphenyl)methane

(Compound No. 292)

Difluoro-(4-(2-(trans-4-(trans-4-ethylcyclohexyl)cyclohexyl) ethyl)phenyloxy) (3-fluoro-4-difluoromethoxyphenyl)methane

(Compound No. 293)

Difluoro-(4-(2-(trans-4-(trans-4-propylcyclohexyl) cyclohexyl)ethyl)phenyloxy) (3-fluoro-4-difluoromethoxyphenyl)methane

(Compound No. 294)

Difluoro-(4-(2-(trans-4-(trans-4-pentylcyclohexyl) cyclohexyl)ethyl)phenyloxy) (3-fluoro-4-difluoromethoxyphenyl)methane

(Compound No. 295)

Difluoro-(4-(2-(trans-4-(trans-4-ethylcyclohexyl)cyclohexyl) ethyl)phenyloxy) (3-fluoro-4-cyanophenyl)methane (Compound No. 296)

Difluoro-(4-(2-(trans-4-(trans-4-propylcyclohexyl) cyclohexyl)ethyl)phenyloxy) (3-fluoro-4-cyanophenyl)methane (Compound No. 297)

Difluoro-(4-(2-(trans-4-(trans-4-pentylcyclohexyl) cyclohexyl)ethyl)phenyloxy) (3-fluoro-4-cyanophenyl)methane (Compound No. 298)

Difluoro-(4-(2-(trans-4-(trans-4-ethylcyclohexyl)cyclohexyl) ethyl)phenyloxy) (3,5-difluoro-4-chlorophenyl)methane (Compound No. 299)

Difluoro-4-(2-(trans-4-(trans-4-propylcyclohexyl)cyclohexyl) ethyl)phenyloxy) (3,5-difluoro-4-chlorophenyl)methane (Compound No. 300)

Difluoro-(4-(2-(trans-4-(trans-4-pentylcyclohexyl) cyclohexyl)ethyl)phenyloxy) (3,5-difluoro-4-chlorophenyl)methane (Compound No. 301)

Difluoro-(4-(2-(trans-4-(trans-4-ethylcyclohexyl)cyclohexyl) ethyl)phenyloxy) (3,5-difluoro-4-trifluoromethylphenyl)methane (Compound No. 302)

Difluoro-(4-(2-(trans-4-(trans-4-propylcyclohexyl) cyclohexyl)ethyl)phenyloxy) (3,5-difluoro-4-trifluoromethylphenyl)methane (Compound No. 303)

Difluoro-(4-(2-(trans-4-(trans-4-pentylcyclohexyl) cyclohexyl)ethyl)phenyloxy) (3,5-difluoro-4-

trifluoromethylphenyl)methane

(Compound No. 304)

Difluoro-(4-(2-(trans-4-(trans-4-ethylcyclohexyl)cyclohexyl) ethyl)phenyloxy) (3,5-difluoro-4-trifluoromethoxyphenyl)methane

(Compound No. 305)

Difluoro-(4-(2-(trans-4-(trans-4-propylcyclohexyl) cyclohexyl)ethyl)phenyloxy) (3,5-difluoro-4-trifluoromethoxyphenyl)methane

(Compound No. 306)

Difluoro-(4-(2-(trans-4-(trans-4-pentylcyclohexyl) cyclohexyl)ethyl)phenyloxy) (3,5-difluoro-4-trifluoromethoxyphenyl)methane

(Compound No. 307)

Difluoro-(4-(2-(trans-4-(trans-4-ethylcyclohexyl)cyclohexyl) ethyl)phenyloxy) (3,5-difluoro-4-difluoromethoxyphenyl)methane

(Compound No. 308)

Difluoro-(4-(2-(trans-4-(trans-4-propylcyclohexyl) cyclohexyl)ethyl)phenyloxy) (3,5-difluoro-4-difluoromethoxyphenyl)methane

(Compound No. 309)

Difluoro-(4-(2-(trans-4-(trans-4-pentylcyclohexyl) cyclohexyl)ethyl)phenyloxy) (3,5-difluoro-4-difluoromethoxyphenyl)methane

(Compound No. 310)

Difluoro-(4-(2-(trans-4-(trans-4-ethylcyclohexyl)cyclohexyl) ethyl)phenyloxy) (3,5-difluoro-4-cyanophenyl)methane

(Compound No. 311)

Difluoro-(4-(2-(trans-4-(trans-4-propylcyclohexyl)cyclohexyl)ethyl)phenyloxy) (3,5-difluoro-4-cyanophenyl)methane (Compound No. 312)

Difluoro-(4-(2-(trans-4-(trans-4-pentylcyclohexyl)cyclohexyl)ethyl)phenyloxy) (3,5-difluoro-4-cyanophenyl)methane

Example 7 (Use Example 1)

Nematic liquid crystal of the liquid crystal composition comprising the following compounds in the amount mentioned below had a clearing point (Cp) of 72.4°C:

4-(trans-4-propylcyclohexyl)benzonitrile 24 % (by weight, hereinafter the same unit is used)

4-(trans-4-pentylcyclohexyl)benzonitrile 36 %

4-(trans-4-heptylcyclohexyl)benzonitrile 25 %

4-(4-propylphenyl)benzonitrile 15 %

When this liquid crystal composition was filled in a TN cell (twisted nematic cell) having a cell thickness of 9 μm, the composition exhibited a driving threshold voltage (Vth) of 1.78 V, value of dielectric anisotropy ($\Delta\varepsilon$) of +11.0, value of optical anisotropy ($\Delta n$) of 0.137, and viscosity at 20°C ($\eta_{20}$) of 27.0 mPa • s. Using this liquid crystal composition as a mother liquid crystal (hereinafter abbreviated to Mother liquid crystal A), 15 parts of the difluoro-(4-(trans-4-propylcyclohexyl)phenyloxy) (3,4,5-trifluorophenyl)methane shown in Example 1 (Compound No. 1) was mixed with 85 parts of Mother liquid crystal A, and physical properties of the mixture were determined to find that Cp was 66.4°C, Vth: 1.56 V, $\Delta\varepsilon$: 10.3, $\Delta n$: 0.129, and $\eta_{20}$: 24.9 mPa • s. While this composition was left in a freezer at -20°C for 40 days, separation of crystals or development of smectic phase was not noticed.

Example 8 (Use Example 2)

As a fluorine type nematic liquid crystal, the liquid crystal composition shown in Table 2 was prepared.

Table 2 Mother liquid crystal B

| | |
|---|---|
| 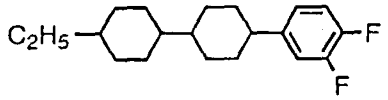 | 16.7 parts by weight (the same, in the rest) |
| 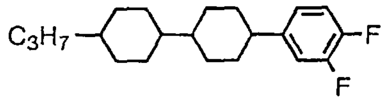 | 16.7 |
| 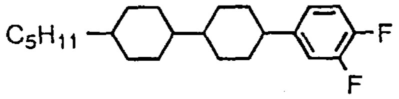 | 16.7 |
| 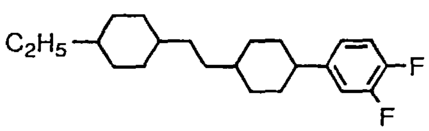 | 10.0 |
| 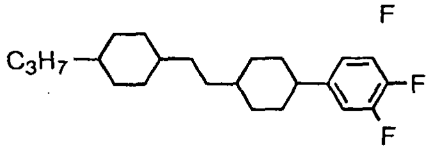 | 5.0 |
| 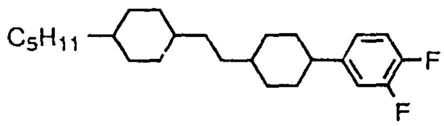 | 10.0 |
| 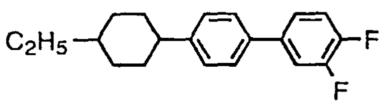 | 6.2 |
| 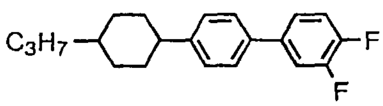 | 6.2 |
| 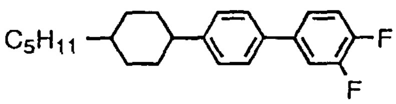 | 12.5 |

This nematic liquid crystal composition had a clearing point (Cp) of 100.1°C. When this liquid crystal composition was filled in in a TN cell (twisted nematic cell) having a cell thickness of 9 μm, the composition exhibited a driving threshold voltage (Vth) of 2.20 V, value of dielectric anisotropy ($\Delta\varepsilon$) of +5.1, and viscosity at 20°C ($\eta_{20}$) of 25.6 mPa • s. Using this liquid crystal composition as another mother liquid crystal (hereinafter abbreviated to Mother liquid crystal B), 20 parts of the difluoro-(4-(trans-4-propylcyclohexyl)phenyloxy) (3,4,5-trifluorophenyl)methane shown in Example 1 (Compound No. 1) was mixed with 80 parts of Mother liquid crystal B, and physical properties of the mixture were determined to find that Cp was 86.4°C, Vth: 2.02 V, $\Delta\varepsilon$: 5.2, and $\eta_{20}$: 22.1 mPa • s. While this composition was left in a freezer at -20°C for 25 days, separation of crystals or development of smectic phase was not noticed.

Examples of liquid crystal compositions are shown below. In the Examples, compounds are indicated by abbreviation according to the definition shown in Table 1 above; and $T_{NI}$ indicates clearing point, $\eta$ does viscosity, and P indicates pitch.

Example 9 (Use Example 3)

| | |
|---|---|
| 3-HBOCF2B(F,F)-F | 5.0 % |
| 1V2-BEB(F,F)-C | 5.0 % |
| 3-HB-C | 25.0 % |
| 1-BTB-3 | 5.0 % |
| 2-BTB-1 | 10.0 % |
| 3-HH-4 | 11.0 % |
| 3-HHB-1 | 6.0 % |
| 3-HHB-3 | 9.0 % |
| 3-H2BTB-2 | 4.0 % |
| 3-H2BTB-3 | 4.0 % |
| 3-H2BTB-4 | 4.0 % |
| 3-HB(F)TB-2 | 6.0 % |
| 3-HB(F)TB-3 | 6.0 % |

$T_{NI}$ = 85.4 (°C)

$\eta$ = 14.4 (mPa·s)

$\Delta n$ = 0.159

$\Delta \varepsilon$ = 7.1

$V_{th}$ = 2.10 (V)

Pitch of a mixture prepared by mixing 100 parts of the liquid crystal composition described above and 0.8 part of CM 33 is shown below.

P = 11 μm

Example 10 (Use Example 4)

| | |
|---|---|
| 3-HBOCF2B(F)-OCF3 | 5.0 % |
| V2-HB-C | 12.0 % |
| 1V2-HB-C | 12.0 % |
| 3-HB-C | 24.0 % |
| 3-HB(F)-C | 5.0 % |
| 2-BTB-1 | 2.0 % |
| 3-HH-4 | 8.0 % |
| 3-HH-VFF | 6.0 % |
| 2-HHB-C | 3.0 % |
| 3-HHB-C | 6.0 % |
| 3-HB(F)TB-2 | 8.0 % |
| 3-H2BTB-3 | 5.0 % |
| 3-H2BTB-4 | 4.0 % |

$T_{NI}$ = 83.5 (°C)

$\eta$ = 17.4 (mPa·s)

$\Delta n$ = 0.145

$\Delta \varepsilon$ = 8.9

$V_{th}$ = 1.95 (V)

Example 11 (Use Example 5

| | |
|---|---|
| 3-HB(F)OCF2B(F,F)-F | 5.0 % |
| 2O1-BEB(F)-C | 5.0 % |
| 3O1-BEB(F)-C | 15.0 % |
| 4O1-BEB(F)-C | 13.0 % |
| 5O1-BEB(F)-C | 13.0 % |
| 2-HHB(F)-C | 15.0 % |
| 3-HHB(F)-C | 15.0 % |
| 3-HB(F)TB-2 | 4.0 % |
| 3-HB(F)TB-3 | 4.0 % |
| 3-HB(F)TB-4 | 4.0 % |
| 3-HHB-1 | 3.0 % |
| 3-HHB-O1 | 4.0 % |

$T_{NI}$ = 85.3 (°C)

$\eta$ = 87.3 (mPa·s)

$\Delta n$ = 0.147

$\Delta \varepsilon$ = 31.0

$V_{th}$ = 0.87 (V)

Example 12 (Use Example 6)

| | |
|---|---|
| 3-HBOCF2B(F,F)-F | 5.0 % |
| 3-HBOCF2B(F)-OCF3 | 4.0 % |
| 5-PyB-F | 4.0 % |
| 3-PyB(F)-F | 4.0 % |
| 2-BB-C | 5.0 % |
| 4-BB-C | 4.0 % |
| 5-BB-C | 5.0 % |
| 2-PyB-2 | 2.0 % |

| | |
|---|---|
| 3-PyB-2 | 2.0 % |
| 4-PyB-2 | 2.0 % |
| 6-PyB-O5 | 3.0 % |
| 6-PyB-O6 | 3.0 % |
| 6-PyB-O7 | 3.0 % |
| 3-PyBB-F | 6.0 % |
| 4-PyBB-F | 6.0 % |
| 5-PyBB-F | 6.0 % |
| 3-HHB-3 | 8.0 % |
| 2-H2BTB-2 | 4.0 % |
| 2-H2BTB-3 | 4.0 % |
| 2-H2BTB-4 | 5.0 % |
| 3-H2BTB-2 | 5.0 % |
| 3-H2BTB-3 | 5.0 % |
| 3-H2BTB-4 | 5.0 % |

$T_{NI}$ = 87.9 (°C)

$\eta$ = 33.7 (mPa·s)

$\Delta n$ = 0.198

$\Delta \varepsilon$ = 6.6

$V_{th}$ = 2.20 (V)

Example 13 (Use Example 7)

| | |
|---|---|
| 3-HBOCF2B(F,F)-F | 3.0 % |
| 3-HB(F)OCF2B(F,F)-F | 3.0 % |
| 3-GB-C | 10.0 % |
| 4-GB-C | 10.0 % |
| 2-BEB-C | 12.0 % |
| 3-BEB-C | 4.0 % |
| 3-PyB(F)-F | 6.0 % |
| 3-HEB-O4 | 8.0 % |
| 4-HEB-O2 | 6.0 % |
| 3-HEB-O2 | 5.0 % |
| 5-HEB-O2 | 4.0 % |
| 5-HEB-5 | 5.0 % |
| 4-HEB-5 | 5.0 % |
| 1O-BEB-2 | 4.0 % |
| 3-HHB-1 | 6.0 % |
| 3-HHEBB-C | 3.0 % |
| 3-HBEBB-C | 3.0 % |
| 5-HBEBB-C | 3.0 % |

$T_{NI}$ = 67.0 (°C)

$\eta$ = 39.3 (mPa·s)

$\Delta n$ = 0.121

$\Delta \varepsilon$ = 11.6

$V_{th}$ = 1.29 (V)

Example 14 (Use Example 8)

| | |
|---|---|
| 3-HBOCF2B(F)-OCF3 | 3.0 % |
| 3-HB(F)OCF2B(F,F)-F | 3.0 % |
| 3-HB-C | 18.0 % |
| 7-HB-C | 3.0 % |
| 1O1-HB-C | 10.0 % |
| 3-HB(F)-C | 10.0 % |
| 2-PyB-2 | 2.0 % |
| 3-PyB-2 | 2.0 % |
| 4-PyB-2 | 2.0 % |

| | |
|---|---|
| 101-HH-3 | 7.0 % |
| 2-BTB-O1 | 7.0 % |
| 3-HHB-1 | 7.0 % |
| 3-HHB-F | 4.0 % |
| 3-HHB-O1 | 4.0 % |
| 3-HHB-3 | 2.0 % |
| 3-H2BTB-2 | 3.0 % |
| 3-H2BTB-3 | 3.0 % |
| 2-PyBH-3 | 4.0 % |
| 3-PyBH-3 | 3.0 % |
| 3-PyBB | 3.0 % |

$T_{NI}$ = 74.0 (°C)

$\eta$ = 25.7 (mPa·s)

$\Delta n$ = 0.137

$\Delta \varepsilon$ = 8.1

$V_{th}$ = 1.75 (V)

Example 15 (Use Example 9)

| | |
|---|---|
| 2-HBOCF2B(F,F)-F | 3.0 % |
| 3-HBOCF2B(F,F)-F | 3.0 % |
| 5-HBOCF2B(F,F)-F | 3.0 % |
| 201-BEB(F)-C | 5.0 % |
| 301-BEB(F)-C | 8.0 % |
| 501-BEB(F)-C | 4.0 % |
| 1V2-BEB(F,F)-C | 10.0 & |
| 3-HH-EMe | 10.0 % |
| 3-HB-O2 | 18.0 % |
| 7-HEB-F | 2.0 % |

| | |
|---|---|
| 3-HHEB-F | 2.0 % |
| 5-HHEB-F | 2.0 % |
| 3-HBEB-F | 4.0 % |
| 201-HBEB(F)-C | 2.0 % |
| 3-HB(F)EB(F)-C | 2.0 % |
| 3-HBEB(F,F)-C | 2.0 % |
| 3-HHB-F | 4.0 % |
| 3-HHB-O1 | 4.0 % |
| 3-HHB-3 | 8.0 % |
| 3-HEBEB-F | 2.0 % |
| 3-HEBEB-1 | 2.0 % |

$T_{NI}$ = 73.0 (°C)

$\eta$ = 33.7 (mPa·s)

$\Delta n$ = 0.111

$\Delta \varepsilon$ = 22.3

$V_{th}$ = 1.00 (V)

Example 16 (Use Example 10)

| | |
|---|---|
| 2-HBOCF2B(F)-OCF3 | 4.0 % |
| 3-HBOCF2B(F)-OCF3 | 4.0 % |
| 5-HBOCF2B(F)-OCF3 | 4.0 % |
| 2O1-BEB(F)-C | 5.0 % |
| 3O1-BEB(F)-C | 9.0 % |
| 5O1-BEB(F)-C | 4.0 % |
| 1V2-BEB(F,F)-C | 16.0 % |
| 3-HB-O2 | 10.0 % |
| 3-HH-4 | 3.0 % |
| 3-HHB-F | 3.0 % |
| 3-HHB-1 | 8.0 % |
| 3-HHB-O1 | 4.0 % |
| 3-HBEB-F | 4.0 % |
| 3-HHEB-F | 7.0 % |
| 5-HHEB-F | 2.0 % |
| 3-H2BTB-2 | 4.0 % |
| 3-H2BTB-3 | 4.0 % |
| 3-HB(F)TB-2 | 5.0 % |

$T_{NI}$ = 82.6 (°C)

$\eta$ = 39.3 (mPa·s)

$\Delta n$ = 0.136

$\Delta \varepsilon$ = 27.3

$V_{th}$ = 1.02 (V)

Example 17 (Use Example 11)

| | |
|---|---|
| 3-HBOCF2B(F)-OCF3 | 4.0 % |
| 2-BEB-C | 12.0 % |
| 3-BEB-C | 4.0 % |
| 4-BEB-C | 6.0 % |
| 3-HB-C | 28.0 % |
| 3-HEB-O4 | 8.0 % |
| 4-HEB-O2 | 8.0 % |
| 5-HEB-O1 | 8.0 % |
| 3-HEB-O2 | 6.0 % |
| 5-HEB-O2 | 5.0 % |
| 3-HHB-1 | 7.0 % |
| 3-HHB-O1 | 4.0 % |

$T_{NI}$ = 62.6 (°C)

$\eta$ = 25.6 (mPa·s)

$\Delta n$ = 0.113

$\Delta \varepsilon$ = 10.2

$V_{th}$ = 1.31 (V)

Example 18 (Use Example 12)

| | |
|---|---|
| 3-HBOCF2B(F,F)-F | 4.0 % |
| 2-BEB-C | 10.0 % |
| 5-BB-C | 12.0 % |
| 7-BB-C | 7.0 % |
| 1-BTB-3 | 7.0 % |
| 2-BTB-1 | 10.0 % |
| 1O-BEB-2 | 10.0 % |
| 1O-BEB-5 | 12.0 % |
| 2-HHB-1 | 4.0 % |
| 3-HHB-F | 4.0 % |
| 3-HHB-1 | 7.0 % |
| 3-HHB-1O | 4.0 % |
| 3-HHB-3 | 9.0 % |

$T_{NI}$ = 63.2 (°C)

$\eta$ = 19.8 (mPa·s)

$\Delta n$ = 0.159

$\Delta \varepsilon$ = 6.6

$V_{th}$ = 1.77 (V)

Example 19 (Use Example 13)

| | |
|---|---|
| 3-HBOCF2B(F,F)-F | 5.0 % |
| 3-HBOCF2B(F)-OCF3 | 5.0 % |
| 3-HB(F)OCF2B(F,F)-F | 5.0 % |

| | |
|---|---|
| 2-HB-C | 5.0 % |
| 3-HB-C | 12.0 % |
| 3-HB-O2 | 15.0 % |
| 2-BTB-1 | 3.0 % |
| 3-HHB-1 | 8.0 % |
| 3-HHB-F | 4.0 % |
| 3-HHB-O1 | 5.0 % |
| 3-HHB-3 | 8.0 % |
| 3-HHEB-F | 4.0 % |
| 5-HHEB-F | 4.0 % |
| 3-HHB(F)-F | 7.0 % |
| 5-HHB(F)-F | 5.0 % |
| 3-HHB(F,F)-F | 5.0 % |

$T_{NI}$ = 90.3 (°C)

$\eta$ = 18.4 (mPa·s)

$\Delta n$ = 0.097

$\Delta \varepsilon$ = 4.4

$V_{th}$ = 2.56 (V)

Example 20 (Use Example 14)

| | |
|---|---|
| 3-HBOCF2B(F,F)-F | 5.0 % |
| 3-HBOCF2B(F)-OCF3 | 5.0 % |
| 3-HB(F)OCF2B(F,F)-F | 5.0 % |
| 2-HHB(F)-F | 8.0 % |
| 3-HHB(F)-F | 17.0 % |
| 5-HHB(F)-F | 16.0 % |
| 2-H2HB(F)-F | 10.0 % |
| 3-H2HB(F)-F | 5.0 % |
| 5-H2HB(F)-F | 10.0 % |
| 3-HBB(F)-F | 6.0 % |
| 5-HBB(F)-F | 13.0 % |

$T_{NI}$ = 95.4 (°C)

$\eta$ = 24.0 (mPa·s)

$\Delta n$ = 0.092

$\Delta \varepsilon$ = 4.8

$V_{th}$ = 2.26 (V)

Pitch of a mixture prepared by mixing 100 parts of the liquid crystal composition described above and 0.3 part of CN is shown below.

P = 78 µm

Example 21 (Use Example 15)

| | |
|---|---|
| 2-HBOCF2B(F,F)-F | 4.0 % |
| 3-HBOCF2B(F,F)-F | 4.0 % |
| 5-HBOCF2B(F,F)-F | 4.0 % |
| 7-HB(F)-F | 5.0 % |
| 5-H2B(F)-F | 5.0 % |
| 3-HB-O2 | 10.0 % |
| 3-HH-4 | 5.0 % |
| 3-HHB(F)-F | 10.0 % |
| 5-HHB(F)-F | 10.0 % |
| 3-H2HB(F)-F | 5.0 % |
| 2-HBB(F)-F | 3.0 % |
| 3-HBB(F)-F | 3.0 % |
| 5-HBB(F)-F | 4.0 % |
| 2-H2BB(F)-F | 5.0 % |
| 3-H2BB(F)-F | 6.0 % |
| 3-HHB-1 | 8.0 % |
| 3-HHB-O1 | 5.0 % |
| 3-HHB-3 | 4.0 % |

$T_{NI}$ = 83.9 (°C)

$\eta$ = 17.1 (mPa·s)

$\Delta n$ = 0.091

$\Delta \varepsilon$ = 3.2

$V_{th}$ = 2.67 (V)

Example 22 (Use Example 16)

| | |
|---|---|
| 2-HBOCF2B(F)-OCF3 | 5.0 % |
| 3-HBOCF2B(F)-OCF3 | 5.0 % |
| 5-HBOCF2B(F)-OCF3 | 5.0 % |
| 7-HB(F,F)-F | 3.0 % |
| 3-HB-O2 | 7.0 % |
| 2-HHB(F)-F | 10.0 % |
| 3-HHB(F)-F | 10.0 % |
| 5-HHB(F)-F | 10.0 % |
| 3-HBB(F)-F | 9.0 % |
| 5-HBB(F)-F | 10.0 % |
| 2-HBB-F | 4.0 % |
| 3-HBB-F | 4.0 % |
| 5-HBB-F | 3.0 % |
| 3-HBB(F,F)-F | 5.0 % |
| 5-HBB(F,F)-F | 10.0 % |

$T_{NI}$ = 83.1 (°C)

$\eta$ = 21.6 (mPa·s)

$\Delta n$ = 0.109

$\Delta \varepsilon$ = 5.8

$V_{th}$ = 1.99 (V)

Example 23 (Use Example 17)

| | |
|---|---|
| 3-HBOCF2B(F,F)-F | 5.0 % |
| 3-HBOCF2B(F)-OCF3 | 5.0 % |
| 7-HB(F,F)-F | 4.0 % |
| 3-H2HB(F,F)-F | 12.0 % |
| 5-H2HB(F,F)-F | 10.0 % |
| 3-HHB(F,F)-F | 10.0 % |
| 4-HHB(F,F)-F | 5.0 % |
| 3-HH2B(F,F)-F | 15.0 % |
| 5-HH2B(F,F)-F | 10.0 % |
| 3-HBB(F,F)-F | 12.0 % |
| 5-HBB(F,F)-F | 12.0 % |

$T_{NI}$ = 68.8 (°C)

$\eta$ = 26.6 (mPa·s)

$\Delta n$ = 0.087

$\Delta \varepsilon$ = 8.1

$V_{th}$ = 1.64 (V)

Example 24 (Use Example 18)

| | |
|---|---|
| 3-HBOCF2B(F,F)-F | 5.0 % |
| 3-HBOCF2B(F)-OCF3 | 5.0 % |
| 3-HB-CL | 10.0 % |
| 5-HB-CL | 4.0 % |
| 7-HB-CL | 4.0 % |
| 101-HH-5 | 5.0 % |

| | |
|---|---|
| 2-HBB(F)-F | 8.0 % |
| 3-HBB(F)-F | 8.0 % |
| 5-HBB(F)-F | 4.0 % |
| 4-HHB-CL | 8.0 % |
| 5-HHB-CL | 8.0 % |
| 3-H2HB(F)-CL | 4.0 % |
| 3-HBB(F,F)-F | 10.0 % |
| 5-H2BB(F,F)-F | 9.0 % |
| 3-HB(F)VB-2 | 4.0 % |
| 3-HB(F)VB-3 | 4.0 % |

$T_{NI}$ = 87.3 (°C)

$\eta$ = 17.7 (mPa·s)

$\Delta n$ = 0.122

$\Delta \varepsilon$ = 5.1

$V_{th}$ = 2.29 (V)

Example 25 (Use Example 19)

| | |
|---|---|
| 3-HBOCF2B(F,F)-F | 5.0 % |
| 3-HBOCF2B(F)-OCF3 | 5.0 % |
| 3-HB(F)OCF2B(F,F)-F | 5.0 % |
| 3-HHB(F,F)-F | 9.0 % |
| 3-H2HB(F,F)-F | 8.0 % |
| 4-H2HB(F,F)-F | 8.0 % |
| 3-HBB(F,F)-F | 14.0 % |
| 5-HBB(F,F)-F | 20.0 % |
| 3-H2BB(F,F)-F | 10.0 % |
| 5-HHBB(F,F)-F | 3.0 % |
| 5-HHEBB-F | 2.0 % |
| 3-HH2BB(F,F)-F | 3.0 % |
| 101-HBBH-4 | 4.0 % |
| 101-HBBH-5 | 4.0 % |

$T_{NI}$ = 92.7 (°C)

$\eta$ = 31.9 (mPa·s)

$\Delta n$ = 0.114

$\Delta \varepsilon$ = 8.3

$V_{th}$ = 1.85 (V)

Example 26 (Use Example 20)

| | |
|---|---|
| 2-HBOCF2B(F)-OCF3 | 4.0 % |
| 3-HBOCF2B(F)-OCF3 | 4.0 % |
| 5-HBOCF2B(F)-OCF3 | 4.0 % |
| 5-HB-F | 12.0 % |
| 6-HB-F | 9.0 % |
| 7-HB-F | 7.0 % |
| 3-HHB-OCF3 | 7.0 % |
| 4-HHB-OCF3 | 7.0 % |
| 5-HHB-OCF3 | 5.0 % |
| 3-HH2B-OCF3 | 4.0 % |
| 5-HH2B-OCF3 | 4.0 % |
| 3-HHB(F,F)-OCF3 | 5.0 % |
| 3-HBB(F)-F | 5.0 % |
| 5-HBB(F)-F | 10.0 % |
| 3-HH2B(F)-F | 3.0 % |
| 3-HB(F)BH-3 | 3.0 % |
| 5-HBBH-3 | 3.0 % |
| 3-HHB(F,F)-OCF2H | 4.0 % |

$T_{NI}$ = 82.0 (°C)

$\eta$ = 13.5 (mPa·s)

$\Delta n$ = 0.090

$\Delta \varepsilon$ = 4.3

$V_{th}$ = 2.43 (V)

Example 27 (Use Example 21)

| | |
|---|---|
| 2-HBOCF2B(F)-OCF3 | 5.0 % |
| 3-HBOCF2B(F)-OCF3 | 5.0 % |
| 5-HBOCF2B(F)-OCF3 | 5.0 % |
| 2-HHB(F)-F | 2.0 % |
| 3-HHB(F)-F | 2.0 % |
| 5-HHB(F)-F | 2.0 % |
| 2-HBB(F)-F | 6.0 % |
| 3-HBB(F)-F | 6.0 % |
| 5-HBB(F)-F | 5.0 % |
| 2-H2BB(F)-F | 9.0 % |
| 3-H2BB(F)-F | 9.0 % |
| 3-HBB(F,F)-F | 25.0 % |
| 5-HBB(F,F)-F | 9.0 % |
| 1O1-HBBH-4 | 5.0 % |
| 1O1-HBBH-5 | 5.0 % |

$T_{NI}$ = 93.8 (°C)

$\eta$ = 32.0 (mPa·s)

$\Delta n$ = 0.130

$\Delta \varepsilon$ = 6.9

$V_{th}$ = 1.96 (V)

Example 28 (Use Example 22)

| | |
|---|---|
| 2-HBOCF2B(F,F)-F | 5.0 % |
| 3-HBOCF2B(F,F)-F | 10.0 % |
| 5-HBOCF2B(F,F)-F | 10.0 % |
| 2-HBOCF2B(F)-OCF3 | 5.0 % |
| 3-HBOCF2B(F)-OCF3 | 10.0 % |
| 5-HBOCF2B(F)-OCF3 | 10.0 % |
| 3-HHB(F)-F | 17.0 % |
| 5-HHB(F)-F | 16.0 % |
| 3-H2HB(F)-F | 5.0 % |
| 2-HBB(F)-F | 6.0 % |
| 3-HBB(F)-F | 6.0 % |

$T_{NI}$ = 81.9 (°C)

$\eta$ = 24.0 (mPa·s)

$\Delta n$ = 0.090

$\Delta \varepsilon$ = 5.0

$V_{th}$ = 2.20 (V)

Example 29 (Use Example 23)

| | |
|---|---|
| 3-HBOCF2B(F,F)-F | 5.0 % |
| 3-BEB(F)-C | 8.0 % |
| 3-HB-C | 8.0 % |
| V-HB-C | 8.0 % |
| 1V-HB-C | 8.0 % |
| 3-HB-O2 | 3.0 % |
| 3-HH-2V | 14.0 % |
| 3-HH-2V1 | 7.0 % |
| V2-HHB-1 | 10.0 % |
| 3-HHB-1 | 5.0 % |
| 3-HHEB-F | 7.0 % |
| 3-H2BTB-2 | 6.0 % |
| 3-H2BTB-3 | 6.0 % |
| 3-H2BTB-4 | 5.0 % |

$T_{NI}$ = 93.3 (°C)

$\eta$ = 15.5 (mPa·s)

$\Delta n$ = 0.131

$\Delta \varepsilon$ = 8.5

$V_{th}$ = 2.19 (V)

Example 30 (Use Example 24)

| | |
|---|---|
| 3-HBOCF2B(F,F)-F | 10.0 % |
| 3-HBOCF2B(F)-OCF3 | 10.0 % |
| 3-HB(F)OCF2B(F,F)-F | 5.0 % |
| 3-H2HB(F,F)-F | 7.0 % |
| 5-H2HB(F,F)-F | 8.0 % |
| 4-HHB(F,F)-F | 5.0 % |
| 3-HH2B(F,F)-F | 9.0 % |
| 5-HH2B(F,F)-F | 9.0 % |
| 3-HBB(F,F)-F | 10.0 % |
| 5-HBB(F,F)-F | 15.0 % |
| 3-HBEB(F,F)-F | 2.0 % |
| 4-HBEB(F,F)-F | 2.0 % |
| 5-HBEB(F,F)-F | 2.0 % |
| 4-HHEB(F,F)-F | 3.0 % |
| 5-HHEB(F,F)-F | 3.0 % |

$T_{NI}$ = 70.7 (°C)

$\eta$ = 27.6 (mPa·s)

$\Delta n$ = 0.091

$\Delta \varepsilon$ = 9.9

$V_{th}$ = 1.88 (V)

Comparative Example 1

As the compound to be compared with the compounds of the present invention, Compound (b-1) ($R=C_3H_7$) described in USP 5,032,313 mentioned in the section of BACKGROUND ART was actually synthesized according to a production method described in the patent publication.

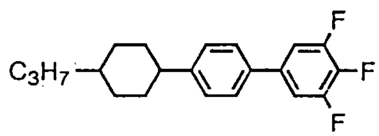

(b-1)

Cr 40.7(N 33.2) Iso

The comparative compound shown above in an amount of 20 parts was mixed with 80 parts of Mother liquid crystal B to prepare a new liquid crystal composition, and physical properties of the composition were determined. Liquid crystal composition prepared with a purpose of evaluating its mutual solubility was left in a freezer at -20°C as it was, and the number of days which passed until crystals were separated or smectic phase was developed for the first time in the liquid crystal compositon was counted. Physical properties of Mother liquid crystal B and the liquid crystal composition prepared anew are shown in Table 3 together with the results in Example 7 (Numerals in the parenthesis are values extrapolated from those of the mother liquid crystals).

Table 3

| | Cp(°C) | Δ ε | $\eta_{20}$(mPa·s) | $V_{th}$ (V) | Mutual solubility*1 (Number of days) |
|---|---|---|---|---|---|
| Mother liquid crystal B | 100.1 | 5.1 | 25.6 | 2.20 | >25 |
| Comparative Example 1 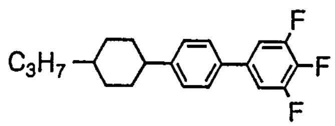 (b-1) | 90.4 | 6.3 (11.1) | 25.4 | 1.95 | 15 |
| Example 7 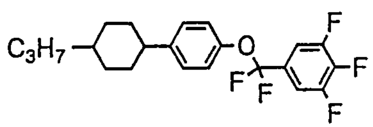 (Compound No. 1) | 86.4 | 5.2 (5.6) | 22.1 | 2.02 | >25 |

*1 Number of days passed until crystals (solid of smectic phase or the like) were separated for the first time after a sample liquid crystal composition was put in a freezer at -20°C.

From Comparative Example 1, it can be seen that Compound (b-1) has a comparatively high dielectric anisotropy, lowers only threshold voltage by as large as 0.25 V without changing viscosity. However, dielectric anisotropy value of the liquid crystal composition prepared by mixing Compound (b-1) is raised by about 24 % compared with the mother

liquid crystal, and thus the compound can be said to be unsuitable for solving the problem of intake of ionic impurities described in the section of BACKGROUND ART. On the other hand, the liquid crystal composition prepared from the compound (Compound No. 1) of the present invention has a viscosity lower than the liquid crystal composition prepared from the comparative compound by as large as about 14 % while having about the same degree of clearing point. Further, to one's surprise, only threshold voltage of the former composition is lowered by as large as about 0.2 V compared with that of the mother liquid crystal despite the fact that dielectric anisotropy value is little changed. With respect to miscibility whereas the separation of crystals was confirmed in 15 days in a freezer at -20°C for the liquid crystal composition prepared from the comparative Compound (b-1), the separation of crystals was not noticed more than 25 days for the liquid crystal composition prepared from the compound of the present invention, confirming the fact that the compounds of the present invention have a remarkably excellent miscibility at low temperatures.

As discussed above, the compounds of the present invention are novel liquid crystalline compounds which

1) exhibit low threshold voltage which can be considered to be caused by a small elastic constant,
2) have low viscosity,
3) keep or lower dielectric anisotropy when added to liquid crystal compositions, and
4) are excellent in miscibility with other known liquid crystalline compounds, particularly in the miscibility at low temperatures, and thus

the present compounds have extremely useful characteristics which can not be found in three-ring system or four-ring system compounds having a similar structure.

INDUSTRIAL APPLICABILITY

Compounds of the present invention expressed by the general formula (1) are very stable against the changes in the outside environment and remarkably low in viscosity, and exhibit a low threshold voltage which can be considered to be caused by their elastic constant. The compounds of the present invention are excellent in miscibility with other liquid crystalline compounds, particularly in the miscibility at low temperatures. As embodiments of the compounds of the present invention, the compounds expressed by the general formula (1) wherein an alkyl group is selected as substituent $R^1$ and an atom or a group other than CN group is selected as X exhibit a high voltage holding ratio, and show preferable characteristics as liquid crystal material for TFT mode display devices, particularly for low voltage driving or high speed response. On the other hand, the present compounds wherein an alkenyl group is selected as substituent $R^1$ or CN group is selected for X have a remarkably low viscosity or high clearing point, and thus are extremely useful as viscosity reducing agent for STN display devices. Besides, they are low in dielectric anisotropy; are extremely small in the intake of ionic impurities, which is a problem particularly in TFT mode, since molecural polarity of the compounds is small; and exhibit a stable, high voltage holding ratio having a small dependency on temperature.

Accordingly, it is possible to provide novel liquid crystal compositions and liquid crystal display devices which are stable against the changes in the outside environment, and can be driven at low voltage and at a high response speed by using the compounds of the present invention.

**Claims**

**1.** An α, α-difluorobenzyl ether derivative expressed by the general formula (1)

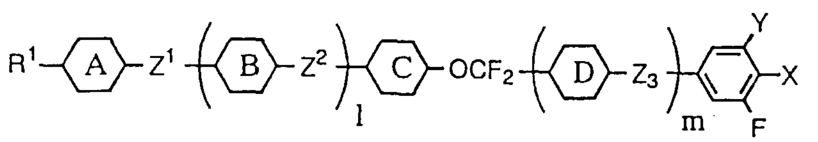

(1)

wherein $R^1$ represents a straight chain or branched alkyl group having 1 to 15 carbon atoms or an alkenyl group having 2 to 15 carbon atoms one or not-adjacent two or more $CH_2$ groups in the alkyl or alkenyl group may be replaced by oxygen atom; ring A and ring B independently represent 1,4-cyclohexylene group one or more $CH_2$ groups in the ring may be replaced by oxygen atom or sulfur atom, or 1,4-phenylene group one or more CH groups in the ring may be replaced by nitrogen atom, and one or more hydrogen atoms on the ring may be replaced by a halogen atom; ring C and ring D independently represent 1,4-phenylene group one or more hydrogen atoms on the ring may be replaced by a halogen atom; $Z^1$, $Z^2$, and $Z^3$ independently represent $-CH_2CH_2-$, -CH=CH-, -C≡C-, or single bond; X represents a halogen atom, $CF_3$, $OCF_3$, $OCHF_2$, or CN group; Y represents hydrogen atom or a halogen atom; ℓ and m are 0 or 1; ℓ + m ≦ 1 ; and an element which constitutes the compound may be replaced by its isotope.

2. The compound according to claim 1 wherein ℓ and m are 0 in the general formula (1).

3. The compound according to claim 2 wherein ring A represents 1,4-cyclohexylene group, and ring C represents 1,4-phenylene group in the general formula (1).

4. The compound according to claim 3 wherein X represents fluorine atom, $CF_3$, or $OCF_3$ group, and Y represents hydrogen atom in the general formula (1).

5. The compound according to claim 3 wherein X represents fluorine atom, $CF_3$, or $OCF_3$ group, and Y represents a halogen atom in the general formula (1).

6. The compound according to claim 1 wherein ℓ is 0, and m is 1 in the general formula (1).

7. The compound according to claim 6 wherein ring A represents 1,4-cyclohexylene group, and ring C and ring D represent 1,4-phenylene group in the general formula (1).

8. The compound according to claim 1 wherein ℓ is 1, and m is 0 in the general formula (1).

9. The compound according to claim 8 wherein ring A and ring B represent 1,4-cyclohexylene group, and ring C represents 1,4-phenylene group in the general formula (1).

10. The compound according to claim 1 wherein $R^1$ represents an alkenyl group in the general formula (1).

11. A liquid crystal composition comprising at least two components and comprising at least one compound expressed by the general formula (1).

12. A liquid crystal composition comprising, as a first component, at least one liquid crystalline compound defined in any one of claims 1 to 10, and comprising, as a second component, at least one compound selected from the group consisting of the compounds expressed by any one of the general formulas (2), (3), and (4)

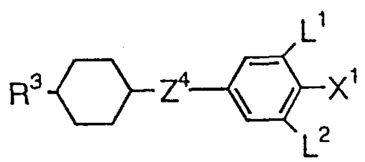

(2)

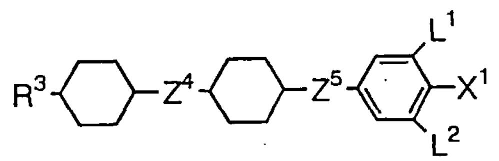

(3)

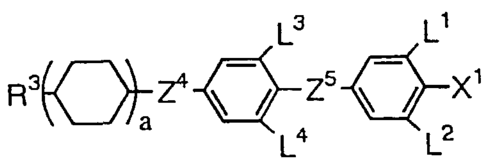

(4)

wherein $R^3$ represents an alkyl group having 1 to 10 carbon atoms, $X^1$ represents F, Cl, $OCF_3$, $OCF_2H$, $CF_3$, $CF_2H$, or $CFH_2$; $L^1$, $L^2$, $L^3$, and $L^4$ independently represent H or F; $Z^4$ and $Z^5$ independently represent $-(CH_2)_2-$, -CH=CH-, or single bond; and a is 1 or 2.

**13.** A liquid crystal composition comprising, as a first component, at least one liquid crystalline compound defined in any one of claims 1 to 10, and comprising, as a second component, at least one compound selected from the group consisting of the compounds expressed by any one of the general formulas (5), (6), (7), (8), and (9)

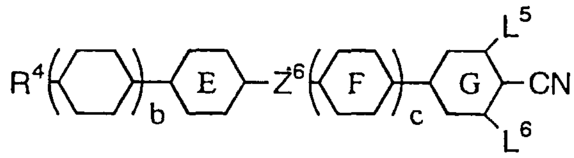

(5)

wherein $R^4$ represents F, an alkyl group having 1 to 10 carbon atoms, or an alkenyl group having 2 to 10 carbon atoms any methylene group ($-CH_2-$) in the alkyl or alkenyl group may be replaced by oxygen atom (-O-), but in no case two or more methylene groups are continuously replaced by oxygen atom; ring E represents trans-1,4-cyclohexylene group, 1,4-phenylene group, pyrimidine-2,5-diyl group, or 1,3-dioxane-2,5-diyl group; ring F represents trans-1,4-cyclohexylene group, 1,4-phenylene group, or pyrimidine-2,5-diyl group; ring G represents trans-1,4-cyclohexylene group or 1,4-phenylene group; $Z^6$ represents $-(CH_2)_2-$, -COO-, or single bond; $L^5$ and $L^6$ independently represent H or F; b and c are independently 0 or 1,

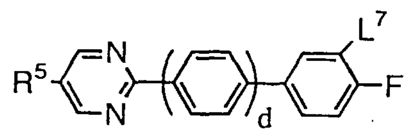

(6)

wherein $R^5$ represents an alkyl group having 1 to 10 carbon atoms; $L^7$ represents H or F; and d is 0 or 1,

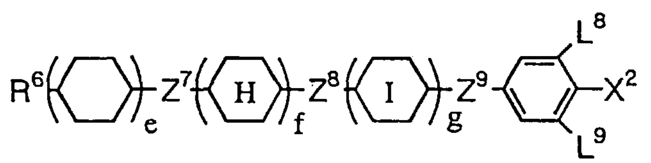

(7)

wherein $R^6$ represents an alkyl group having 1 to 10 carbon atoms; ring H and ring I independently represent trans-1,4-cyclohexylene group or 1,4-phenylene group; $Z^7$ and $Z^8$ independently represent -COO- or single bond; $Z^9$ represents -COO- or -C≡C-; $L^8$ and $L^9$ independently represent H or F; $X^2$ represents F, $OCF_3$, $OCF_2H$, $CF_3$, $CF_2H$, or $CFH_2$ provided that when $X^2$ represents $OCF_3$, $OCF_2H$, $CF_3$, $CF_2H$, or $CFH_2$, both $L^8$ and $L^9$ represent H; and e, f, and g are independently 0 or 1,

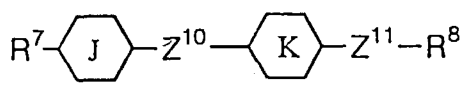

(8)

wherein $R^7$ and $R^8$ independently represent an alkyl group having 1 to 10 carbon atoms or an alkenyl group having 2 to 10 carbon atoms any methylene group ($-CH_2-$) in the groups may be replaced by oxygen atom (-O-), but in no case two or more methylene groups are continuously replaced by oxygen atom; ring J represents trans-1,4-cyclohexylene group, 1,4-phenylene group, or pyrimidine-2,5-diyl group; ring K represents trans-1,4-cyclohexylene group or 1,4-phenylene group; $Z^{10}$ represents -C≡C-, -COO-, - $(CH_2)_2$-, -CH=CH-C≡C-, or single bond; and $Z^{11}$ represents -COO- or single bond,

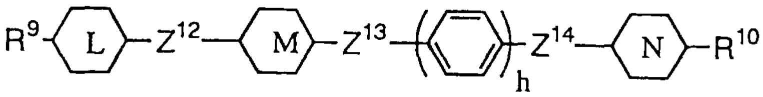 (9)

wherein $R^9$ and $R^{10}$ independently represent an alkyl group having 1 to 10 carbon atoms or an alkenyl group having 2 to 10 carbon atoms any methylene group ($-CH_2-$) in the groups may be replaced by oxygen atom (-O-), but in no case two or more methylene groups are continuously replaced by oxygen atom; ring L represents trans-1,4-cyclohexylene group, 1,4-phenylene group, or pyrimidine-2,5-diyl group; ring M represents trans-1,4-cyclohexylene group, 1,4-phenylene group one or more hydrogen atoms on the ring may be replaced by F, or pyrimidine-2,5-diyl group; ring N represents trans-1,4-cyclohexylene group or 1,4-phenylene group; $Z^{12}$ and $Z^{14}$ independently represent -COO-, $-(CH_2)_2-$, or single bond; $Z^{13}$ represents -CH=CH-, -C≡C-, -COO-, or a covalent bond; and h is 0 or 1.

**14.** A liquid crystal composition comprising, as a first component, at least one liquid crystalline compound defined in any one of claims 1 to 10, comprising, as a part of a second component, at least one compound selected from the group consisting of the compounds expressed by any one of the general formulas (2), (3), and (4), and comprising, as another part of the second component, at least one compound selected from the group consisting of the compounds expressed by any one of the general formulas (5), (6), (7), (8), and (9).

**15.** A liquid crystal display device comprising the liquid crystal composition defined in any one of claims 11 to 14.

## INTERNATIONAL SEARCH REPORT

International application No.
PCT/JP97/01214

**A. CLASSIFICATION OF SUBJECT MATTER**

Int. Cl$^6$ C07C43/225, 255/54, C09K19/20, 30, 42, G02F1/13

According to International Patent Classification (IPC) or to both national classification and IPC

**B. FIELDS SEARCHED**

Minimum documentation searched (classification system followed by classification symbols)

Int. Cl$^6$ C07C43/225, 255/54, C09K19/20, 30, 42, G02F1/13

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| A | DE, 19531165, A1 (Merck Patent GmbH.), March 7, 1996 (07. 03. 96) & US, 5589102, A | 1 - 15 |
| A | JP, 5-112778, A (Asahi Glass Co., Ltd., Seimi Chemical Co., Ltd.), May 7, 1993 (07. 05. 93)(Family: none) | 1 - 15 |
| A | JP, 2-289529, A (Merck Patent GmbH.), November 29, 1990 (29. 11. 90) & DE, 4006921, A1 & GB, 2229438, A & US, 5045229, A | 1 - 15 |

☐ Further documents are listed in the continuation of Box C. ☐ See patent family annex.

* Special categories of cited documents:
- "A" document defining the general state of the art which is not considered to be of particular relevance
- "E" earlier document but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| June 17, 1997 (17. 06. 97) | July 1, 1997 (01. 07. 97) |
| Name and mailing address of the ISA/ Japanese Patent Office | Authorized officer |
| Facsimile No. | Telephone No. |

Form PCT/ISA/210 (second sheet) (July 1992)